# EUROPEAN PATENT APPLICATION

(11) **EP 4 740 963 A2**
(43) Date of publication of application: **13.05.2026**
(21) Application number: 26150898.0
(22) Date of filing: 14.10.2022
(51) Int. Cl.: A61K 47/10

(54) **FORMULATIONS OF FACTOR VIII CHIMERIC PROTEINS AND USES THEREOF**

(30) Priority: 15.10.2021 US 202163256432 P
(62) Divisional of application: 22813008.4
(71) Applicant: Bioverativ Therapeutics Inc., Waltham MA 02451 (US)
(72) Inventor: CARLAGE, Tyler, Waltham, 02451 (US); MAULDIN, Randall, Waltham, 02451 (US); MCCOY, Timothy R., Waltham, 02451 (US); TAZI, Loubna Mzaalak, Waltham, 02451 (US)
(74) Representative: Zwicker, Jörk

(57) **Abstract**

The present disclosure provides pharmaceutical compositions of a chimeric protein comprising a factor VIII (FVIII) polypeptide and a von Willebrand factor (VWF) polypeptide. Also disclosed are pharmaceutical kits and methods of using the disclosed pharmaceutical compositions to treat hemophilia A.

## Description

### RELATED APPLICATION

This application claims priority to U.S. provisional application no. 63/256,432, filed October 15, 2021, the contents of which are incorporated herein in their entirety.

### REFERENCE TO SEQUENCE LISTING SUBMITTED ELECTRONICALLY

The content of the electronically submitted sequence listing in XML file format (Name: SA9-483PC_SL_ST26.xml; Size: 55,697 bytes; Created: October 4, 2022) is incorporated herein by reference in its entirety.

### BACKGROUND OF THE DISCLOSURE

Hemophilia A is a bleeding disorder caused by defects in the gene encoding coagulation factor VIII (FVIII) and affects 1-2 in 10,000 male births. Graw et al., Nat. Rev. Genet. 6(6): 488-501 (2005). Patients affected with hemophilia A can be treated with infusions of purified plasma FVIII or recombinantly produced FVIII. Many commercially available FVIII products are known to have a half-life of about 8-12 hours, requiring frequent intravenous administration to the patients. See Weiner M.A. and Cairo, M.S., Pediatric Hematology Secrets, Lee, M.T., 12. Disorders of Coagulation, Elsevier Health Sciences, 2001; Lillicrap, D. Thromb. Res. 122 Suppl 4:S2-8 (2008). In addition, a number of approaches have been tried in order to extend the FVIII half-life. For example, the approaches in development to extend the half-life of clotting factors include pegylation, glycopegylation, and conjugation with albumin. See Dumont et al., Blood. 119(13): 3024-3030 (2012). Consistent results have been demonstrated in humans, for example, rFVIllFc was reported to improve half-life up to ~1.7-fold compared with ADVATE^{®} in hemophilia A patients. See Powell et al., Blood. 119(13): 3031-3037 (2012). Therefore, the half-life increases, despite minor improvements, indicate the presence of other half-life limiting factors, such as clearance by VWF. Pipe et al., Blood. 128(16):2007-2016 (2016)).

Efanesoctocog alfa (also known as Efa and BIVV001) is a fusion protein that is designed to uncouple recombinant clotting factor VIII from VWF in circulation. The chimeric protein comprises a single recombinant factor VIII protein fused to dimeric Fc, a D'D3 domain of VWF, and two ELNN Polypeptides. Chhabra et al. Blood 135(17): 1484-1496 (2020). In one early study involving patients with severe hemophilia A, a single intravenous injection of efanesoctocog alfa resulted in high sustained factor VIII activity levels, with a half-life that was up to four times the half-life associated with recombinant factor VIII. See Konkle et al., NEJM. 383:1018-27 (2020).

However, there remains a need for improved pharmaceutical compositions.

### SUMMARY OF THE DISCLOSURE

The present disclosure is directed to, *inter alia,* pharmaceutical compositions comprising a Factor VIII ("FVIII") protein, kits comprising such pharmaceutical compositions, and therapeutic methods and uses of the pharmaceutical compositions.

In some embodiments, the pharmaceutical composition comprises a chimeric protein or protein which comprises a first polypeptide chain which comprises a FVIII protein or a portion thereof and a first immunoglobulin ("Ig") constant region or a portion thereof, and a second polypeptide chain which comprises a von Willebrand Factor ("VWF") protein and a second Ig constant region or a portion thereof. In some embodiments, the chimeric protein comprises (i) a FVIII protein comprising a FVIII polypeptide, an ELNN Polypeptide inserted within the B domain (*e.g.*, replacing at least a portion of the B domain) of the FVIII polypeptide, and a first Fc region; and (ii) a VWF protein comprising a VWF fragment (*e.g.*, a fragment comprising the D'D3 domains of VWF, which fragment may comprise mutations), a second ELNN Polypeptide, a thrombin-cleavable linker (such as an a2 linker), and a second Fc region. In some embodiments, the chimeric protein disclosed herein is a FVIII-ELNN-Fc/D'D3-ELNN-Fc heterodimer.

In some embodiments, the pharmaceutical composition comprises: (a) a FVIII protein; (b) about 1% (w/v) to about 7.5% (w/v) sucrose; (c) about 5 mM to about 15 mM histidine; (d) about 200 mM to about 300 mM arginine; (e) about 2.5 mM to about 10 mM calcium chloride; and (f) about 0.01% (w/v) to about 1.0% (w/v) of a poloxamer. In some embodiments, the pharmaceutical composition comprises: (a) a FVIII protein; (b) about 1% (w/v) to about 7.5% (w/v) sucrose; (c) about 5 mM to about 15 mM L-histidine; (d) about 200 mM to about 300 mM L-arginine; (e) about 2.5 mM to about 10 mM calcium chloride; and (f) about 0.01% (w/v) to about 1.0% (w/v) of a poloxamer. In some embodiments, the pharmaceutical composition comprising: (a) a Factor VIII ("FVIII") protein; (b) about 1% (w/v) to about 7.5% (w/v) sucrose; (c) about 5 mM to about 15 mM L-histidine; (d) about 200 mM to about 300 mM L-arginine-HCI; (e) about 2.5 mM to about 10 mM calcium chloride dihydrate; and (f) about 0.008% (w/v) to about 0.1% (w/v) of a poloxamer.

In some embodiments, the composition comprises about 250 mM L-arginine. In some embodiments, the composition comprises about 250 mM L-arginine-HCI. In some embodiments, the poloxamer is poloxamer 188 (poloxamer 188, which is also known as P188). Poloxamer 188 is well known in the art.

In some embodiments, disclosed herein is a pharmaceutical composition comprising:
(a) a chimeric protein comprising a first polypeptide chain which comprises a Factor VIII ("FVIII") protein and a first immunoglobulin ("Ig") constant region or a portion thereof, and a second polypeptide chain which comprises a von Willebrand Factor ("VWF") protein and a second Ig constant region or a portion thereof;
(b) about 1% (w/v) to about 7.5% (w/v) sucrose;
(c) about 5 mM to about 15 mM histidine;
(d) about 200 mM to about 300 mM arginine;
(e) about 2.5 mM to about 10 mM calcium chloride; and
(f) about 0.01% (w/v) to about 1.0% (w/v) of poloxamer 188.

In some embodiments, the pharmaceutical composition comprises about 250 IU, 500 IU, 1000 IU, 2000 IU, 3000 IU, or 4,000 IU of the chimeric protein. In some embodiments, the pharmaceutical composition comprises more than about 250 IU of the chimeric protein. In some embodiments, the pharmaceutical composition comprises more than about 300 IU of the chimeric protein. In some embodiments, the pharmaceutical composition comprises more than about 500 IU of the chimeric protein. In some embodiments, the pharmaceutical composition comprises at least about 500 IU of the chimeric protein. In some embodiments, the pharmaceutical composition comprises more than about 85 IU/ml of the chimeric protein. In some embodiments, the pharmaceutical composition comprises more than about 100 IU/ml of the chimeric protein. In some embodiments, the pharmaceutical composition comprises more than about 200 IU/ml of the chimeric protein. In some embodiments, the pharmaceutical composition comprises at least about 200 IU/ml of the chimeric protein.

In some embodiments, the first polypeptide chain comprises the amino acid sequence set forth as SEQ ID NO: 1 and the second polypeptide chain comprises the amino acid sequence set forth as SEQ ID NO: 2, wherein the first polypeptide chain and the second polypeptide chain are covalently linked by two disulfide bonds between Fc domains in the first and second polypeptide chains. In some embodiments, the chimeric protein is efanesoctocog alfa.

In some embodiments, the pharmaceutical composition comprises about 1% (w/v) to about 5% (w/v) sucrose. In some embodments, the pharmaceutical composition comprises about 5 mM to about 15 mM histidine. In some embodments, the pharmaceutical composition comprises about 200 mM to about 300 mM arginine. In some embodments, the pharmaceutical composition comprises about 2.5 mM to about 10 mM calcium chloride. In some embodments, the pharmaceutical composition comprises poloxamer 188 (P188). In some embodiments, the pharmaceutical composition comprises about 0.01% (w/v) to about 1.0% (w/v) poloxamer 188.

In some embodiments, disclosed herein is a pharmaceutical composition comprising:
(a) about 1% (w/v) to about 5% (w/v) sucrose;
(b) about 5 mM to about 15 mM histidine;
(c) about 200 mM to about 300 mM arginine;
(d) about 2.5 mM to about 10 mM calcium chloride; and
(e) about 0.01% (w/v) to about 1.0% (w/v) poloxamer 188.

In some embodiments, disclosed herein is a pharmaceutical composition comprising:
(a) about 1%, 2%, or 5% (w/v) sucrose;
(b) about 10 mM histidine;
(c) about 250 mM arginine;
(d) about 5 mM calcium chloride; and
(e) about 0.1% (w/v) poloxamer 188.

In some embodiments, disclosed herein is a pharmaceutical composition comprising:
(a) about 5% (w/v) sucrose;
(b) about 10 mM L-histidine;
(c) about 250 mM L-arginine-HCI;
(d) about 5 mM calcium chloride; and
(e) about 0.1% (w/v) poloxamer 188.

In some embodiments, disclosed herein is a pharmaceutical composition comprising:
(a) about 2% (w/v) sucrose;
(b) about 10 mM L-histidine;
(c) about 250 mM L-arginine-HCI;
(d) about 5 mM calcium chloride; and
(e) about 0.1% (w/v) poloxamer 188.

In some embodiments, disclosed herein is a pharmaceutical composition comprising:
(a) about 1% (w/v) sucrose;
(b) about 10 mM L-histidine;
(c) about 250 mM L-arginine-HCI;
(d) about 5 mM calcium chloride; and
(e) about 0.1% (w/v) poloxamer 188.

In some embodiments, disclosed herein is a pharmaceutical composition comprising:
(a) 10 mg/mL to 60 mg/mL sucrose;
(b) 1.5 mg/mL to 2.0 mg/mL L-histidine;
(c) 40 mg/mL to 70 mg/mL L-arginine-HCI;
(d) 0.4 mg/mL to 0.9 mg/mL calcium chloride; and
(e) 0.6 mg/mL to 1.6 mg/mL poloxamer 188.

In some embodiments, disclosed herein is a pharmaceutical composition comprising:
(a) 56.12 mg/ml sucrose;
(b) 1.74 mg/ml L-histidine;
(c) 59.11 mg/ml L-arginine-HCI;
(d) 0.82 mg/ml calcium chloride dihydrate; and
(e) 1.12 mg/ml poloxamer 188.

In some embodiments, disclosed herein is a pharmaceutical composition comprising:
(a) 22.45 mg/ml sucrose;
(b) 1.74 mg/ml L-histidine;
(c) 59.11 mg/ml L-arginine-HCI;
(d) 0.82 mg/ml calcium chloride dihydrate; and
(e) 1.12 mg/ml poloxamer 188.

In some embodiments, disclosed herein is a pharmaceutical composition comprising:
(a) 22.45 mg/ml sucrose;
(b) 1.74 mg/ml L-histidine;
(c) 59.11 mg/ml L-arginine-HCI;
(d) 0.62 mg/ml calcium chloride; and
(e) 1.12 mg/ml poloxamer 188.

In some embodiments, disclosed herein is a pharmaceutical composition comprising:
(a) 11.23 mg/ml sucrose;
(b) 1.74 mg/ml L-histidine;
(c) 59.11 mg/ml L-arginine-HCI;
(d) 0.82 mg/ml calcium chloride dihydrate; and
(e) 1.12 mg/ml poloxamer 188.

In some embodiments, disclosed herein is a pharmaceutical composition comprising:
(a) 11.23 mg/ml sucrose;
(b) 1.74 mg/ml L-histidine;
(c) 59.11 mg/ml L-arginine-HCI;
(d) 0.62 mg/ml calcium chloride; and
(e) 1.12 mg/ml poloxamer 188.

In some embodiments, disclosed herein is a pharmaceutical composition comprising:
(a) 10 mg/mL to 60 mg/mL sucrose;
(b) 1.5 mg/mL to 2.0 mg/mL L-histidine;
(c) 40 mg/mL to 70 mg/mL L-arginine-HCI;
(d) 0.5 mg/mL to 0.9 mg/mL calcium chloride dihydrate; and
(e) 0.6 mg/mL to 1.6 mg/mL poloxamer 188.

In some embodiments, disclosed herein is a pharmaceutical composition comprising:
(a) about 50 mg/mL sucrose;
(b) about 1.6 mg/mL L-histidine;
(c) about 52.7 mg/mL L-arginine-HCI;
(d) about 0.7 mg/mL calcium chloride dihydrate; and
(e) about 1 mg/mL poloxamer 188.

In some embodiments, disclosed herein is a pharmaceutical composition comprising:
(a) about 20 mg/mL sucrose;
(b) about 1.6 mg/mL L-histidine;
(c) about 52.7 mg/mL L-arginine-HCI;
(d) about 0.7 mg/mL calcium chloride dihydrate; and
(e) about 1 mg/mL poloxamer 188.

In some embodiments, disclosed herein is a pharmaceutical composition comprising:
(a) about 20 mg/mL sucrose;
(b) about 1.6 mg/mL L-histidine;
(c) about 52.7 mg/mL L-arginine-HCI;
(d) about 0.6 mg/mL calcium chloride; and
(e) about 1 mg/mL poloxamer 188.

In some embodiments, disclosed herein is a pharmaceutical composition comprising:
(a) about 20 mg/mL sucrose;
(b) about 1.6 mg/mL L-histidine;
(c) about 43.6 mg/mL L-arginine;
(d) about 0.7 mg/mL calcium chloride dihydrate; and
(e) about 1 mg/mL poloxamer 188.

In some embodiments, disclosed herein is a pharmaceutical composition comprising:
(a) about 10 mg/mL sucrose;
(b) about 1.6 mg/mL L-histidine;
(c) about 43.6 mg/mL L-arginine;
(d) about 0.6 mg/mL calcium chloride; and
(e) about 1 mg/mL poloxamer 188.

In some embodiments, disclosed herein is a pharmaceutical composition comprising:
(a) about 10 mg/mL sucrose;
(b) about 1.6 mg/mL L-histidine;
(c) about 52.7 mg/mL L-arginine-HCI;
(d) about 0.7 mg/mL calcium chloride dihydrate; and
(e) about 1 mg/mL poloxamer 188.

In some embodiments, disclosed herein is a pharmaceutical composition comprising:
(a) about 10 mg/mL sucrose;
(b) about 1.6 mg/mL L-histidine;
(c) about 52.7 mg/mL L-arginine-HCI;
(d) about 0.6 mg/mL calcium chloride; and
(e) about 1 mg/mL poloxamer 188.

In some embodiments, disclosed herein is a pharmaceutical composition comprising:
(a) about 10 mg/mL sucrose;
(b) about 1.6 mg/mL L-histidine;
(c) about 43.6 mg/mL L-arginine;
(d) about 0.7 mg/mL calcium chloride dihydrate; and
(e) about 1 mg/mL poloxamer 188.

In some embodiments, disclosed herein is a pharmaceutical composition comprising:
(a) about 10 mg/mL sucrose;
(b) about 1.6 mg/mL L-histidine;
(c) about 43.6 mg/mL L-arginine;
(d) about 0.6 mg/mL calcium chloride; and
(e) about 1 mg/mL poloxamer 188.

In some embodiments, disclosed herein is a pharmaceutical composition comprising:
(a) 10 mg/mL to 60 mg/mL sucrose;
(b) 1.5 mg/mL to 2.0 mg/mL L-histidine;
(c) 50 mg/mL to 70 mg/mL L-arginine-HCI;
(d) 0.7 mg/mL to 0.9 mg/mL calcium chloride dihydrate; and
(e) 0.6 mg/mL to 1.6 mg/mL poloxamer 188.

In some embodiments, disclosed herein is a pharmaceutical composition comprising:
(a) 10 mg/mL to 60 mg/mL sucrose;
(b) 1.5 mg/mL to 2.0 mg/mL L-histidine;
(c) 50 mg/mL to 70 mg/mL L-arginine-HCI;
(d) 0.4 mg/mL to 0.8 mg/mL calcium chloride; and
(e) 0.6 mg/mL to 1.6 mg/mL poloxamer 188.

In some embodiments, disclosed herein is a pharmaceutical composition comprising:
(a) 10 mg/mL to 60 mg/mL sucrose;
(b) 1.5 mg/mL to 2.0 mg/mL L-histidine;
(c) 40 mg/mL to 60 mg/mL L-arginine;
(d) 0.7 mg/mL to 0.9 mg/mL calcium chloride; and
(e) 0.6 mg/mL to 1.6 mg/mL poloxamer 188.

In some embodiments, disclosed herein is a pharmaceutical composition comprising:
(a) 10 mg/mL to 60 mg/mL sucrose;
(b) 1.5 mg/mL to 2.0 mg/mL L-histidine;
(c) 40 mg/mL to 60 mg/mL L-arginine;
(d) 0.4 mg/mL to 0.7 mg/mL calcium chloride dihydrate; and
(e) 0.6 mg/mL to 1.6 mg/mL poloxamer 188.

In some embodiments, disclosed herein is a pharmaceutical composition comprising:
(a) 56.12 mg/ml sucrose;
(b) 1.74 mg/ml L-histidine;
(c) 59.11 mg/ml L-arginine-HCI;
(d) 0.82 mg/ml calcium chloride dihydrate; and
(e) 1.12 mg/ml poloxamer 188.

In some embodiments, disclosed herein is a pharmaceutical composition comprising:
(a) 22.45 mg/ml sucrose;
(b) 1.74 mg/ml L-histidine;
(c) 59.11 mg/ml L-arginine-HCI;
(d) 0.82 mg/ml calcium chloride dihydrate; and
(e) 1.12 mg/ml poloxamer 188.

In some embodiments, disclosed herein is a pharmaceutical composition comprising:
(a) 22.45 mg/ml sucrose;
(b) 1.74 mg/ml L-histidine;
(c) 48.88 mg/ml L-arginine;
(d) 0.82 mg/ml calcium chloride dihydrate; and
(e) 1.12 mg/ml poloxamer 188.

In some embodiments, disclosed herein is a pharmaceutical composition comprising:
(a) 22.45 mg/ml sucrose;
(b) 1.74 mg/ml L-histidine;
(c) 59.11 mg/ml L-arginine-HCI;
(d) 0.62 mg/ml calcium chloride; and
(e) 1.12 mg/ml poloxamer 188.

In some embodiments, disclosed herein is a pharmaceutical composition comprising:
(a) 22.45 mg/ml sucrose;
(b) 1.74 mg/ml L-histidine;
(c) 48.88 mg/ml L-arginine;
(d) 0.62 mg/ml calcium chloride; and
(e) 1.12 mg/ml poloxamer 188.

In some embodiments, disclosed herein is a pharmaceutical composition comprising:
(a) 11.23 mg/ml sucrose;
(b) 1.74 mg/ml L-histidine;
(c) 59.11 mg/ml L-arginine-HCI;
(d) 0.82 mg/ml calcium chloride dihydrate; and
(e) 1.12 mg/ml poloxamer 188.

In some embodiments, disclosed herein is a pharmaceutical composition comprising:
(a) 11.23 mg/ml sucrose;
(b) 1.74 mg/ml L-histidine;
(c) 48.88 mg/ml L-arginine;
(d) 0.82 mg/ml calcium chloride dihydrate; and
(e) 1.12 mg/ml poloxamer 188.

In some embodiments, disclosed herein is a pharmaceutical composition comprising:
(a) 11.23 mg/ml sucrose;
(b) 1.74 mg/ml L-histidine;
(c) 59.11 mg/ml L-arginine-HCI;
(d) 0.62 mg/ml calcium chloride; and
(e) 1.12 mg/ml poloxamer 188.

In some embodiments, disclosed herein is a pharmaceutical composition comprising:
(a) 11.23 mg/ml sucrose;
(b) 1.74 mg/ml L-histidine;
(c) 48.88 mg/ml L-arginine;
(d) 0.62 mg/ml calcium chloride; and
(e) 1.12 mg/ml poloxamer 188.

In some embodiments, disclosed herein is a pharmaceutical composition according to any of the disclosed embodiments, which further comprises a pH of about 6.5 to about 7.5. In some embodiments, the pharmaceutical composition disclosed herein has a pH of about 7.0. In some embodiments, the pharmaceutical composition disclosed herein has a pH of about 6.8. In some embodiments the pharmaceutical composition disclosed herein does not comprise NaCl. In some embodiments the pharmaceutical composition disclosed herein comprises less than 8.8 mg/mL sodium chloride (NaCl). In some embodiments, the pharmaceutical composition disclosed herein comprises L-histidine. In some embodiments, the pharmaceutical composition disclosed herein comprises L-arginine. In some embodiments, the pharmaceutical composition disclosed herein comprises arginine-HCl. In some embodiments, the pharmaceutical composition disclosed herein comprises L-arginine-HCI. In some embodiments, the pharmaceutical composition disclosed herein comprises calcium chloride dihydrate.

In some embodiments, disclosed herein is a pharmaceutical composition according to any of the disclosed embodiments, wherein the pharmaceutical composition has a chimeric protein concentration of about 0.8 to about 1.2 mg/mL. In some embodiments, the pharmaceutical composition disclosed herein comprises 75 IU/mL to 2,000 IU/mL of the chimeric protein. In some embodiments, the pharmaceutical composition disclosed herein has an osmolality about 525 to about 725 mOsm/kg. In some embodiments, the pharmaceutical composition disclosed herein has an osmolality about 500 to about 650 mOsm/kg. In some embodiments, the pharmaceutical composition disclosed herein has an osmolality about 600 to about 650 mOsm/kg. In some embodiments, the pharmaceutical composition disclosed herein has a turbidity of less than about 7 Nephelometric Turbidity Units (NTU).

In some embodiments, disclosed herein is a method of storing a pharmaceutical composition, comprising maintaining a pharmaceutical composition disclosed herein at a temperature of from about -20°C to about -40°C for at least about 1 to about 36 months. In some embodiments, the pharmaceutical composition is stored at a temperature of about -20°C, about -25°C, about -30°C, about -35°C, or about -40°C for at least about 1 to about 36 months. In some embodiments, the pharmaceutical composition is stored at a temperature of about -20°C, about -25°C, about -30°C, about -35°C, or about -40°C for at least about 3, 6, 9, 12, 18, 21, 24, 27, 30, 33, or 36 months. In some embodiments, the pharmaceutical composition is stored at a temperature of about -30°C for at least about 1, 2, 3, 4, 5, 6, 7, 8, 9, 11, or 12 months.

In some embodiments, the pharmaceutical composition has been stored at a temperature of from about -20°C to about -40°C for at least about 1 to about 36 months. In some embodiments, the pharmaceutical composition has been stored at a temperature of about -20°C, about -25°C, about -30°C, about -35°C, or about -40°C for at least about 1 to about 36 months. In some embodiments, the pharmaceutical composition has been stored at a temperature of about -20°C, about -25°C, about -30°C, about -35°C, or about -40°C for at least about 3, 6, 9, 12, 18, 21, 24, 27, 30, 33, or 36 months. In some embodiments, the pharmaceutical composition has been stored at a temperature of about -30°C for at least about 1, 2, 3, 4, 5, 6, 7, 8, 9, 11, or 12 months.

In some embodiments, the pharmaceutical composition is a liquid pharmaceutical composition that has been frozen and then thawed 2-5 times. In some embodiments, the pharmaceutical composition is a liquid pharmaceutical composition that has been frozen and then thawed 2 times. In some embodiments, the pharmaceutical composition is a liquid pharmaceutical composition that has been frozen and then thawed 3 times. In some embodiments, the pharmaceutical composition is a liquid pharmaceutical composition that has been frozen and then thawed 4 times. In some embodiments, the pharmaceutical composition is a liquid pharmaceutical composition that has been frozen and then thawed 5 times.

In some embodiments, the pharmaceutical composition comprises about 0.5 mg/ml to about 10 mg/ml of the chimeric protein. In some embodiments, the pharmaceutical composition comprises about 0.5 mg/ml to about 5 mg/ml of the chimeric protein. In some embodiments, the pharmaceutical composition comprises about 0.5 mg/ml to about 2 mg/ml of the chimeric protein. In some embodiments, the pharmaceutical composition comprises about 0.5 mg/ml of the chimeric protein. In some embodiments, the pharmaceutical composition comprises about 1 mg/ml of the chimeric protein. In some embodiments, the pharmaceutical composition comprises about 1.5 mg/ml of the chimeric protein. In some embodiments, the pharmaceutical composition comprises about 2 mg/ml of the chimeric protein.

Also disclosed herein is a method of treating hemophilia A in a subject in need thereof, comprising administering to the subject an effective amount of the pharmaceutical composition according to any of the embodiments disclosed herein. In some embodiments, the pharmaceutical composition is self-administered. In some embodiments, the pharmaceutical composition is administered intravenously. In some embodiments, the pharmaceutical composition is administered intravenously at a dose of about 20 IU/kg to about 70 IU/kg. In some embodiments, the pharmaceutical composition is administered intravenously at a dose of about 50 IU/kg. In some embodiments, the pharmaceutical composition is administered intravenously once every 7-10 days. In some embodiments, the pharmaceutical composition is administered intravenously once weekly.

Also disclosed herein is a pharmaceutical kit comprising (i) a first container comprising a lyophilized pharmaceutical composition comprising
(a) a chimeric protein comprising a first polypeptide chain which comprises a Factor VIII ("FVIII") protein or a portion thereof and a first immunoglobulin ("Ig") constant region or a portion thereof, and a second polypeptide chain which comprises a von Willebrand Factor ("VWF") protein and a second Ig constant region or a portion thereof;
(b) about 10 mg to about 200 mg sucrose;
(c) about 2.5 mg to about 7.5 mg histidine;
(d) about 140 mg to about 200 mg arginine;
(e) about 1.5 mg to about 5 mg calcium chloride; and
(f) about 1 mg to about 10 mg poloxamer 188, and
(ii) a second container comprising sterile water.

In some embodiments, the chimeric protein comprises a first polypeptide chain comprising the amino acid sequence set forth as SEQ ID NO: 1 and a second polypeptide chain comprising the amino acid sequence set forth as SEQ ID NO: 2, wherein the first polypeptide chain and the second polypeptide chain are covalently linked by two disulfide bonds between Fc domains in the first and second polypeptide chains.

In some embodiments, the pharmaceutical kit disclosed herein comprises a lyophilized pharmaceutical composition comprising:
(a) about 10 mg to about 200 mg sucrose;
(b) about 2.5 mg to about 7.5 mg histidine;
(c) about 140 mg to about 200 mg arginine;
(d) about 1.5 mg to about 5 mg calcium chloride; and
(e) about 1 mg to about 10 mg poloxamer 188.

In some embodiments the pharmaceutical kit comprises a lyophilized pharmaceutical composition that does not comprise NaCl. In some embodiments the lyophilized pharmaceutical composition comprises less than 8.8 mg/mL sodium chloride (NaCl). In some embodiments, the lyophilized pharmaceutical composition comprises L-histidine. In some embodiments, the lyophilized pharmaceutical composition comprises L-arginine. In some embodiments, the lyophilized pharmaceutical composition comprises arginine-HCl. In some embodiments, the lyophilized pharmaceutical composition comprises L-arginine-HCl. In some embodiments, the lyophilized pharmaceutical composition disclosed herein comprises calcium chloride dihydrate.

In some embodiments, the pharmaceutical kit comprises a lyophilized pharmaceutical composition comprising:
(a) about 33.7 mg sucrose;
(b) about 5.2 mg L-histidine;
(c) about 177.3 mg L-arginine-HCI;
(d) about 2.5 mg calcium chloride; and
(e) about 3.4 mg poloxamer 188.

In some embodiments, the pharmaceutical kit comprises a lyophilized pharmaceutical composition comprising:
(a) about 67.3 mg sucrose;
(b) about 5.2 mg L-histidine;
(c) about 177.3 mg L-arginine-HCI;
(d) about 2.5 mg calcium chloride; and
(e) about 3.4 mg poloxamer 188.

In some embodiments, the pharmaceutical kit comprises a lyophilized pharmaceutical composition comprising:
(a) about 67.3 mg sucrose;
(b) about 5.2 mg L-histidine;
(c) about 146.6 mg L-arginine-HCI;
(d) about 2.5 mg calcium chloride; and
(e) about 3.4 mg poloxamer 188.

In some embodiments, the pharmaceutical kit comprises a lyophilized pharmaceutical composition comprising:
(a) about 168.3 mg sucrose;
(b) about 5.2 mg L-histidine;
(c) about 177.3 mg L-arginine-HCl;
(d) about 2.5 mg calcium chloride; and
(e) about 3.4 mg poloxamer 188.

In some embodiments, the pharmaceutical kit comprises a lyophilized pharmaceutical composition having a moisture content of less than 2%. In some embodiments, the lyophilized pharmaceutical composition has a moisture content of less than 1.8%. In some embodiments, the lyophilized pharmaceutical composition has a moisture content of less than 1.6%. In some embodiments, the lyophilized pharmaceutical composition is in a lyophilized cake. In some embodiments, the lyophilized cake is white. In some embodiments, the lyophilized cake is less than Y4 in the European Pharmacopoeia color scale. In some embodiments, the lyophilized pharmaceutical composition is a powder.

In some embodiments, the pharmaceutical kit comprises a first container comprising 100 IU to 10,000 IU of the chimeric protein. In some embodiments, the first container comprises 250 IU, 500 IU, 1000 IU, 2000 IU, 3000 IU, or 4,000 IU of the chimeric protein. In some embodiments, the first container comprises more than about 250 IU of the chimeric protein. In some embodiments, the first container comprises more than about 300 IU of the chimeric protein. In some embodiments, the first container comprises more than about 500 IU of the chimeric protein. In some embodiments, the first container comprises at least about 500 IU of the chimeric protein.

In some embodiments, the pharmaceutical kit further comprises instructions for combining the lyophilized pharmaceutical composition and sterile water. In some embodiments, when the lyophilized pharmaceutical composition and the sterile water are combined, then the lyophilized pharmaceutical composition is reconstituted within 7 to 12 seconds.

In some embodiments, the pharmaceutical composition comprises a second container comprising sterilized water at a volume sufficient to produce, when combined with the lyophilized powder of the first container, a solution comprising

In some embodiments, when the lyophilized pharmaceutical composition and the sterile water are combined, then the osmolality of the resulting solution is about 525 to about 725 mOsm/kg. In some embodiments, the pharmaceutical composition disclosed herein has an osmolality about 500 to about 650 mOsm/kg. In some embodiments, the osmolality of the resulting solution is about 600 to about 650 mOsm/kg. In some embodiments, the pH of the resulting solution is about 6.5 to about 7.5. In some embodiments, the pH of the resulting solution is about 7.0. In some embodiments, the pH of the resulting solution is about 6.8. In some embodiments, the the turbidity of the resulting solution is less than about 7 Nephelometric Turbidity Units (NTU). In some embodiments, the protein concentration of the resulting solution is about 0.8 to about 1.2 mg/mL. In some embodiments, less than 3% of the protein is aggregated.

In some embodiments, the pharmaceutical kit comprises a second container comprising sterilized water at a volume sufficient to produce, when combined with the lyophilized pharmaceutical composition of the first container, a solution comprising:
(a) 10 mg/mL to 60 mg/mL sucrose;
(b) 1.5 mg/mL to 2.0 mg/mL L-histidine;
(c) 40 mg/mL to 70 mg/mL L-arginine-HCI;
(d) 0.5 mg/mL to 0.9 mg/mL calcium chloride; and
(e) 0.6 mg/mL to 1.6 mg/mL poloxamer 188.

In some embodiments of the pharmaceutical kit disclosed herein, when the lyophilized pharmaceutical composition and the sterile water are combined, then the resulting solution comprises:
(a) 10 mg/mL to 60 mg/mL sucrose;
(b) 1.5 mg/mL to 2.0 mg/mL L-histidine;
(c) 40 mg/mL to 70 mg/mL L-arginine-HCI;
(d) 0.5 mg/mL to 0.9 mg/mL calcium chloride; and
(e) 0.6 mg/mL to 1.6 mg/mL poloxamer 188.

In some embodiments of the pharmaceutical kit disclosed herein, when the lyophilized pharmaceutical composition and the sterile water are combined, then the resulting solution comprises:
(a) 11.23 mg/ml sucrose;
(b) 1.74 mg/ml L-histidine;
(c) 59.11 mg/ml L-arginine-HCI;
(d) 0.62 mg/ml calcium chloride; and
(e) 1.12 mg/ml poloxamer 188.

In some embodiments of the pharmaceutical kit disclosed herein, when the lyophilized pharmaceutical composition and the sterile water are combined, then the resulting solution comprises:
(a) 22.45 mg/ml sucrose;
(b) 1.74 mg/ml L-histidine;
(c) 59.11 mg/ml L-arginine-HCI;
(d) 0.62 mg/ml calcium chloride; and
(e) 1.12 mg/ml poloxamer 188.

In some embodiments of the pharmaceutical kit disclosed herein, when the lyophilized pharmaceutical composition and the sterile water are combined, then the resulting solution comprises:
(a) 22.45 mg/ml sucrose;
(b) 1.74 mg/ml L-histidine;
(c) 48.88 mg/ml L-arginine;
(d) 0.62 mg/ml calcium chloride; and
(e) 1.12 mg/ml poloxamer 188.

In some embodiments of the pharmaceutical kit disclosed herein, when the lyophilized pharmaceutical composition and the sterile water are combined, then the resulting solution comprises:
(a) 56.12 mg/ml sucrose;
(b) 1.74 mg/ml L-histidine;
(c) 59.11 mg/ml L-arginine-HCI;
(d) 0.62 mg/ml calcium chloride; and
(e) 1.12 mg/ml poloxamer 188.

In some embodiments, the pharmaceutical kit comprises a second container comprising about 2 mL to about 5 mL of sterile water. In some embodiments, the pharmaceutical kit comprises a second container comprising about 3 mL of sterile water. In some embodiments, the pharmaceutical kit comprises a second container comprising about 3.3 mL of sterile water.

In some embodiments, the pharmaceutical kit is stored at a temperature of from about - 20°C to about -40°C for at least about 1 to about 36 months. In some embodiments, the pharmaceutical kit is stored at a temperature of about -20°C, about -25°C, about -30°C, about -35°C, or about -40°C for at least about 1 to about 36 months. In some embodiments, the pharmaceutical kit is stored at a temperature of about -20°C, about -25°C, about -30°C, about -35°C, or about -40°C for at least about 3, 6, 9, 12, 18, 21, 24, 27, 30, 33, or 36 months. In some embodiments, the pharmaceutical kit is stored at a temperature of about -30°C for at least about 1, 2, 3, 4, 5, 6, 7, 8, 9, 11, or 12 months.

In some embodiments, the pharmaceutical kit has been stored at a temperature of from about -20°C to about -40°C for at least about 1 to about 36 months. In some embodiments, the pharmaceutical kit has been stored at a temperature of about -20°C, about -25°C, about -30°C, about -35°C, or about -40°C for at least about 1 to about 36 months. In some embodiments, the pharmaceutical kit has been stored at a temperature of about -20°C, about -25°C, about -30°C, about -35°C, or about -40°C for at least about 3, 6, 9, 12, 18, 21, 24, 27, 30, 33, or 36 months. In some embodiments, the pharmaceutical kit has been stored at a temperature of about -30°C for at least about 1, 2, 3, 4, 5, 6, 7, 8, 9, 11, or 12 months.

In some embodiments, the pharmaceutical kit comprises a first container which is a glass vial comprising a rubber stopper. In some embodiments, the pharmaceutical kit comprises a second container which is a syringe body. In some embodiments, the sterile water is in the syringe body. In some embodiments, the syringe body is associated with a plunger. In some embodiments, the pharmaceutical kit further comprises an adaptor to connect the glass vial to the syringe body. In some embodiments, the pharmaceutical kit further comprises infusion tubing associated with a needle to be connected to the syringe body, suitable for intravenous infusion.

Also disclosed herein is a method of treating hemophilia A in a subject in need thereof, comprising combining the lyophilized pharmaceutical composition and the sterile water of the pharmaceutical kit according to any of the embodiments disclosed herein, and administering to the subject an effective amount of the resulting combination (i.e. solution). In some embodiments, the subject combines the lyophilized pharmaceutical composition and the sterile water of the kit. In some embodiments, the combination is self-administered by the subject.

### BRIEF DESCRIPTION OF THE DRAWINGS

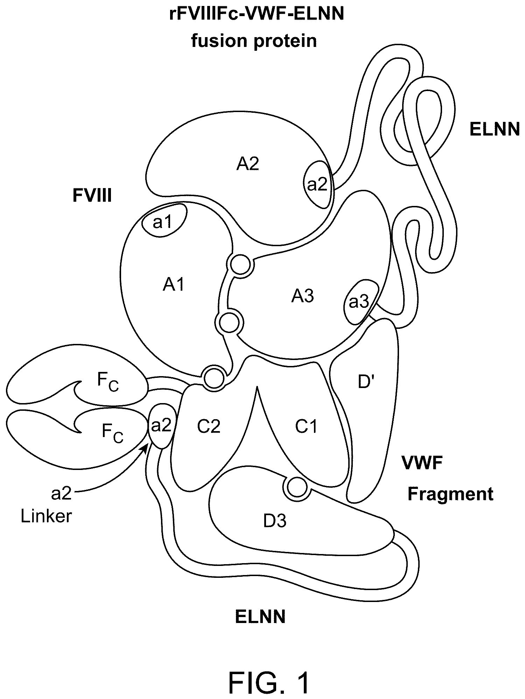

FIG. 1 is a schematic representation of efanesoctocog alfa, an exemplary FVIII-ELNN-Fc/D'D3-ELNN-Fc heterodimer. FVIII: factor VIII; VWF: von Willebrand Factor; A1, A2, A3, C1, C2: domains of FVIII; D'D3: domains of VWF; Fc: Fc region of immunoglobulin constant region.

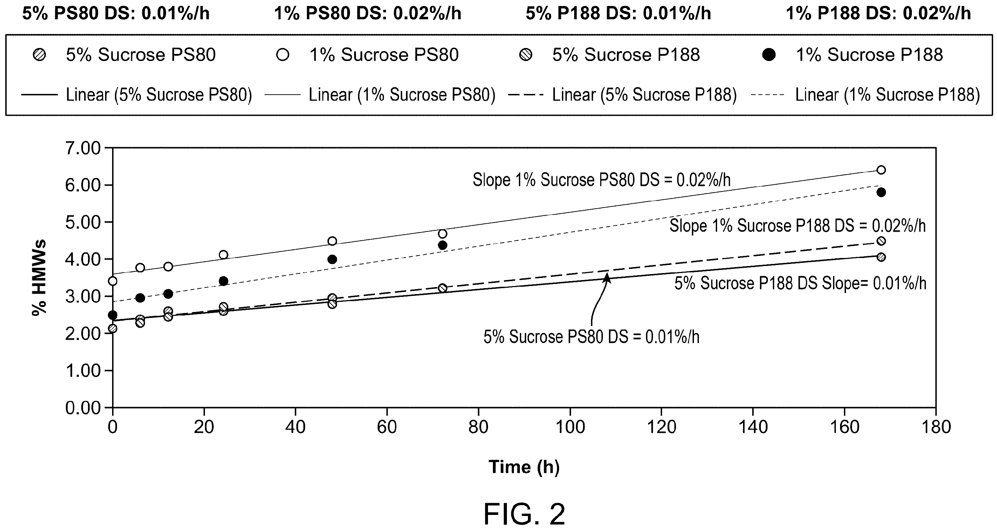

FIG. 2 shows the aggregation levels (%HMWS) over time (hours) at room temperature (RT)/room light (RL) conditions for efanesoctocog alfa drug substance (DS) compositions (1 mg/mL) containing either 0.05% (w/v) PS80 or 0.1% (w/v) poloxamer 188 as a surfactant and either 1% or 5% (w/v) sucrose. Rates of change in aggregation levels (slope) are also provided (% per hour)

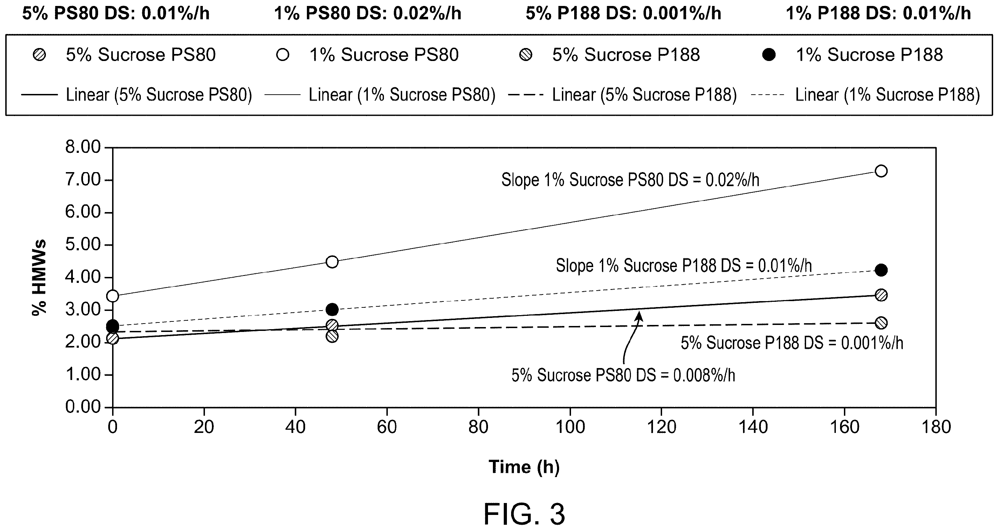

FIG. 3 shows the aggregation levels (%HMWS) over time (hours) at 2-8 °C for efanesoctocog alfa drug substance (DS) compositions (1 mg/mL) containing either 0.05% (w/v) PS80 or 0.1% (w/v) poloxamer 188 as a surfactant and either 1% or 5% (w/v) sucrose. Rates of change in aggregation levels (slope) are also provided (% per hour).

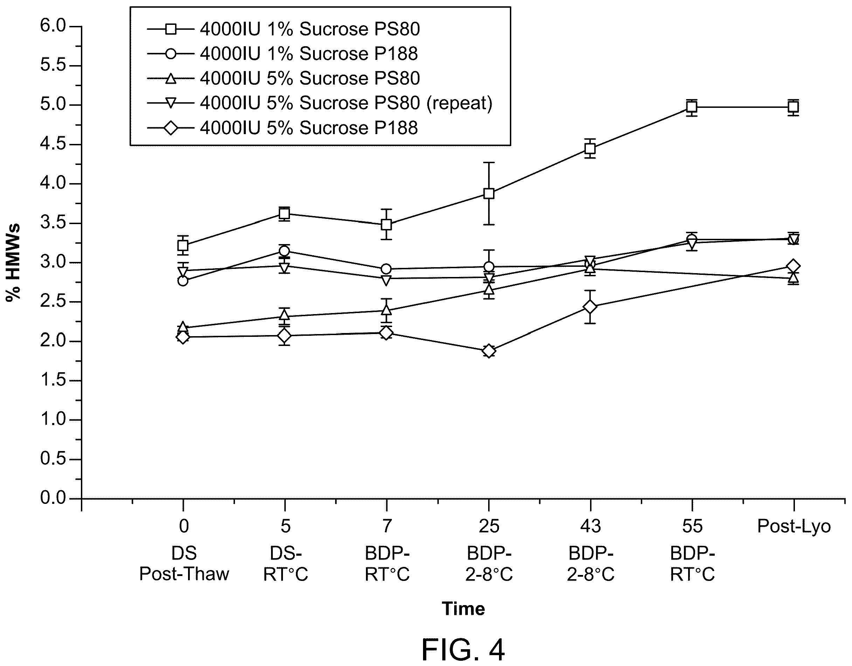

FIG. 4 shows the aggregation levels (%HMWS) of efanesoctocog alfa drug substance (DS) compositions flowed by dilution to 4000 IU bulk drug product at 7 hours and containing either 0.05% (w/v) PS80 or 0.1% (w/v) poloxamer 188 as a surfactant and 1% or 5% (w/v) sucrose. Samples were held at RT and tested after 0, 5, 7, 25, 43, and 55 hours. Samples were also tested after lyophilization (Post-Lyo).

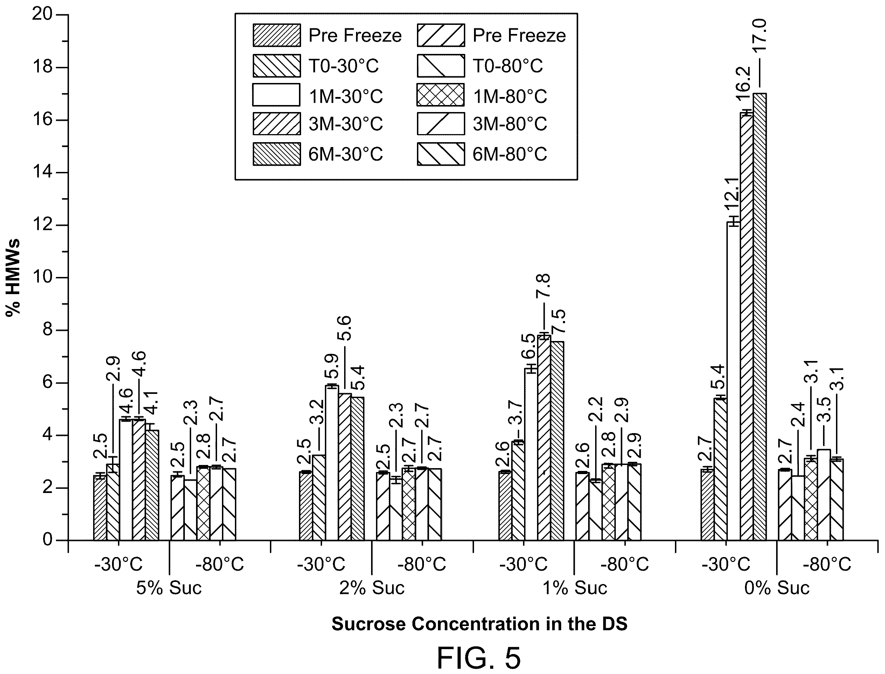

FIG. 5 shows a frozen storage stability study which measured the aggregation levels (%HMWS) of efanesoctocog alfa DS compositions (1 mg/mL) containing 0.05% (w/v) PS80 as a surfactant. Compositions containing 0, 1, 2, or 5% (w/v) sucrose were held at freezing temperatures (-80°C or -30°C). Time points measured were pre-freeze, start of experiment (T0), one month (T1M), 3 months (T3M), and 6 months (T6M).

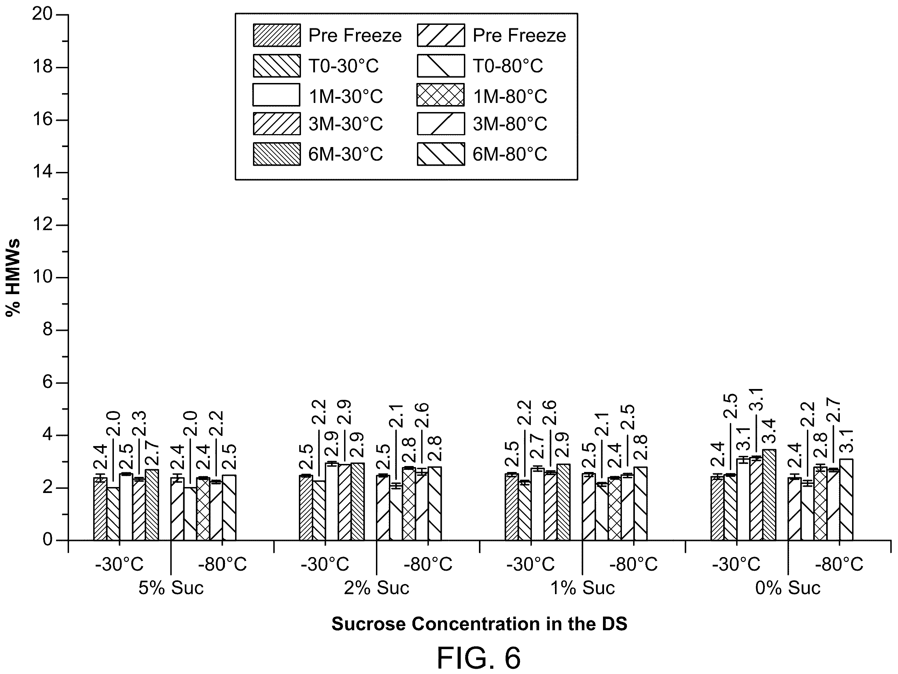

FIG. 6 shows a frozen storage stability study which measured the aggregation levels (%HMWS) of efanesoctocog alfa DS compositions (1 mg/mL) containing 0.1% (w/v) poloxamer 188 as a surfactant. Compositions containing 0, 1, 2, or 5% (w/v) sucrose were held at freezing temperatures (-80°C or -30°C). Time points measured are pre-freeze, start of experiment (T0), one month (T1M), 3 months (T3M), and 6 months (T6M).

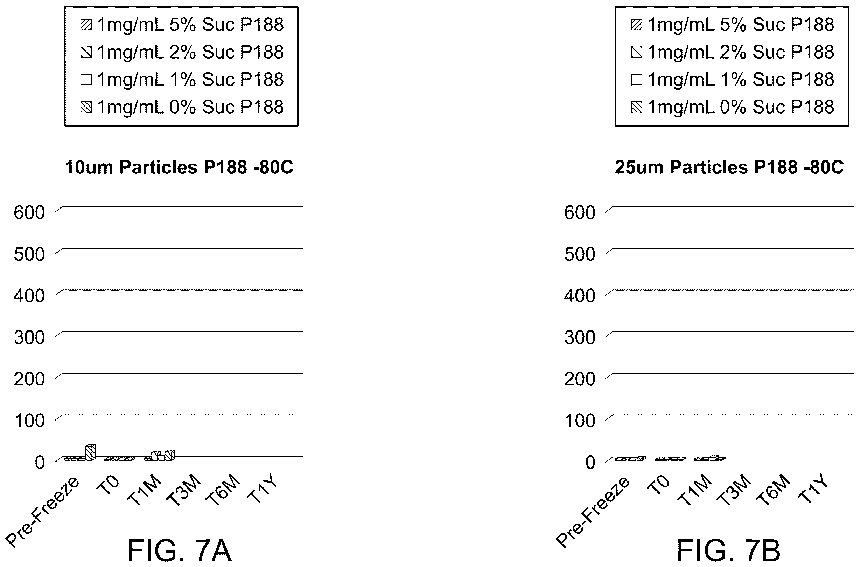

FIG. 7 shows the results of an analysis of efanesoctocog alfa DS compositions (1 mg/mL) by particle testing using high accuracy liquid particle counter (HIAC). DS compositions containing 0.1% (w/v) poloxamer 188 and 0, 1, 2, or 5% (w/v) sucrose were held at -80°C and assessed at the following time points: pre-freeze, start of experiment (T0), one month (T1M), 3 months (T3M), and 6 months (T6M). Results for ≥10 µm particles are shown in FIG. 7A. Results for ≥25 µm particles are shown in FIG. 7B.

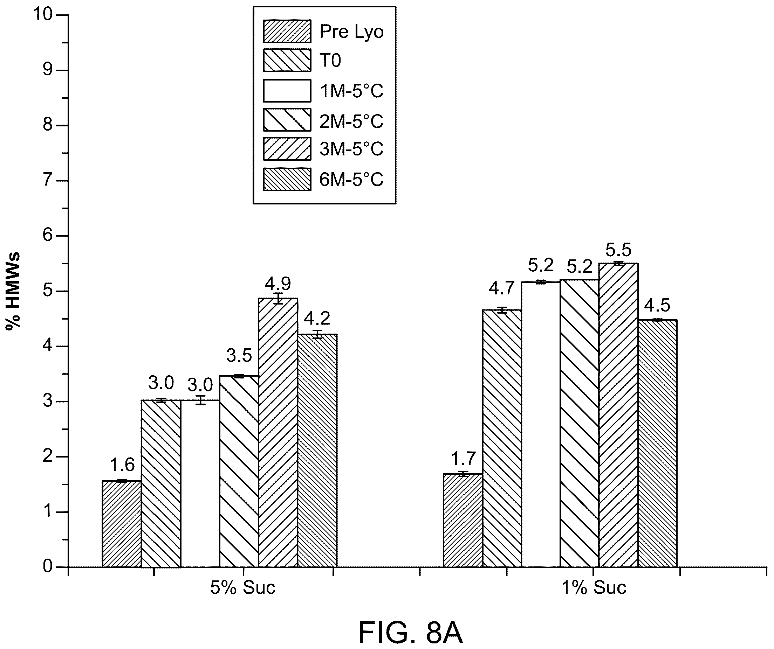

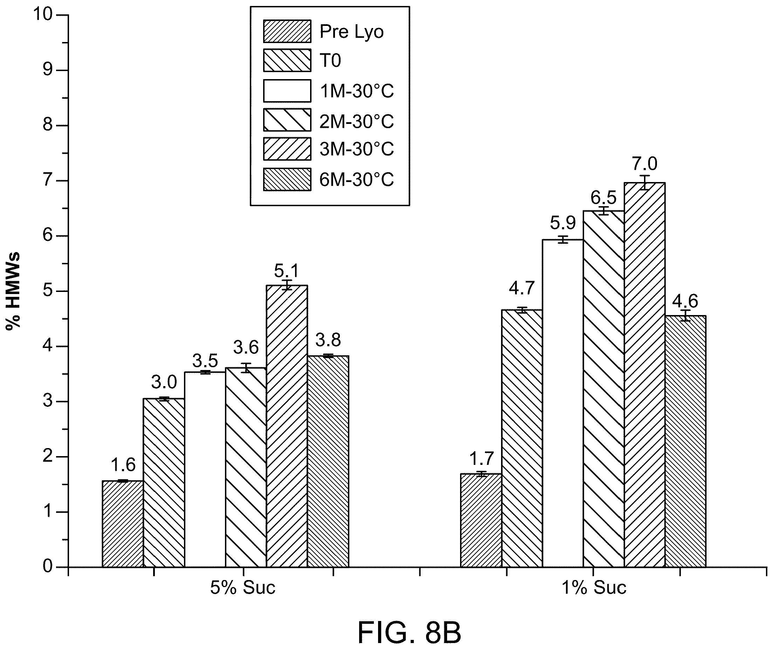

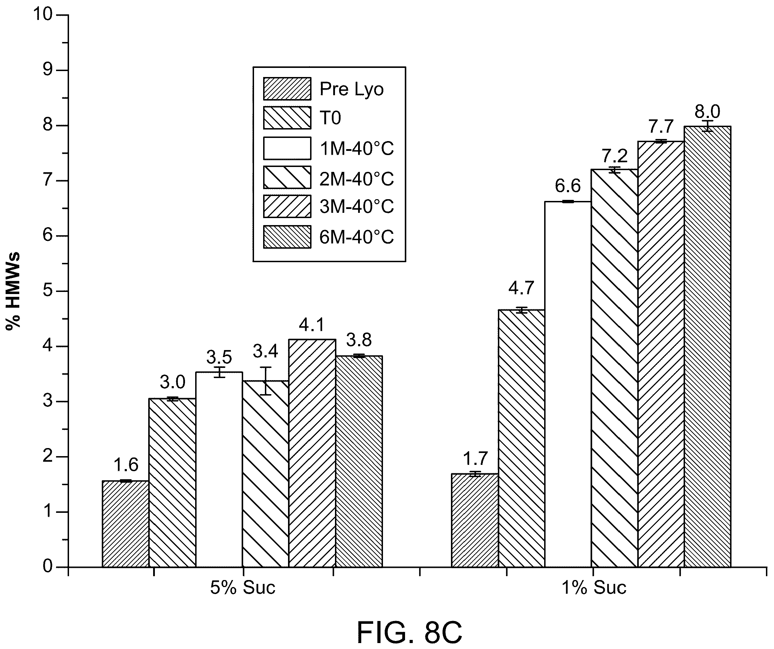

FIG. 8 shows the aggregation levels (%HMWS) for compositions of efanesoctocog alfa lyophilized drug product (Lyo DP) at 250 IU and comprising 0.1% (w/v) poloxamer 188 as surfactant. Lyo DP compositions containing 1% or 5% (w/v) sucrose were tested. Samples were held at 5°C, 30°C, or 40°C and tested at T0, 1 month, 2 months, 3 months, and 6 months. Results are shown in FIG. 8A (5°C), FIG. 8B (30°C), and FIG. 8C (40°C).

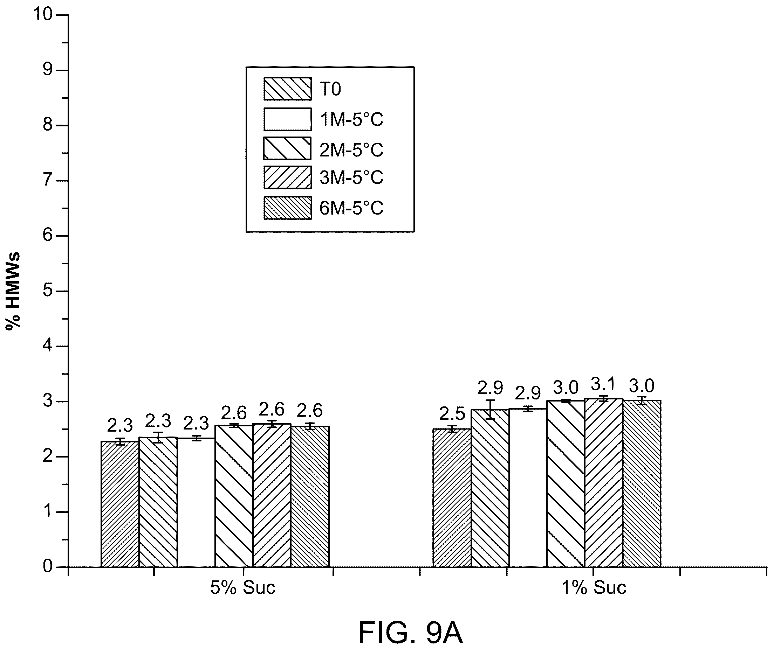

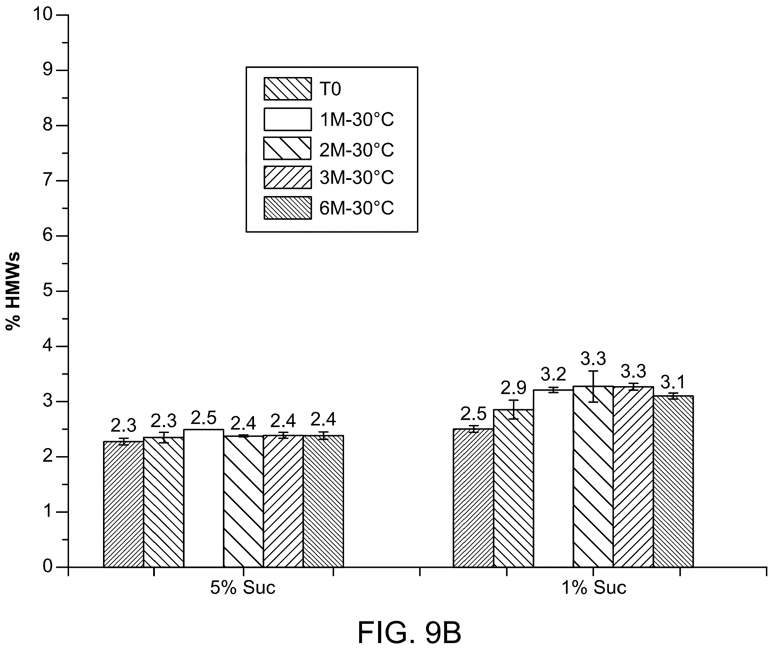

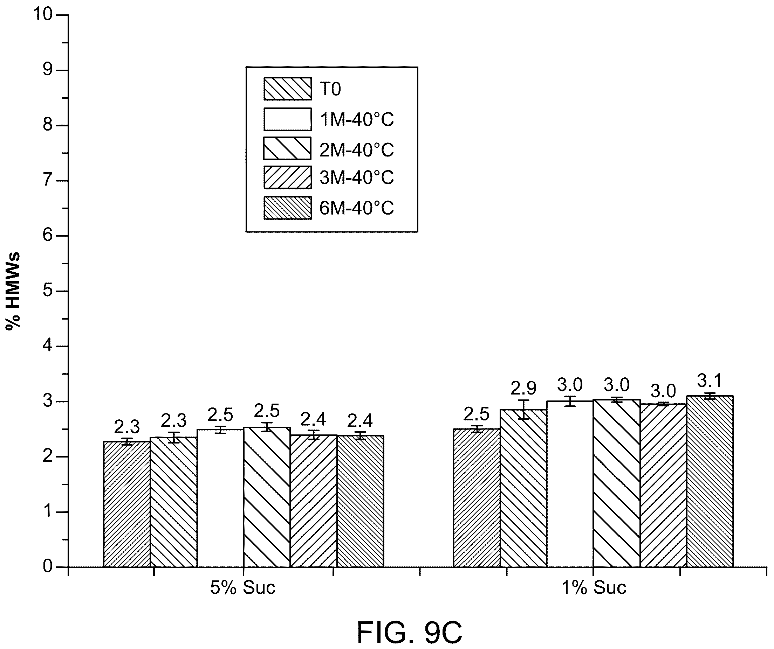

FIG. 9 shows the aggregation levels (%HMWS) for compositions of efanesoctocog alfa lyophilized drug product (Lyo DP) at 4000 IU and comprising 0.1% (w/v) poloxamer 188 as surfactant. Lyo DP compositions containing 1% or 5% (w/v) sucrose were tested. Samples were held at 5°C, 30°C, or 40°C and tested at T0, 1 month, 2 months, 3 months, and 6 months. Results are shown in FIG. 9A (5°C), FIG. 9B (30°C), and FIG. 9C (40°C).

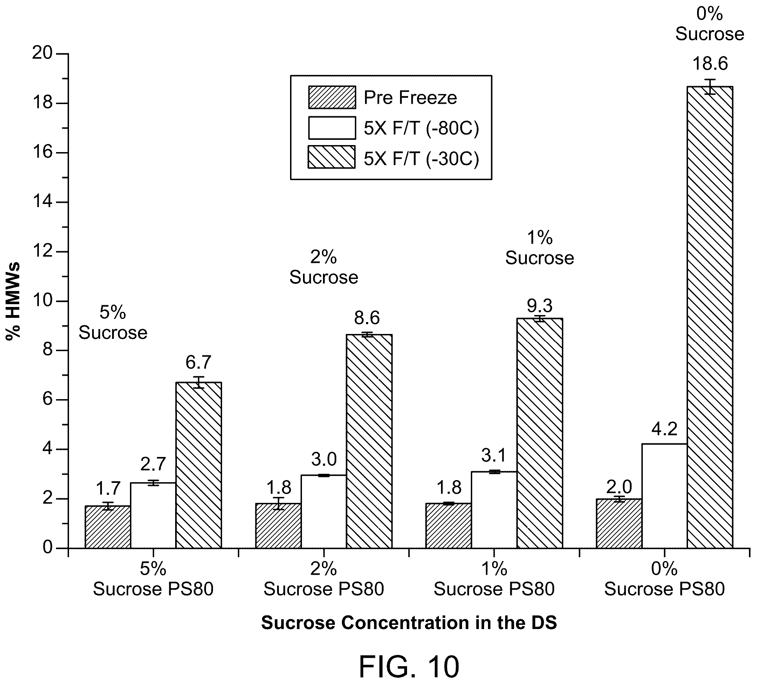

FIG. 10 shows the aggregation levels (%HMWS) of compositions of efanesoctocog alfa DS (1 mg/mL) before (pre freeze) and after stress by freeze/thaw (F/T) at either -80°C or -30°C for 5 cycles (5X)(not less than 24hrs, thaw at room temperature). FIG. 10 shows the results for DS compositions containing 0.05% (w/v) PS80 and 0, 1, 2, or 5% (w/v) sucrose.

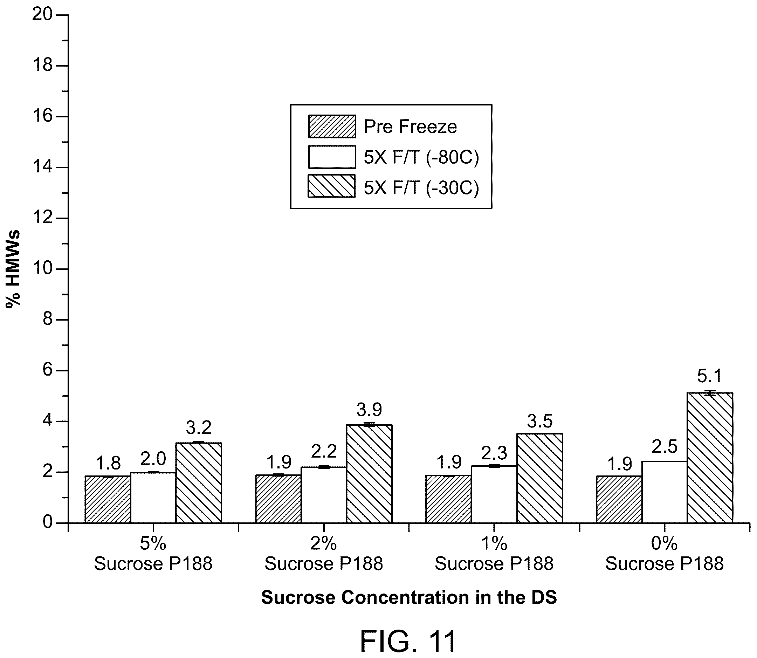

FIG. 11 shows the aggregation levels (%HMWS) of compositions of efanesoctocog alfa DS (1 mg/mL) before (pre freeze) and after stress by freeze/thaw (F/T) at either -80°C or -30°C for 5 cycles (5X)(not less than 24hrs, thaw at room temperature). FIG. 11 shows the results for DS compositions containing 0.1% (w/v) poloxamer 188 and 0, 1, 2, or 5% (w/v) sucrose.

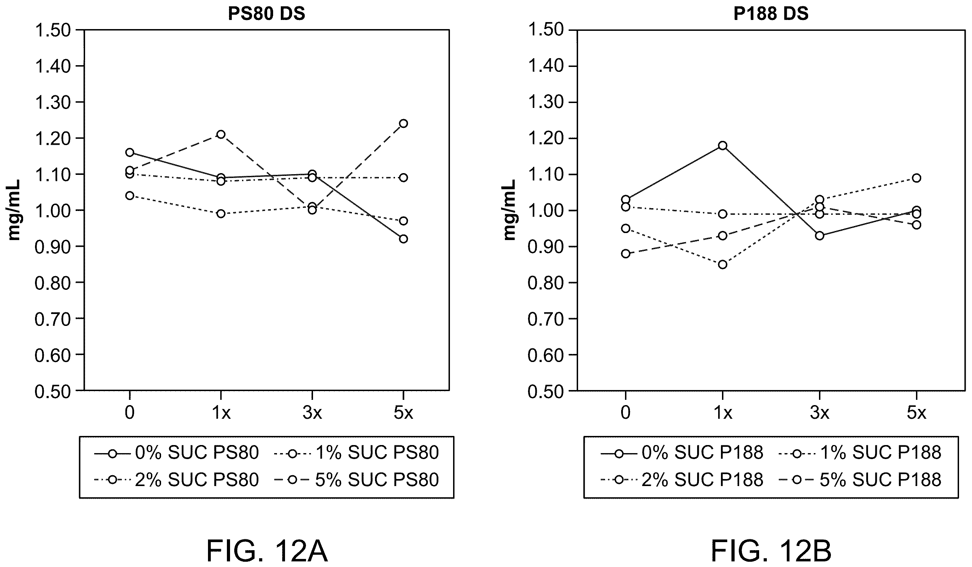

FIG. 12 shows protein concentration analysis by A280 (absorbance at 280nm) of different sucrose concentrations in efanesoctocog alfa DS (1 mg/mL) compositions following F/T stress. Samples were analyzed before (pre freeze) and after 1, 3, or 5 F/T cycles. Results for DS compositions containing 0.05% (w/v) PS80 and 0, 1, 2, or 5% (w/v) sucrose are shown in FIG. 12A. Results for DS compositions containing 0.1% (w/v) poloxamer 188 and 0, 1, 2, or 5% (w/v) sucrose are shown in FIG. 12B.

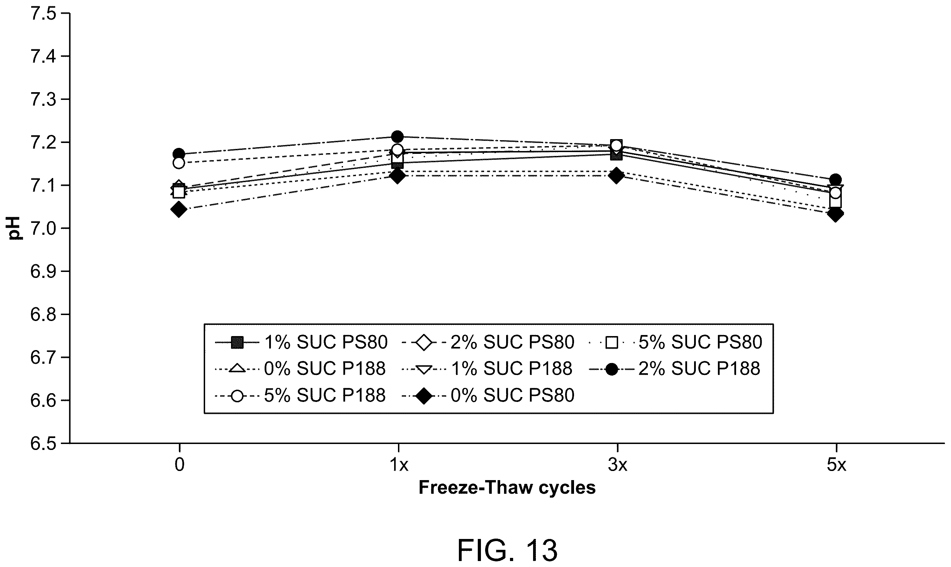

FIG. 13 shows an analysis of pH following F/T stress for efanesoctocog alfa DS compositions containing either 0.05% (w/v) PS80 or 0.1% (w/v) poloxamer 188 as a surfactant and 0, 1, 2, or 5% (w/v) sucrose. pH was measured after 0, 1, 3, or 5 F/T cycles.

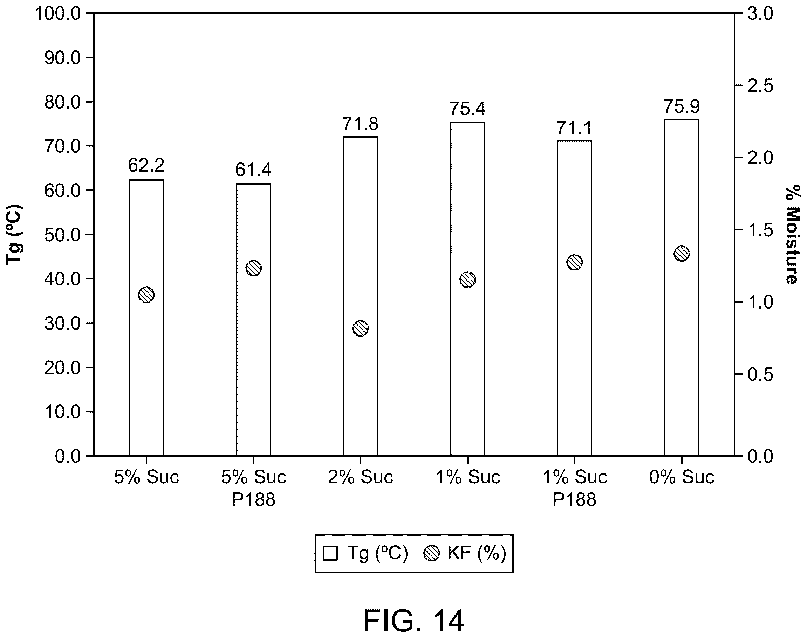

FIG. 14 shows the glass transition temperature (Tg) and residual moisture content (%) at the start of experiment (T0) for compositions of efanesoctocog alfa lyophilized drug product (Lyo DP) at 250 IU. Lyo DP compositions containing either 0.05% (w/v) PS80 or 0.1% (w/v) poloxamer 188 and 0, 1, 2, or 5% (w/v) sucrose were tested.

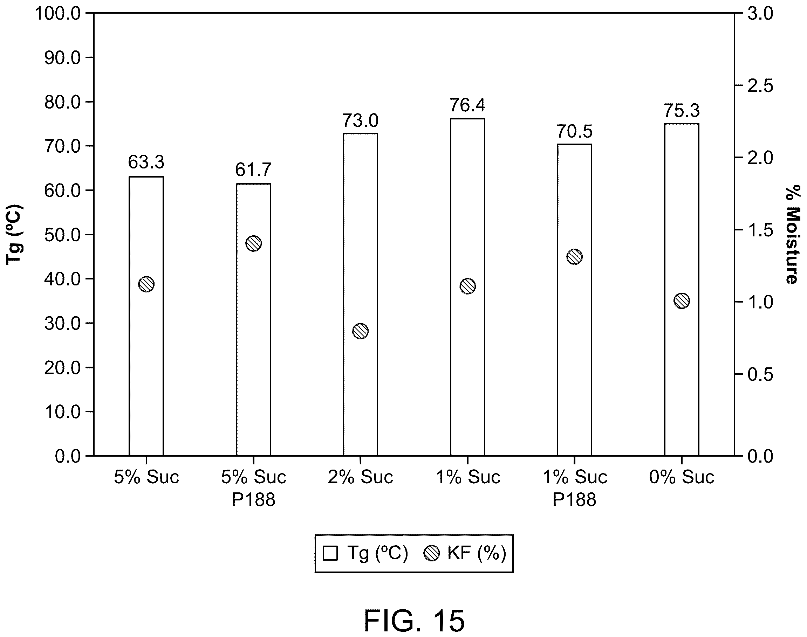

FIG. 15 shows the glass transition temperature (Tg) and residual moisture content (%) at the start of experiment (T0) for compositions of efanesoctocog alfa lyophilized drug product (Lyo DP) at 4000 IU. Lyo DP compositions containing either 0.05% (w/v) PS80 or 0.1% (w/v) poloxamer 188 and 0, 1, 2, or 5% (w/v) sucrose were tested.

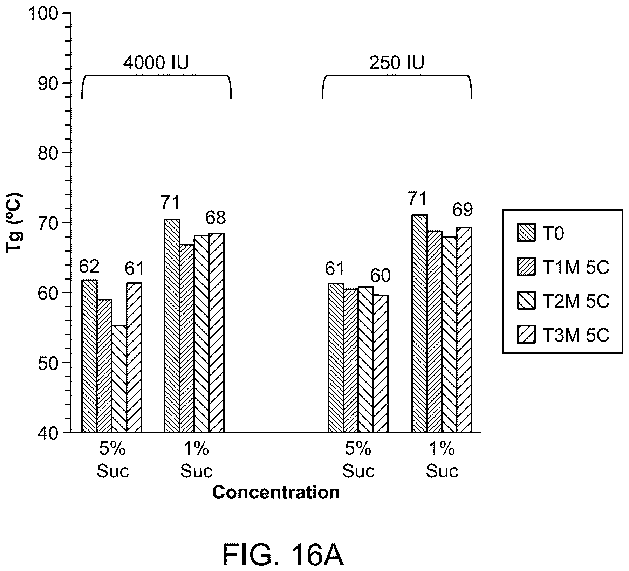

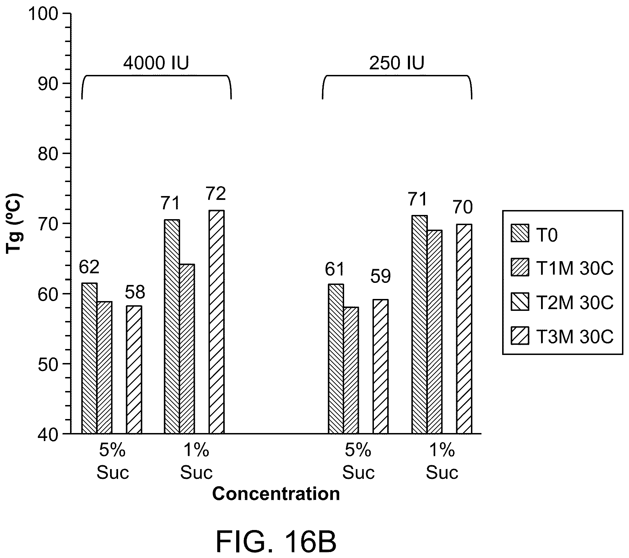

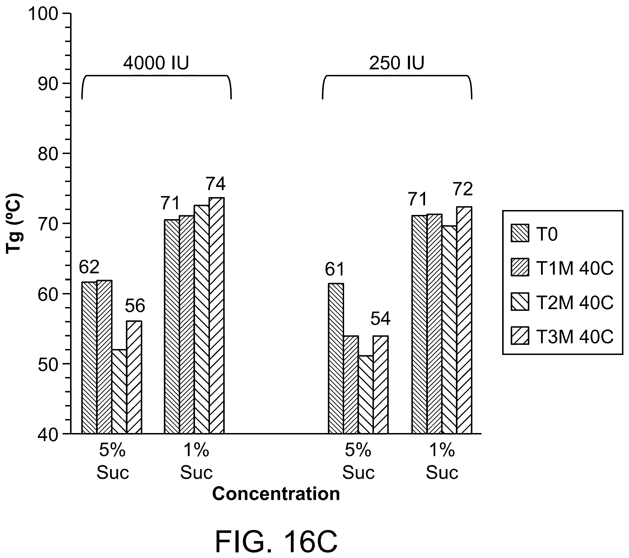

FIG. 16 shows the glass transition temperature (Tg) of efanesoctocog alfa lyophilized drug product (Lyo DP) compositions at 4000 IU or 250 IU and containing 0.1% (w/v) poloxamer 188 as a surfactant and either 5% or 1% (w/v) sucrose. Samples were held at 5°C, 30°C, or 40°C and tested at T0, 1 month, 2 months, 3 months, and 6 months. Results are shown in FIG. 16A (5°C), FIG. 16B (30°C), and FIG. 16C (40°C).

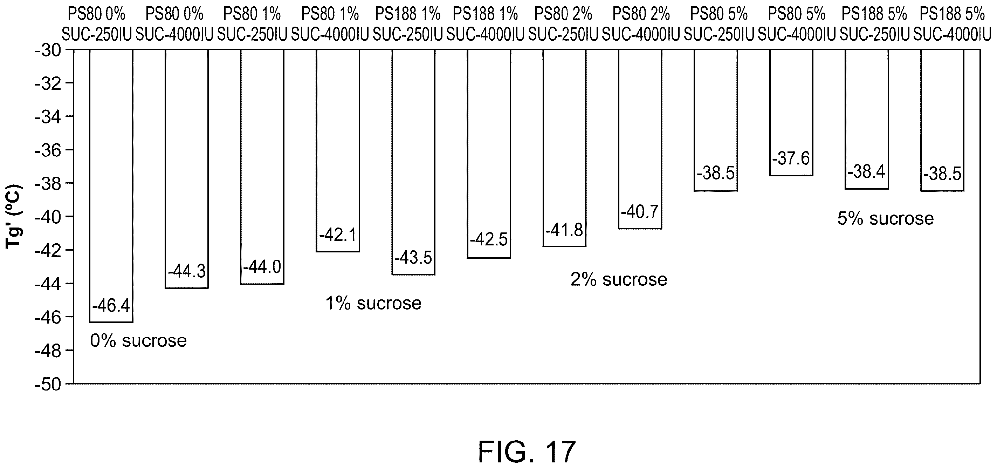

FIG. 17 shows the glass transition temperature (Tg) of efanesoctocog alfa liquid bulk drug product (BDP) compositions at 250 IU or 4000 IU and containing either 0.05% (w/v) PS80 or 0.1% (w/v) poloxamer 188 as a surfactant and 0, 1, 2, or 5% (w/v) sucrose.

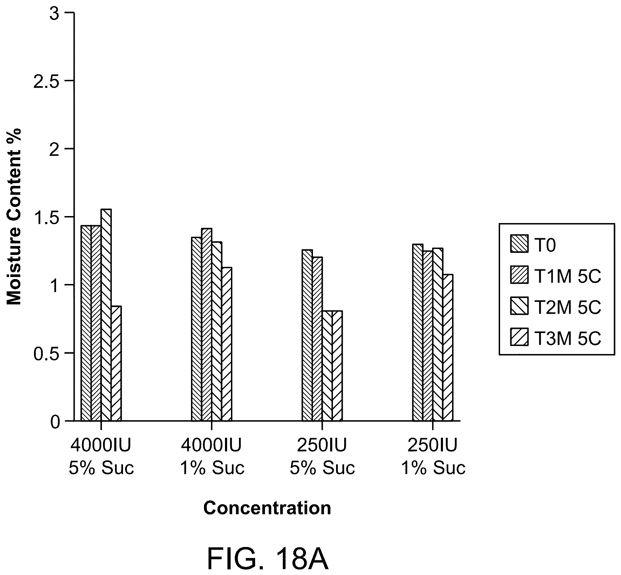

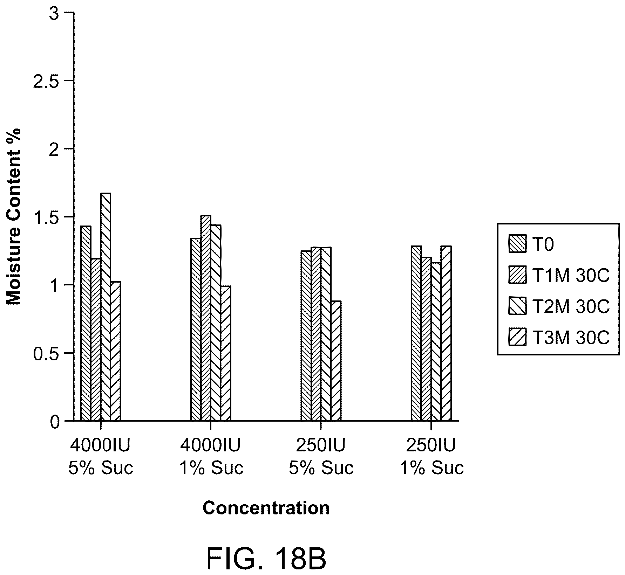

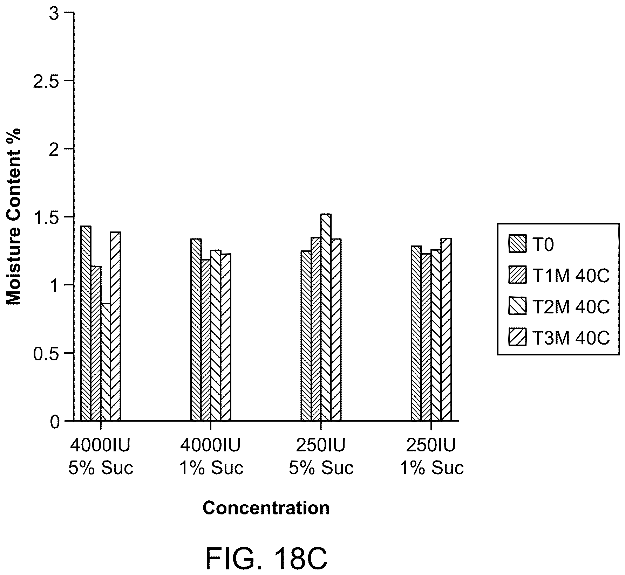

FIG. 18 shows the residual moisture content (%) of compositions of efanesoctocog alfa lyophilized drug product (Lyo DP) at 4000 IU or 250 IU. Lyo DP compositions containing 0.1% (w/v) poloxamer 188 as surfactant and 1% or 5% (w/v) sucrose were tested. Samples were held at 5°C, 30°C, or 40°C and tested at T0, 1 month, 2 months, 3 months, and 6 months. Results are shown in FIG. 18A (5°C), FIG. 18B (30°C), and FIG. 18C (40°C).

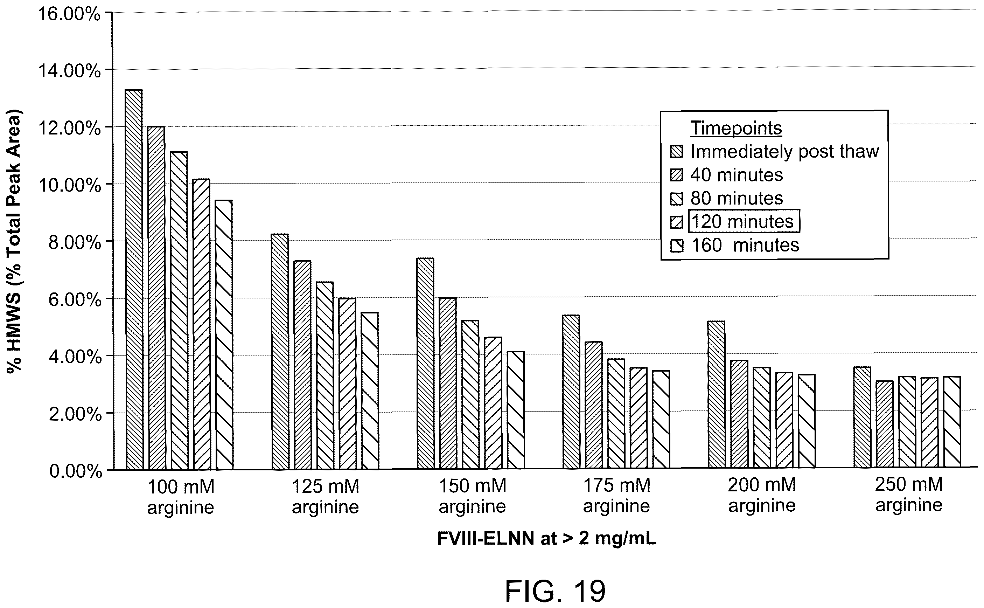

FIG. 19 shows the aggregation levels (%HMWS) after stress by freeze/thaw (F/T) for compositions of efanesoctocog alfa DS (concentration: >2 mg/mL) comprising increasing concentrations of arginine (Arg). Compositions contained Arginine concentrations tested were 100, 125, 150, 175, 200, and 250 mM. Each column represents repeat injections from the same vial after thawing for 30 minutes at RT. Time points measured were immediately post-thaw and 40, 80, 120, and 160 minutes post-thaw.

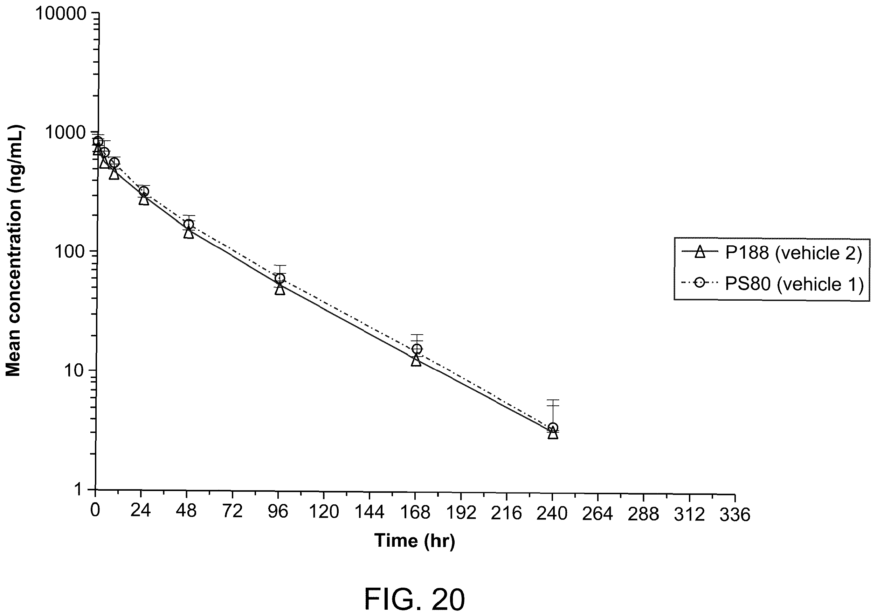

FIG. 20 shows the mean concentration (ng/mL) of efanesoctocog alfa over time as determined by ELISA following a single IV bolus injection of a composition comprising either PS80 or P188. Vehicle 1: 10mM L-Histidine, 250mM Arginine-HCl, 5mM calcium chloride, 5% w/v Sucrose, 0.05% Polysorbate-80. Vehicle 2: 10mM L-Histidine, 250mM Arginine-HCl, 5mM Calcium Chloride, 5% w/v Sucrose, 0.1% Poloxamer-188.

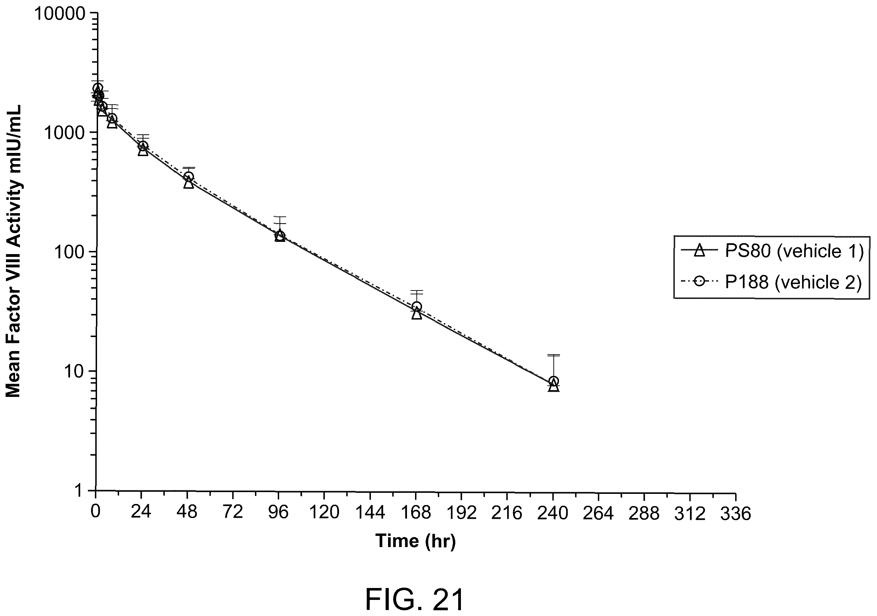

FIG. 21 shows the mean FVIII activity (mIU/mL) of efanesoctocog alfa over time as determined by chromogenic assay following a single IV bolus injection of a composition comprising either PS80 or P188. Vehicle 1: 10mM L-Histidine, 250mM Arginine-HCl, 5mM Calcium Chloride, 5% w/v Sucrose, 0.05% Polysorbate 80. Vehicle 2: 10mM L-Histidine, 250mM Arginine-HCl, 5mM Calcium Chloride, 5% w/v Sucrose, 0.1% Poloxamer 188.

### DETAILED DESCRIPTION OF THE DISCLOSURE

The present disclosure is directed to, *inter alia,* formulations (including aqueous and lyophilized formulations, as well as related kits) comprising FVIII proteins. In some embodiments, the FVIII protein is a chimeric FVIII protein such as efanesoctocog alfacomprising two polypeptides, i.e., a first polypeptide comprising a FVIII protein comprising a first ELNN Polypeptide sequence insert fused to a first Fc region, and a second polypeptide comprising a VWF protein fused to a second Ig constant region by a second ELNN Polypeptide sequence, wherein the first ELNN Polypeptide sequence contains about 288 amino acids and the second ELNN Polypeptide sequence contains about 144 amino acids, and the first Ig constant region and the second Ig constant region are covalently linked together by disulfide bonds.

The present disclosure is provides to formulations (including aqueous and lyophilized formulations, as well as related kits) for a chimeric protein chimeric protein comprising (i) a factor VIII (FVIII) polypeptide and (ii) a von Willebrand factor (VWF) fragment comprising a D' domain of VWF and a D3 domain of VWF. Included herein are compositions that may be lyophilized, as well as compositions formed upon reconstitution of lyophilized formulations with a diluent. Therapeutic methods and uses are also provided.

### I. Definitions

It is to be noted that the term "a" or "an" entity refers to one or more of that entity; for example, "a nucleotide sequence," is understood to represent one or more nucleotide sequences. As such, the terms "a" (or "an"), "one or more," and "at least one" can be used interchangeably herein.

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

It is understood that wherever aspects are described herein with the language "comprising," otherwise analogous aspects described in terms of "consisting of" and/or "consisting essentially of" are also provided.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, may provide one of skill with a general dictionary of many of the terms used in this disclosure.

Units, prefixes, and symbols are denoted in their Système International de Unites (SI) accepted form. Numeric ranges are inclusive of the numbers defining the range. Unless otherwise indicated, amino acid sequences are written left to right in amino to carboxy orientation. The headings provided herein are not limitations of the various aspects of the disclosure. Accordingly, the terms defined immediately below are more fully defined by reference to the specification in its entirety.

The term "about" is used herein to mean approximately, roughly, around, or in the regions of. When the term "about" is used in conjunction with a numerical range, it modifies that range by extending the boundaries above and below the numerical values set forth. In general, the term "about" can modify a numerical value above and below the stated value by a variance of, e.g., 10 percent, up or down (higher or lower). In some embodiments, the term indicates deviation from the indicated numerical value by ±10%, ±5%, ±4%, ±3%, ±2%, ±1%, ±0.9%, ±0.8%, ±0.7%, ±0.6%, ±0.5%, ±0.4%, ±0.3%, ±0.2%, ±0.1%, ±0.05%, or ±0.01%. In some embodiments, "about" indicates deviation from the indicated numerical value by ±10%. In some embodiments, "about" indicates deviation from the indicated numerical value by ±5%. In some embodiments, "about" indicates deviation from the indicated numerical value by ±4%. In some embodiments, "about" indicates deviation from the indicated numerical value by ±3%. In some embodiments, "about" indicates deviation from the indicated numerical value by ±2%. In some embodiments, "about" indicates deviation from the indicated numerical value by ±1%. In some embodiments, "about" indicates deviation from the indicated numerical value by ±0.9%. In some embodiments, "about" indicates deviation from the indicated numerical value by ±0.8%. In some embodiments, "about" indicates deviation from the indicated numerical value by ±0.7%. In some embodiments, "about" indicates deviation from the indicated numerical value by ±0.6%. In some embodiments, "about" indicates deviation from the indicated numerical value by ±0.5%. In some embodiments, "about" indicates deviation from the indicated numerical value by ±0.4%. In some embodiments, "about" indicates deviation from the indicated numerical value by ±0.3%. In some embodiments, "about" indicates deviation from the indicated numerical value by ±0.1%. In some embodiments, "about" indicates deviation from the indicated numerical value by ±0.05%. In some embodiments, "about" indicates deviation from the indicated numerical value by ±0.01%.

Depending on context, the term "polynucleotide" or "nucleotide" may encompass a singular nucleic acid as well as plural nucleic acids. In some embodiments, a polynucleotide is an isolated nucleic acid molecule or construct, e.g., messenger RNA (mRNA) or plasmid DNA (pDNA). In some embodiments, a polynucleotide comprises a conventional phosphodiester bond. In some embodiments, a polynucleotide comprises a non-conventional bond (e.g., an amide bond, such as found in peptide nucleic acids (PNA)). The term "nucleic acid" may refer to any one or more nucleic acid segments, e.g., DNA or RNA fragments, present in a polynucleotide. By "isolated" nucleic acid or polynucleotide is intended a nucleic acid molecule, DNA or RNA, which has been removed from its native environment. For example, a recombinant polynucleotide encoding a Factor VIII polypeptide contained in a vector is considered isolated for the purposes of the present disclosure. Further examples of an isolated polynucleotide include recombinant polynucleotides maintained in heterologous host cells or purified (partially or substantially) from other polynucleotides in a solution. Isolated RNA molecules include *in vivo* or *in vitro* RNA transcripts of polynucleotides of the present disclosure. Isolated polynucleotides or nucleic acids according to the present disclosure further include such molecules produced synthetically. In addition, a polynucleotide or a nucleic acid can include regulatory elements such as promoters, enhancers, ribosome binding sites, or transcription termination signals.

Certain proteins secreted by mammalian cells are associated with a secretory signal peptide which is cleaved from the mature protein once export of the growing protein chain across the rough endoplasmic reticulum has been initiated. Those of ordinary skill in the art are aware that signal peptides are generally fused to the N-terminus of the polypeptide, and are cleaved from the complete or "full-length" polypeptide to produce a secreted or "mature" form of the polypeptide. In some embodiments, a native signal peptide or a functional derivative of that sequence that retains the ability to direct the secretion of the polypeptide that is operably associated with it. Alternatively, a heterologous mammalian signal peptide, *e.g.*, a human tissue plasminogen activator (TPA) or mouse ß-glucuronidase signal peptide, or a functional derivative thereof, can be used.

As used herein, the term "polypeptide" is intended to encompass a singular "polypeptide" as well as plural "polypeptides," and refers to a molecule composed of monomers (amino acids) linearly linked by amide bonds (also known as peptide bonds). The term "polypeptide" refers to any chain or chains of two or more amino acids, and does not refer to a specific length of the product. Thus, peptides, dipeptides, tripeptides, oligopeptides, "protein," "amino acid chain," or any other term used to refer to a chain or chains of two or more amino acids, are included within the definition of "polypeptide," and the term "polypeptide" can be used instead of, or interchangeably with any of these terms. The term "polypeptide" is also intended to refer to the products of post-expression modifications of the polypeptide, including without limitation glycosylation, acetylation, phosphorylation, amidation, derivatization by known protecting/blocking groups, proteolytic cleavage, or modification by non-naturally occurring amino acids. A polypeptide can be derived from a natural biological source or produced recombinant technology, but is not necessarily translated from a designated nucleic acid sequence. It can be generated in any manner, including by chemical synthesis.

An "isolated" polypeptide or a fragment, variant, or derivative thereof refers to a polypeptide that is not in its natural milieu. No particular level of purification is required. For example, an isolated polypeptide can simply be removed from its native or natural environment. Recombinantly produced polypeptides and proteins expressed in host cells are considered isolated for the purpose of the disclosure, as are native or recombinant polypeptides which have been separated, fractionated, or partially or substantially purified by any suitable technique.

Also included in the present disclosure are fragments or variants of polypeptides, and any combination thereof. The term "fragment" or "variant" when referring to polypeptide binding domains or binding molecules of the present disclosure include any polypeptides which retain at least some of the properties (*e.g.*, FcRn binding affinity for an FcRn binding domain or Fc variant, coagulation activity for an FVIII variant, or FVIII binding activity for the VWF fragment) of the reference polypeptide. Fragments of polypeptides include proteolytic fragments, as well as deletion fragments, in addition to specific antibody fragments discussed elsewhere herein, but do not include the naturally occurring full-length polypeptide (or mature polypeptide). Variants of polypeptide binding domains or binding molecules of the present disclosure include fragments as described above, and also polypeptides with altered amino acid sequences due to amino acid substitutions, deletions, or insertions. Variants can be naturally or non-naturally occurring. Non-naturally occurring variants can be produced using art-known mutagenesis techniques. Variant polypeptides can comprise conservative or non-conservative amino acid substitutions, deletions or additions.

The term "VWF protein" as used herein means any VWF fragment that interacts with FVIII and retains at least one or more properties that are normally provided to FVIII by full-length VWF, e.g., preventing premature activation to FVIlla, preventing premature proteolysis, preventing clearance, preventing association with phospholipid membranes that could lead to premature clearance, preventing binding to FVIII clearance receptors that can bind naked FVIII but not VWF-bound FVIII, and/or stabilizing the FVIII heavy chain and light chain interactions. A VWF fragment referred to herein is a VWF polypeptide that is less than the full-length VWF protein, wherein the VWF fragment retains the ability to interact with and/or bind to FVIII. In some embodiments, a VWF protein is a fragment (which may be mutated) of full-length VWF that binds to a FVIII protein such that the FVIII protein has reduced binding to, or does not bind, full length VWF (e.g., endogenous VWF in a subject).

A "conservative amino acid substitution" is one in which the amino acid residue is replaced with an amino acid residue having a similar side chain. Families of amino acid residues having similar side chains have been defined in the art, including basic side chains (*e.g.*, lysine, arginine, histidine), acidic side chains (*e.g.*, aspartic acid, glutamic acid), uncharged polar side chains (*e.g.*, glycine, asparagine, glutamine, serine, threonine, tyrosine, cysteine), nonpolar side chains (*e.g.*, alanine, valine, leucine, isoleucine, proline, phenylalanine, methionine, tryptophan), beta-branched side chains (*e.g.*, threonine, valine, isoleucine) and aromatic side chains (*e.g.*, tyrosine, phenylalanine, tryptophan, histidine). Thus, if an amino acid in a polypeptide is replaced with another amino acid from the same side chain family, the substitution is considered to be conservative. In some embodiments, a string of amino acids can be conservatively replaced with a structurally similar string that differs in order and/or composition of side chain family members.

As known in the art, "sequence identity" between two polypeptides is determined by comparing the amino acid sequence of one polypeptide to the sequence of a second polypeptide. Similarly, "sequence identity" between two polynucleotides is determined by comparing the nucleotide sequence of one polynucleotide to the sequence of a second polynucleotide. The terms "% identical", "% identity" or similar terms are intended to refer, in particular, to the percentage of nucleotides or amino acids (as applicable) which are identical in an optimal alignment between the sequences to be compared. Said percentage is purely statistical, and the differences between the two sequences may be but are not necessarily randomly distributed over the entire length of the sequences to be compared. Comparisons of two sequences are usually carried out by comparing the sequences, after optimal alignment, with respect to a segment or "window of comparison", in order to identify local regions of corresponding sequences. For example, the optimal alignment for a comparison may be carried out manually or with the aid of the local homology algorithm by Smith and Waterman, 1981, Ads App. Math. 2, 482, with the aid of the local homology algorithm by Neddleman and Wunsch, 1970, J. Mol. Biol. 48, 443, with the aid of the similarity search algorithm by Pearson and Lipman, 1988, Proc. Natl Acad. Sci. USA 88, 2444, or with the aid of computer programs using said algorithms (GAP, BESTFIT, FASTA, BLAST P, BLAST N and TFASTA in Wisconsin Genetics Software Package, Genetics Computer Group, 575 Science Drive, Madison, Wis.). In some embodiments, percent identity of two sequences is determined using the BLASTN or BLASTP algorithm, as available on the United States National Center for Biotechnology Information (NCBI) website (e.g, at blast.ncbi.nlm.nih.gov/Blast.cgi?PAGE_TYPE=BlastSearch&BLAST_SPEC=blast2seq&L INK_LOC=align2seq). In some embodiments, the algorithm parameters used for BLASTN algorithm on the NCBI website include: (i) Expect Threshold set to 10; (ii) Word Size set to 28; (iii) Max matches in a query range set to 0; (iv) Match/Mismatch Scores set to 1, -2; (v) Gap Costs set to Linear; and (vi) the filter for low complexity regions being used. In some embodiments, the algorithm parameters used for BLASTP algorithm on the NCBI website include: (i) Expect Threshold set to 10; (ii) Word Size set to 3; (iii) Max matches in a query range set to 0; (iv) Matrix set to BLOSUM62; (v) Gap Costs set to Existence: 11 Extension: 1; and (vi) conditional compositional score matrix adjustment. When discussed herein, whether any particular polypeptide is at least about 50%, 60%, 70%, 75%, 80%, 85%, 90%, 95%, 99%, or 100% identical to another polypeptide can be determined using methods and computer programs/software known in the art such as, but not limited to, the BESTFIT program (Wisconsin Sequence Analysis Package, Version 8 for Unix, Genetics Computer Group, University Research Park, 575 Science Drive, Madison, WI 53711). BESTFIT uses the local homology algorithm of Smith and Waterman, Advances in Applied Mathematics 2:482-489 (1981), to find the best segment of homology between two sequences. When using BESTFIT or any other sequence alignment program to determine whether a particular sequence is, for example, 95% identical to a reference sequence according to the present disclosure, the parameters are set, of course, such that the percentage of identity is calculated over the full-length of the reference polypeptide sequence and that gaps in homology of up to 5% of the total number of amino acids in the reference sequence are allowed.

As used herein, an "amino acid corresponding to" or an "equivalent amino acid" in a VWF sequence or a FVIII protein sequence is identified by alignment to maximize the identity or similarity between a first VWF or FVIII sequence and a second VWF or FVIII sequence. The number used to identify an equivalent amino acid in a second VWF or FVIII sequence is based on the number used to identify the corresponding amino acid in the first VWF or FVIII sequence.

As used herein, the term "insertion site" refers to a position in a FVIII polypeptide, or fragment, variant, or derivative thereof, which is immediately downstream of the position at which a half-life extending moiety or heterologous moiety can be inserted. An "insertion site" is specified as a number, the number being the number of the amino acid in mature native FVIII (SEQ ID NO: 9) to which the insertion site corresponds, which is immediately C-terminal to the position of the insertion. For example, the phrase "comprises an ELNN Polypeptide at an insertion site which corresponds to amino acid 1656 of SEQ ID NO: 9" indicates that the heterologous moiety is located between two amino acids corresponding to amino acid 1656 and amino acid 1657 of SEQ ID NO: 9.

The phrase "immediately downstream of an amino acid" as used herein refers to position right next to the terminal carboxyl group of the amino acid. For example, an insertion site immediately downstream of amino acid 745 corresponding to the mature wild type FVIII protein (SEQ ID NO: 9) means that the insertion site is between amino acid 745 and amino acid 746 corresponding to the mature wild type FVIII protein. Similarly, the phrase "immediately upstream of an amino acid" refers to the position right next to the terminal amine group of the amino acid.

The phrase "between two amino acids of an insertion site" as used herein refers to a position in which an ELNN Polypeptide or any other polypeptide is inserted between two adjacent amino acids. Thus, the phrases "inserted immediately downstream of an amino acid" and "inserted between two amino acids of an insertion site" are used synonymously with "inserted at an insertion site."

The terms "inserted," "is inserted," "inserted into" or grammatically related terms, as used herein with respect to insertions of ELNN Polypeptide into FVIII refers to the position of an ELNN Polypeptide in a chimeric protein relative to the analogous position in native mature human FVIII. As used herein the terms refer to the characteristics of the recombinant FVIII polypeptide relative to native mature human FVIII, and do not indicate, imply or infer any methods or process by which the chimeric protein was made. For example, in reference to a chimeric protein provided herein, the phrase "an ELNN Polypeptide is inserted immediately downstream of residue 745 of the FVIII polypeptide" means that the chimeric protein comprises an ELNN Polypeptide immediately downstream of an amino acid which corresponds to amino acid 745 in native mature human FVIII, e.g., bounded by amino acids corresponding to amino acids 745 and 746 of native mature human FVIII (without requiring the presence of an amino acid corresponding to 746 of native mature human FVIII), and does not connote an order or method of production for which the chimeric protein was constructed.

As used herein, the terms "ELNN Polypeptide" and "ELNN" are synonymous, and refer to extended length polypeptides with non-naturally occurring, substantially non-repetitive sequences that are composed mainly of small hydrophilic amino acids, with the sequence having a low degree or no secondary or tertiary structure under physiologic conditions. ELNNs can confer certain desirable pharmacokinetic, physicochemical and pharmaceutical properties when linked to a VWF protein or a FVIII sequence of the disclosure to create a chimeric polypeptide. Such desirable properties include but are not limited to enhanced pharmacokinetic parameters and solubility characteristics. As used herein, the terms "ELNN Polypeptide" and "ELNN" specifically exclude antibodies or antibody fragments such as single-chain antibodies or Fc fragments of a light chain or a heavy chain. ELNN polypeptides are known in the art, and non-limiting descriptions relating to and examples of ELNN polypeptides known as XTEN^{®} polypeptides are available in Schellenberger et al., (2009) Nat Biotechnol 27(12):1186-90; Brandl et al., (2020) Journal of Controlled Release 327:186-197; and Radon et al., (2021) Advanced Functional Materials 31, 2101633 (pages 1-33), the entire contents of each of which are incorporated herein by reference.

A "fusion" or "chimeric" protein comprises a first amino acid sequence linked to a second amino acid sequence with which it is not naturally linked in nature. The amino acid sequences which normally exist in separate proteins can be brought together in the fusion polypeptide, or the amino acid sequences which normally exist in the same protein can be placed in a new arrangement in the fusion polypeptide, e.g., fusion of a Factor VIII domain of the disclosure with an Ig Fc domain. A fusion protein is created, for example, by chemical synthesis, or by creating and translating a polynucleotide in which the peptide regions are encoded in the desired relationship. A chimeric protein can further comprise a second amino acid sequence associated with the first amino acid sequence by a covalent, non-peptide bond or a non-covalent bond.

With respect to sequences, the term "linked" as used herein refers to a first amino acid sequence or nucleotide sequence covalently or non-covalently joined to a second amino acid sequence or nucleotide sequence, respectively. The first amino acid or nucleotide sequence can be directly joined or juxtaposed to the second amino acid or nucleotide sequence or alternatively an intervening sequence can covalently join the first sequence to the second sequence. Depending on context, the term "linked" means not only a fusion of a first amino acid sequence to a second amino acid sequence at the C-terminus or the N-terminus, but also includes insertion of the whole first amino acid sequence (or the second amino acid sequence) into any two amino acids in the second amino acid sequence (or the first amino acid sequence, respectively). In some embodiments, the first amino acid sequence can be linked to a second amino acid sequence by a peptide bond or a linker. The first nucleotide sequence can be linked to a second nucleotide sequence by a phosphodiester bond or a linker. The linker can be a peptide or a polypeptide (for polypeptide chains) or a nucleotide or a nucleotide chain (for nucleotide chains) or any chemical moiety (for both polypeptide and polynucleotide chains). The term "linked" may also be indicated by a hyphen (-).

With respect to two polypeptides, the term "associated with" refers to one or more covalent or non-covalent bonds formed between a first polypeptide and a second polypeptide. In some embodiments, the term "associated with" means a covalent, non-peptide bond or a non-covalent bond. This association can be indicated by a colon, i.e., (:). In some embodiments, it means a covalent bond except a peptide bond. For example, the amino acid cysteine comprises a thiol group that can form a disulfide bond or bridge with a thiol group on a second cysteine residue. In most naturally occurring IgG molecules, the CH1 and CL regions are associated by a disulfide bond and the two heavy chains are associated by two disulfide bonds at positions corresponding to 239 and 242 using the Kabat numbering system (position 226 or 229, EU numbering system). Examples of covalent bonds include, but are not limited to, a peptide bond, a metal bond, a hydrogen bond, a disulfide bond, a sigma bond, a pi bond, a delta bond, a glycosidic bond, an agnostic bond, a bent bond, a dipolar bond, a Pi backbond, a double bond, a triple bond, a quadruple bond, a quintuple bond, a sextuple bond, conjugation, hyperconjugation, aromaticity, hapticity, or antibonding. Non-limiting examples of non-covalent bond include an ionic bond (*e.g.*, cation-pi bond or salt bond), a metal bond, a hydrogen bond (*e.g.*, dihydrogen bond, dihydrogen complex, low-barrier hydrogen bond, or symmetric hydrogen bond), van der Walls force, London dispersion force, a mechanical bond, a halogen bond, aurophilicity, intercalation, stacking, entropic force, or chemical polarity. In some embodiments, the one or more covalent bonds between the first amino acid chain and the second amino acid chain is two disulfide bonds. In some embodiments, the one or more covalent bonds between the first amino acid chain and the second amino acid chain is two disulfide bonds between a first Fc portion on the first amino acid chain and a second Fc portion on the second amino acid chain, wherein the two disulfide bonds occur in the hinge region of the two Fc portions.

In some embodiments, a polypeptide has an enzymatic cleavage site cleaved by an enzyme that is activated during the clotting cascade, such that cleavage of such sites occurs at the site of clot formation. Exemplary such sites include, e.g., those recognized by thrombin, Factor XIa or Factor Xa. SEQ ID NO: 9SEQ ID NO: 15Other enzymatic cleavage sites are known in the art and described in elsewhere herein. In constructs that include more than one processing or cleavage site, it will be understood that such sites can be the same or different. As used herein, the term "half-life" refers to a biological half-life of a particular polypeptide *in vivo.* Half-life can be represented by the time required for half the quantity administered to a subject to be cleared from the circulation and/or other tissues in the animal. In some embodiments, when a clearance curve of a given polypeptide is constructed as a function of time, the curve is usually biphasic with a rapid α-phase and longer β-phase. The α-phase typically represents an equilibration of the administered Fc polypeptide between the intra- and extra-vascular space and is, in part, determined by the size of the polypeptide. The β-phase typically represents the catabolism of the polypeptide in the intravascular space. In some embodiments, FVIII and chimeric proteins comprising FVIII are monophasic, and thus do not have an alpha phase, but just the single beta phase. Therefore, in some embodiments, the term half-life as used herein refers to the half-life of the polypeptide in the β-phase. The typical beta phase half-life of a human antibody in humans is 21 days. In some embodiments, the half-life is expressed as the half-life of the terminal phase.

In some embodiments, a subject has hemophilia A. In some embodiments, the hemophilia A is severe hemophilia A.

"Administer" or "administering," as used herein refers to delivering to a subject a composition described herein, e.g., a chimeric protein. The composition, e.g., the chimeric protein, can be administered to a subject using methods known in the art. In particular, the composition can be administered intravenously, subcutaneously, intramuscularly, intradermally, or via any mucosal surface, e.g., orally, sublingually, buccally, nasally, rectally, vaginally or via pulmonary route. In some embodiments, the administration is intravenous. In some embodiments, the administration is subcutaneous. In some embodiments, the administration is self-administration. In some embodiments, a parent administers the chimeric protein to a child. In some embodiments, the chimeric protein is administered to a subject by a healthcare practitioner such as a medical doctor, a medic, or a nurse.

As used herein, the term "dose" refers to a single administration of a composition to a subject. A single dose can be administered all at once, e.g., as a bullous, or over a period of time, e.g., via an intravenous infusion. The term "multiple doses" means more than one dose, e.g., more than one administration.

When referring to co-administration of more than one composition, a dose of composition A can be administered concurrently with a dose of composition B. Alternatively, a dose of composition A can be administered before or after a dose of composition B. In some embodiments, composition A and composition B are combined into a single formulation.

As used herein, the term "interval" or "dosing interval" refers to the amount of time that elapses between a first dose of composition A and a subsequent dose of the same composition administered to a subject. A dosing interval can refer to the time that elapses between a first dose and a second dose, or a dosing interval can refer to the amount of time that elapses between multiple doses.

The term "dosing frequency" as used herein refers to the number of doses administered per a specific dosing interval. For example, a dosing frequency can be written as once a week, once every two weeks, etc. Therefore, a dosing interval of 7 days can be also written as a dosing interval of once in 7 days or once every week, or once a week.

As used herein the term "prophylactic treatment" refers to the administration of a therapy for the treatment of hemophilia A, where such treatment is intended to prevent or reduce the severity of one or more symptoms of hemophilia A, e.g., bleeding episodes, e.g., one or more spontaneous bleeding episodes, and/or joint damage. See Jimenez-Yuste et al., Blood Transfus. 12(3):314-19 (2014). To prevent or reduce the severity of such symptoms, e.g., bleeding episodes and the progression of joint disease, hemophilia A patients may receive regular infusions of clotting factor as part of a prophylactic treatment regimen. The basis of such prophylactic treatment is the observation that hemophilia patients with a clotting factor, e.g., FVIII, level of 1% or more rarely experience spontaneous bleeding episodes and have fewer hemophilia-related comorbidities as compared to patients with severe hemophilia. See, e.g., Coppola A. et al, Semin. Thromb. Hemost. 38(1): 79-94 (2012). Health care practitioners treating these hemophilia patients surmised that maintaining factor levels at around 1% with regular infusions could potentially reduce the risk of hemophilia symptoms, including bleeding episodes and joint damage. See id. Subsequent research has confirmed these benefits in pediatric hemophilia patients receiving prophylactic treatment with clotting factor, rendering prophylactic treatment the goal for people with severe hemophilia. See id.

A "prophylactic" treatment can also refer to the preemptive administration of the composition described herein, e.g., a protein (such as a chimeric protein), to a subject in order to control, manage, prevent, or reduce the occurrence or severity of one or more symptoms of hemophilia A, e.g., bleeding episodes. In some embodiments, prophylactic treatment with a clotting factor, e.g., FVIII, is used to treat subjects with severe hemophilia A. In some embodiments, prophylactic treatment refers to administering a composition disclosed herein to a subject in need thereof to reduce the occurrence of one or more symptom of hemophilia A. A prophylactic treatment can include administration of multiple doses. The multiple doses used in prophylactic treatment are typically administered at particular dosing intervals. In some embodiments, the annualized bleeding rate can be reduced to less than 10, less than 9, less than 8, less than 7, less than 6, less than 5, less than 4, less than 3, less than 2, or less than 1.

The term "on-demand treatment" or "episodic treatment" refers to the "as needed" administration of a chimeric molecule in response to symptoms of hemophilia A, e.g., a bleeding episode, or before an activity that can cause bleeding. In some aspects, the on-demand treatment can be given to a subject when bleeding starts, such as after an injury, or when bleeding is expected, such as before surgery. In some aspects, the on-demand treatment can be given prior to activities that increase the risk of bleeding, such as contact sports. In some embodiments, the on-demand treatment is given as a single dose. In some embodiments, the on-demand treatment is given as a first dose, followed by one or more additional doses. When the chimeric protein is administered on-demand, the one or more additional doses can be administered at least about 12 hours, at least about 24 hours, at least about 36 hours, at least about 48 hours, at least about 60 hours, at least about 72 hours, at least about 84 hours, at least about 96 hours, at least about 108 hours, or at least about 120 hours after the first dose. It should be noted, however, that the dosing interval associated with on-demand treatment is not the same as the dosing interval used for prophylactic treatment.

In some embodiments, the subject in need of a general hemostatic agent is undergoing, or is about to undergo, surgery. The chimeric protein of the disclosure can be administered prior to or after surgery. The chimeric protein of the disclosure can also be administered during or after surgery to control an acute bleeding episode. When the chimeric protein is administered prior to surgery, the administration can be at least about 1 hour, at least about 2 hours, at least about 4 hours, at least about 8 hours, at least about 12 hours, at least about 24 hours, at least about 36 hours, at least about 48 hours, or at least about 72 hours prior to surgery. When the chimeric protein is administered to after surgery, the administration can be at least about 1 hour, at least about 2 hours, at least about 4 hours, at least about 8 hours, at least about 12 hours, at least about 24 hours, at least about 36 hours, at least about 48 hours, or at least about 72 hours after surgery. The surgery can include, but is not limited to, liver transplantation, liver resection, dental procedures, or stem cell transplantation.

"Treat", "treatment", "treating", as used herein refers to, e.g., the reduction in severity of a disease or condition; the reduction in the duration of a disease course; the amelioration of one or more symptoms associated with a disease or condition; the provision of beneficial effects to a subject with a disease or condition, without necessarily curing the disease or condition, or the prophylaxis of one or more symptoms associated with a disease or condition. In some embodiments, treating or treatment comprises maintaining a FVIII trough level at least about 1 IU/dL, 2 IU/dL, 3 IU/dL, 4 IU/dL, 5 IU/dL, 6 IU/dL, 7 IU/dL, 8 IU/dL, 9 IU/dL, 10 IU/dL, 11 IU/dL, 12 IU/dL, 13 IU/dL, 14 IU/dL, 15 IU/dL, 16 IU/dL, 17 IU/dL, 18 IU/dL, 19 IU/dL, or 20 IU/dL in a subject by administering a chimeric protein of the disclosure. As used herein, a "trough level" in a hemophilia A patient is the measurement of the lowest concentration reached by a factor therapy, e.g., a FVIII therapy, before the next dose is administered. In some embodiments, treating or treatment means maintaining a FVIII trough level of at least about 1 IU/dL between the dosing interval. In some embodiments, treating or treatment means maintaining a FVIII trough level of at least about 3 IU/dL between the dosing interval. In some embodiments, treating or treatment means maintaining a FVIII trough level of at least about 5 IU/dL between the dosing interval. In some embodiments, treating or treatment means maintaining a FVIII trough level between about 1 and about 20 IU/dL, about 2 and about 20 IU/dL, about 3 and about 20 IU/dL, about 4 and about 20 IU/dL, about 5 and about 20 IU/dL, about 6 and about 20 IU/dL, about 7 and about 20 IU/dL, about 8 and about 20 IU/dL, about 9 and about 20 IU/dL, or about 10 and about 20 IU/dL during the dosing interval.

In some embodiments, treatment or treating of a disease or condition comprises maintaining FVIII activity in a subject at a level comparable to at least about 1%, 2%, 3%, 4%, 5%, 6%, 7%, 8%, 9%, 10%, 11%, 12%, 13%, 14%, 15%, 16%, 17%, 18%, 19%, or 20% of the FVIII activity in a non-hemophiliac subject between the dosing interval. In some embodiments, treating or treatment means maintaining a FVIII activity level of at least about 1% between the dosing interval. In some embodiments, treating or treatment means maintaining a FVIII activity level of at least about 2% between the dosing interval. In some embodiments, treating or treatment means maintaining a FVIII activity level of at least about 3% between the dosing interval. In some embodiments, treating or treatment means maintaining a FVIII activity level of at least about 4% between the dosing interval. In some embodiments, treating or treatment means maintaining a FVIII activity level of at least about 5%. between the dosing interval. In some embodiments, treating or treatment means maintaining a FVIII activity level of at least about 6% between the dosing interval. In some embodiments, treating or treatment means maintaining a FVIII activity level of at least about 7% between the dosing interval. In some embodiments, treating or treatment means maintaining a FVIII activity level of at least about 8% between the dosing interval. In some embodiments, treating or treatment means maintaining a FVIII activity level of at least about 9% between the dosing interval. In some embodiments, treating or treatment means maintaining a FVIII activity level of at least about 10% between the dosing interval. In some embodiments, the minimum trough level required for treatment can be measured by one or more known methods (for example, the activated partial thromboplastin time (aPTT) assays or chromogenic assays, which are well known in the art) and can be adjusted (increased or decreased) for each person. Non-limiting examples of assays for measuring trough level are disclosed in U.S. Application Publication No. 20190375822, which is hereby incorporated by reference in its entirety.

### II. Chimeric Proteins

In an aspect, the present disclosure is directed to pharmaceutical compositions comprising a chimeric protein or protein which comprises a first polypeptide chain which comprises a Factor VIII ("FVIII") protein or a portion thereof and a first immunoglobulin ("Ig") constant region or a portion thereof, and a second polypeptide chain which comprises a von Willebrand Factor ("VWF") protein and a second Ig constant region or a portion thereof. In some embodiments, the chimeric protein comprises (i) a FVIII protein comprising a FVIII polypeptide, an ELNN Polypeptide inserted within the B domain of the FVIII polypeptide, and a first Fc region; and (ii) a VWF protein comprising a VWF fragment, a second ELNN Polypeptide sequence, an a2 linker, and a second Fc region. In some embodiments, the chimeric protein disclosed herein is a FVIII-ELNN-Fc/D'D3-ELNN-Fc heterodimer.

In some embodiments, the chimeric protein is efanesoctocog alfa. Efanesoctocog alfa, also known as "BIVV001", "efanesoctocogum alfa", "rFVIIIFc-VWF-ELNN", and "rFVIIIFc-VWF-XTEN", is described in Chhabra et al. Blood 135(17): 1484-1496 (2020), the entire contents of which are hereby incorporated by reference in its entirety. A schematic representation of efanesoctocog alfa, as an exemplary FVIII-ELNN-Fc/D'D3-ELNN-Fc heterodimer, is presented in FIG. 1.

Efanesoctocog alfa is a large protein (over 300 kDa) comprising multiple parts on each of two polypeptide chains that are associated by covalent bonds and non-covalent interactions. The protein has a tendency to aggregate under certain conditions, which can reduce the stability of formulations unless an excipient such as L-arginine is selected and present in an amount that is sufficient to reduce the aggregation. For example, the aggregation may be reduced by adding high levels of L-arginine (e.g., about 250 mM).

Additional information regarding efanesoctocog alfa can be found in the International Nonproprietary Names for Pharmaceutical Substances (INN) WHO Drug Information, 2019, Vol. 33, No. 4, p.828-30. In some embodiments, the chimeric protein is a FVIII-ELNN-Fc/D'D3-ELNN-Fc heterodimer comprising (i) a first polypeptide comprising the amino acid sequence of SEQ ID NO: 1 and (ii) a second polyleptide comprising the amino acid sequence of SEQ ID NO: 2. In some embodiments, the chimeric protein comprises (i) a first polypeptide and (ii) a second polypeptide that are covalently linked via one or more disulfide bonds (e.g., two disulfide bonds). In some embodiments, the chimeric protein comprises a FVIII protein encoded by the nucleic acid sequence of SEQ ID NO: 4. In some embodiments, the chimeric protein comprises a VWF protein encoded by the nucleic acid sequence of SEQ ID NO: 6. In some emodiments, the efanesoctocog alfa has an activity of at least 1600 IU/mg. In some emodiments, the efanesoctocog alfa has an activity of at least 1700 IU/mg. In some emodiments, the efanesoctocog alfa has an activity of at least 1800 IU/mg. In some emodiments, the efanesoctocog alfa has an activity of at least 1900 IU/mg. In some emodiments, the efanesoctocog alfa has an activity of 1600 IU/mg to 2000 IU/mg.

In some embodiments, the chimeric protein comprises a FVIII protein comprising the amino acid sequence of SEQ ID NO: 1. In some embodiments, the chimeric protein comprises a FVIII protein comprising one or more disulfide bridges at one or more of the following locations: residues 153-179, 248-329, 528-554, 630-711, 1220-1246, 1287-1291, 1409-1557, 1562-1714, 1761-1821, and/or 1867-1925 of SEQ ID NO: 1. In some embodiments, the chimeric protein comprises a FVIII protein comprising one or more disulfide bridges at each of the following locations: residues 153-179, 248-329, 528-554, 630-711, 1220-1246, 1287-1291, 1409-1557, 1562-1714, 1761-1821, and 1867-1925 of SEQ ID NO: 1. In some embodiments, the chimeric protein comprises a FVIII protein comprising one or more Cys-SH residues at residues 310, 692, and/or 1388 of SEQ ID NO: 1. In some embodiments, the chimeric protein comprises a FVIII protein comprising a Cys-SH residues at each of residues 310, 692, and/or 1388 of SEQ ID NO: 1.

In some embodiments, the chimeric protein comprises a FVIII protein comprises one or more N-glycosylation sites at residues N41, N239, N1198, N1506, and/or N1797 of SEQ ID NO: 1. In some embodiments, the chimeric protein comprises a FVIII protein comprises one or more O-glycosylation sites at residues 746-1036 of SEQ ID NO: 1 and/or the Ser and Thr residues in the linker peptides. In some embodiments, the chimeric protein comprises a FVIII protein comprises one or more Tyr-sulfation sites at residues 346, 718, 719, 723, 729, 1052, and/or 1068 of SEQ ID NO: 1.

In some embodiments, the chimeric protein comprises a VWF protein comprising the amino acid sequence of SEQ ID NO: 2. In some embodiments, the chimeric protein comprises a VWF protein comprising one or more disulfide bridges at one or more of the following locations: residues 4-45, 13-41, 25-36, 29-64, 47-58, 66-88, 83-100, 86-95, 104-233, 126-268, 135-230, 151-158, 283-326, 297-321, 308-348, 328-334, 338-363, 367-410, 386-406, 390-402, 394-433, 414-427, 436-464, 459-474, 462-471, 698-758, and/or 804-862 of SEQ ID NO: 2. In some embodiments, the chimeric protein comprises a VWF protein comprising one or more disulfide bridges at each of the following locations: residues 4-45, 13-41, 25-36, 29-64, 47-58, 66-88, 83-100, 86-95, 104-233, 126-268, 135-230, 151-158, 283-326, 297-321, 308-348, 328-334, 338-363, 367-410, 386-406, 390-402, 394-433, 414-427, 436-464, 459-474, 462-471, 698-758, and/or 804-862 of SEQ ID NO: 2.

In some embodiments, the chimeric protein comprises a VWF protein comprises one or more N-glycosylation sites at residues N94, N384, N734 of SEQ ID NO: 2. In some embodiments, the chimeric protein comprises a VWF protein comprises one or more O-glycosylation sites at residues 478-625 of SEQ ID NO: 2 and/or the Ser and Thr residues in the linker peptides. In some embodiments, the chimeric protein comprises a VWF protein comprises one or more Tyr-sulfation sites at residues 632, 633, 637, and/or 643 of SEQ ID NO: 2.In some embodiments, the VWF protein comprises a VWF fragment comprising a D1, D2, D', and/or D3 domain of VWF. In one embodiment, the VWF fragment comprises a D1D2 region of VWF comprising the amino acid sequence of SEQ ID NO: 20. In some embodiments, the VWF protein further comprises a VWF signal peptide sequence. In one embodiment, the VWF signal peptide comprises the amino acid sequence of SEQ ID NO: 19. In one specific embodiment, the VWF protein comprises a VWF signal peptide comprising the amino acid sequence of SEQ ID NO: 19, a D1D2 region of VWF comprising the amino acid sequence of SEQ ID NO: 20, a D' domain of VWF comprising the amino acid sequence of SEQ ID NO: 21, a D3 domain of VWF comprising the amino acid sequence of SEQ ID NO: 22, an ELNN Polypeptide sequence comprising the amino acid sequence of SEQ ID NO: 14 (AE144_5A), an a2 linker comprising the amino acid sequence of SEQ ID NO: 15, and/or a Fc region comprising the amino acid sequence of SEQ ID NO: 23.

In some embodiments, the chimeric protein of the present disclosure comprises: (i) a FVIII protein comprising a FVIII polypeptide, a first ELNN Polypeptide sequence, and a first Fc region; and (ii) a VWF fragment comprising a D' domain of VWF and a D3 domain of VWF, a second ELNN Polypeptide sequence, an a2 linker of FVIII, and a second Fc region; wherein: the FVIII protein has a deletion of amino acids 746 to 1648 corresponding to mature FVIII; the first ELNN Polypeptide sequence is inserted within the FVIII polypeptide immediately downstream of amino acid 745 corresponding to mature FVIII; the first ELNN Polypeptide sequence comprises an amino acid sequence having at least about 70%, at least about 75%, at least about 80%, at least about 85%, at least about 90%, at least about 95%, at least about 96%, at least about 97%, at least about 98%, at least about 99%, or about 100% sequence identity to the amino acid sequence of AE288 (SEQ ID NO: 9); the first Fc region is fused to the C-terminus of the FVIII polypeptide; the second ELNN Polypeptide sequence is fused to the C-terminus of the VWF fragment; the second ELNN Polypeptide sequence comprises an amino acid sequence having at least about 70%, at least about 75%, at least about 80%, at least about 85%, at least about 90%, at least about 95%, at least about 96%, at least about 97%, at least about 98%, at least about 99%, or about 100% sequence identity to the amino acid sequence of AE144_5A (SEQ ID NO: 14); the a2 linker is fused to the C-terminus of the ELNN Polypeptide; the a2 linker comprises an amino acid sequence having at least about 70%, at least about 75%, at least about 80%, at least about 85%, at least about 90%, at least about 95%, at least about 96%, at least about 97%, at least about 98%, at least about 99%, or about 100% sequence identity to the amino acid sequence of SEQ ID NO: 9; the second Fc region is fused to the C-terminus of the a2 linker; and the first Fc region is covalently linked to the second Fc region by a disulfide bond(e.g., two disulfide bonds).

In some embodiments, the chimeric protein of the disclosure comprises two polypeptide sequences, a first polypeptide sequence comprising an amino acid sequence at least about 80%, 90%, 95%, or 100% identical to the amino acid sequence set forth in SEQ ID NO: 1; and a second polypeptide sequence comprising a VWF fragment comprising a D' domain of VWF and a D3 domain of VWF and an Fc region. In some embodiments, the chimeric protein of the disclosure comprises two polypeptide sequences, a first polypeptide sequence comprising FVIII polypeptide and an Fc region; and a second polypeptide sequence comprising an amino acid sequence at least about 80%, 90%, 95%, or 100% identical to the amino acid sequence set forth in SEQ ID NO: 2. In some embodiments, the chimeric protein of the disclosure comprises two polypeptide sequences, a first polypeptide sequence comprising an amino acid sequence at least about 80%, 90%, 95%, or 100% identical to the amino acid sequence set forth in SEQ ID NO: 1 and a second polypeptide sequence comprising an amino acid sequence at least about 80%, 90%, 95%, or 100% identical to the amino acid sequence set forth in SEQ ID NO: 2. In some embodiments, the chimeric protein of the disclosure comprises two polypeptide sequences, a first polypeptide sequence comprising the amino acid sequence set forth in SEQ ID NO: 7 and a second polypeptide sequence comprising the amino acid sequence set forth in SEQ ID NO: 2. In some embodiments, the chimeric protein of the disclosure comprises two polypeptide sequences, a first polypeptide sequence comprising the amino acid sequence set forth in SEQ ID NO: 1 and a second polypeptide sequence comprising the amino acid sequence set forth in SEQ ID NO: 2, wherein the first polypeptide sequence and the second polypeptide sequence are linked to each other by a disulfide bond. In some embodiments, the chimeric protein of the disclosure comprises two polypeptide sequences, a first polypeptide sequence comprising the amino acid sequence set forth in SEQ ID NO: 1 and a second polypeptide sequence comprising the amino acid sequence set forth in SEQ ID NO: 2, wherein the first polypeptide sequence and the second polypeptide sequence are linked to each other by two disulfide bonds. In some embodiments, the chimeric protein of the disclosure comprises two polypeptide sequences, a first polypeptide sequence comprising the amino acid sequence set forth in SEQ ID NO: 1 and a second polypeptide sequence comprising the amino acid sequence set forth in SEQ ID NO: 2, wherein the first polypeptide sequence comprises a first Fc portion, wherein the second polypeptide sequence comprises a second Fc portion, wherein the first Fc portion and the second Fc portion are linked to each other by two disulfide bonds in the hinge region.

In some embodiments, the chimeric protein of the disclosure comprises a FVIII protein comprising an amino acid sequence at least about 80%, 90%, 95%, or 100% identical to SEQ ID NO: 7, SEQ ID NO: 3, or SEQ ID NO: 1; and a VWF protein comprising an amino acid sequence at least about 80%, 90%, 95%, or 100% identical to SEQ ID NO: 2 or SEQ ID NO: 5.

In some embodiments, the chimeric protein of the disclosure comprises: (i) a FVIII protein comprising a first FVIII polypeptide fragment comprising the amino acid sequence of SEQ ID NO: 17; a first ELNN Polypeptide sequence comprising the amino acid sequence of SEQ ID NO: 9 (AE288); a second FVIII polypeptide fragment comprising the amino acid sequence of SEQ ID NO: 18; and a first Fc region comprising the amino acid sequence of SEQ ID NO: 23; and (ii) a VWF protein comprising: a D' domain of VWF comprising the amino acid sequence of SEQ ID NO: 21; a D3 domain of VWF comprising the amino acid sequence of SEQ ID NO: 22; a second ELNN Polypeptide sequence comprising the amino acid sequence of SEQ ID NO: 14 (AE144_5A); an a2 linker comprising the amino acid sequence of SEQ ID NO: 15; and a second Fc region comprising the amino acid sequence of SEQ ID NO: 23, and wherein the first Fc region is covalently linked to the second Fc region by a disulfide bond (e.g., two disulfide bonds).

In some embodiments, the chimeric protein of the disclosure comprises a FVIII protein comprising a FVIII polypeptide, a first ELNN Polypeptide sequence, a first Fc region, and a VWF protein comprising a D' domain of VWF, a D3 domain of VWF, a second ELNN Polypeptide sequence, an a2 linker of FVIII and a second Fc region, wherein the FVIII polypeptide comprises the amino acid sequence of SEQ ID NO: 17, the first ELNN Polypeptide sequence comprises the amino acid sequence of AE288 (SEQ ID NO: 9) and is fused to the C-terminus of SEQ ID NO: 17, the FVIII polypeptide further comprises the amino acid sequence of SEQ ID NO: 18, the first Fc region comprises the amino acid sequence of SEQ ID NO: 23 and is fused to the C-terminus of SEQ ID NO: 18; the D' domain of VWF comprises the amino acid sequence of SEQ ID NO: 21; the D3 domain of VWF comprises the amino acid sequence of SEQ ID NO: 214, the second ELNN Polypeptide sequence comprises the amino acid sequence of AE144_5A (SEQ ID NO: 14) and is fused to the C-terminus of the D3 domain of VWF; the a2 linker comprises the amino acid sequence of SEQ ID NO: 15 and is fused to the C-terminus of the second ELNN Polypeptide sequence; the second Fc region comprises the amino acid sequence of SEQ ID NO: 23 and is fused to the C-terminus of the a2 linker; and wherein the first Fc region is covalently linked to the second Fc region by a disulfide bond.

In some embodiments, the chimeric protein of the disclosure comprises a FVIII protein comprising a FVIII signal peptide comprising the amino acid sequence of SEQ ID NO: 16. In some embodiments, the chimeric protein comprises a VWF protein comprising a VWF signal peptide comprising the amino acid sequence of SEQ ID NO: 19. In some embodiments, the chimeric protein comprises a VWF protein comprising a D1D2 domain of VWF comprising the amino acid sequence of SEQ ID NO: 20.

In some embodiments, the chimeric protein comprises a first polypeptide comprising the amino acid sequence of SEQ ID NO: 3 and a second polypeptide comprising the amino acid sequence of SEQ ID NO: 5. In some embodiments, the chimeric protein comprises a first polypeptide comprising the amino acid sequence of SEQ ID NO: 7 and a second polypeptide comprising the amino acid sequence of SEQ ID NO: 2. In some embodiments, the chimeric protein comprises a first polypeptide comprising the amino acid sequence of SEQ ID NO: 1 and a second polypeptide comprising the amino acid sequence of SEQ ID NO: 2.

In some embodiments, the chimeric protein comprises one or more disulfide bridges between the first polypeptide and the second polypeptide. In some embodiments, the chimeric protein comprises two disulfide bridges between the first polypeptide and the second polypeptide. In some embodiments, the chimeric protein comprises a first polypeptide comprising the amino acid sequence of SEQ ID NO: 1 and a second polypeptide comprising the amino acid sequence of SEQ ID NO: 2, wherein the chimeric protein comprises a disulfide bridge between residue 1726 of SEQ ID NO: 1 and residue 663 of SEQ ID NO: 2, and a disulfide bridge between residue 1729 of SEQ ID NO: 1 and residue 666 of SEQ ID NO: 2.

### IV. Pharmaceutical Compositions

In an aspect, the present disclosure is directed to pharmaceutical compositions of a chimeric protein which are formulated to improve protein stability. In some embodiments, the disclosed pharmaceutical compositions demonstrate increased stability based on analysis by visual inspection, protein concentration, pH stability, formation of high molecular weight species (HMWS), and/or change in turbidity. Analysis of these properties of stability can be made using conventional techniques, including size exclusion chromatography (SEC), reversed-phase high-performance liquid chromatography (RP-HPLC), and many others.

The pharmaceutical compositions disclosed herein comprise a specified amount of the chimeric protein. In some embodiments, the pharmaceutical composition has a chimeric protein concentration of about 0.8 to about 1.2 mg/mL. In some embodiments, the pharmaceutical composition has a chimeric protein concentration of about 0.8 mg/mL. In some embodiments, the pharmaceutical composition has a chimeric protein concentration of about 0.9 mg/mL. In some embodiments, the pharmaceutical composition has a chimeric protein concentration of about 1.0 mg/mL. In some embodiments, the pharmaceutical composition has a chimeric protein concentration of about 1.1 mg/mL. In some embodiments, the pharmaceutical composition has a chimeric protein concentration of about 1.2 mg/mL.

In some embodiments, the pharmaceutical composition comprises about 75 IU/mL to about 2,000 IU/mL of the chimeric protein. In some embodiments, the pharmaceutical composition comprises about 75 IU/mL of the chimeric protein. In some embodiments, the pharmaceutical composition comprises about 100 IU/mL of the chimeric protein. In some embodiments, the pharmaceutical composition comprises about 150 IU/mL of the chimeric protein. In some embodiments, the pharmaceutical composition comprises about 200 IU/mL of the chimeric protein. In some embodiments, the pharmaceutical composition comprises about 250 IU/mL of the chimeric protein. In some embodiments, the pharmaceutical composition comprises about 300 IU/mL of the chimeric protein. In some embodiments, the pharmaceutical composition comprises about 350 IU/mL of the chimeric protein. In some embodiments, the pharmaceutical composition comprises about 400 IU/mL of the chimeric protein. In some embodiments, the pharmaceutical composition comprises about 450 IU/mL of the chimeric protein. In some embodiments, the pharmaceutical composition comprises about 500 IU/mL of the chimeric protein. In some embodiments, the pharmaceutical composition comprises about 550 IU/mL of the chimeric protein. In some embodiments, the pharmaceutical composition comprises about 600 IU/mL of the chimeric protein. In some embodiments, the pharmaceutical composition comprises about 650 IU/mL of the chimeric protein. In some embodiments, the pharmaceutical composition comprises about 700 IU/mL of the chimeric protein. In some embodiments, the pharmaceutical composition comprises about 750 IU/mL of the chimeric protein. In some embodiments, the pharmaceutical composition comprises about 800 IU/mL of the chimeric protein. In some embodiments, the pharmaceutical composition comprises about 850 IU/mL of the chimeric protein. In some embodiments, the pharmaceutical composition comprises about 900 IU/mL of the chimeric protein. In some embodiments, the pharmaceutical composition comprises about 950 IU/mL of the chimeric protein. In some embodiments, the pharmaceutical composition comprises about 1000 IU/mL of the chimeric protein. In some embodiments, the pharmaceutical composition comprises more than about 250 IU of the chimeric protein. In some embodiments, the pharmaceutical composition comprises more than about 300 IU of the chimeric protein. In some embodiments, the pharmaceutical composition comprises more than about 500 IU of the chimeric protein. In some embodiments, the pharmaceutical composition comprises at least about 500 IU of the chimeric protein. In some embodiments, the pharmaceutical composition comprises more than about 85 IU/ml of the chimeric protein. In some embodiments, the pharmaceutical composition comprises more than about 100 IU/ml of the chimeric protein. In some embodiments, the pharmaceutical composition comprises more than about 200 IU/ml of the chimeric protein. In some embodiments, the pharmaceutical composition comprises at least about 200 IU/ml of the chimeric protein.

In some embodiments, the pharmaceutical composition comprises about 1100 IU/mL of the chimeric protein. In some embodiments, the pharmaceutical composition comprises about 1150 IU/mL of the chimeric protein. In some embodiments, the pharmaceutical composition comprises about 1200 IU/mL of the chimeric protein. In some embodiments, the pharmaceutical composition comprises about 1250 IU/mL of the chimeric protein. In some embodiments, the pharmaceutical composition comprises about 1300 IU/mL of the chimeric protein. In some embodiments, the pharmaceutical composition comprises about 1350 IU/mL of the chimeric protein. In some embodiments, the pharmaceutical composition comprises about 1400 IU/mL of the chimeric protein. In some embodiments, the pharmaceutical composition comprises about 1450 IU/mL of the chimeric protein. In some embodiments, the pharmaceutical composition comprises about 1500 IU/mL of the chimeric protein. In some embodiments, the pharmaceutical composition comprises about 1550 IU/mL of the chimeric protein. In some embodiments, the pharmaceutical composition comprises about 1600 IU/mL of the chimeric protein. In some embodiments, the pharmaceutical composition comprises about 1650 IU/mL of the chimeric protein. In some embodiments, the pharmaceutical composition comprises about 1700 IU/mL of the chimeric protein. In some embodiments, the pharmaceutical composition comprises about 1750 IU/mL of the chimeric protein. In some embodiments, the pharmaceutical composition comprises about 1800 IU/mL of the chimeric protein. In some embodiments, the pharmaceutical composition comprises about 1850 IU/mL of the chimeric protein. In some embodiments, the pharmaceutical composition comprises about 1900 IU/mL of the chimeric protein. In some embodiments, the pharmaceutical composition comprises about 1950 IU/mL of the chimeric protein. In some embodiments, the pharmaceutical composition comprises about 2000 IU/mL of the chimeric protein.

Pharmaceutical compositions containing the chimeric protein of the present disclosure also contain a suitable pharmaceutically acceptable carrier. For example, they can contain excipients and/or auxiliaries that provide enhanced stability of the chimeric protein or facilitate processing of the active compounds into preparations designed for delivery to the site of action.

In an aspect, disclosed herein are pharmaceutical compositions comprising a specified amount of a chimeric protein along with excipients as disclosed. The pharmaceutical compositions disclosed herein comprise various concentrations of these excipients as disclosed, and the concentrations can be expressed in various ways. For example, the concentration of a given excipient can be expressed as a molar concentration (e.g., M or mM), as a weight/volume percent, (e.g., grams per 100 ml diluent), or as milligrams per milliliter (mg/ml). Pharmaceutical compositions provided herein can contain specified amounts of the various excipients at a level of precision ranging from approximate, e.g., concentrations expressed only to one significant figure (e.g., about 0.1% (w/v)), or with more precision, e.g., out to 2, 3, 4, 5, or 6 significant figures (e.g., about 3.88 mg/ml, with precision out to three significant figures). The necessary level of precision can vary depending on, e.g., the requirements of a given regulatory agency, or the manufacturing process.

In some embodiments, the pharmaceutical composition comprises 1% (w/v) to 4% (w/v) sucrose. In some embodiments, the pharmaceutical composition comprises 2% (w/v) to 5% (w/v) sucrose. In some embodiments, the pharmaceutical composition comprises 4% (w/v) to 8% (w/v) sucrose. In some embodiments, the pharmaceutical composition comprises about 1.5% (w/v) to about 2.5% (w/v) sucrose. In some embodiments, the pharmaceutical composition comprises about 5% (w/v) to about 7.5% (w/v) sucrose.

In some embodiments, the pharmaceutical composition comprises about 1.0% (w/v) sucrose. In some embodiments, the pharmaceutical composition comprises about 1.1% (w/v) sucrose. In some embodiments, the pharmaceutical composition comprises about 1.2% (w/v) sucrose. In some embodiments, the pharmaceutical composition comprises about 1.3% (w/v) sucrose. In some embodiments, the pharmaceutical composition comprises about 1.4% (w/v) sucrose. In some embodiments, the pharmaceutical composition comprises about 1.5% (w/v) sucrose. In some embodiments, the pharmaceutical composition comprises about 1.6% (w/v) sucrose. In some embodiments, the pharmaceutical composition comprises about 1.7% (w/v) sucrose. In some embodiments, the pharmaceutical composition comprises about 1.8% (w/v) sucrose. In some embodiments, the pharmaceutical composition comprises about 1.9% (w/v) sucrose. In some embodiments, the pharmaceutical composition comprises about 2.0% (w/v) sucrose. In some embodiments, the pharmaceutical composition comprises about 2.1% (w/v) sucrose. In some embodiments, the pharmaceutical composition comprises about 2.2% (w/v) sucrose. In some embodiments, the pharmaceutical composition comprises about 2.3% (w/v) sucrose. In some embodiments, the pharmaceutical composition comprises about 2.4% (w/v) sucrose. In some embodiments, the pharmaceutical composition comprises about 2.5% (w/v) sucrose. In some embodiments, the pharmaceutical composition comprises about 2.6% (w/v) sucrose. In some embodiments, the pharmaceutical composition comprises about 2.7% (w/v) sucrose. In some embodiments, the pharmaceutical composition comprises about 2.8% (w/v) sucrose. In some embodiments, the pharmaceutical composition comprises about 2.9% (w/v) sucrose. In some embodiments, the pharmaceutical composition comprises about 3.0% (w/v) sucrose. In some embodiments, the pharmaceutical composition comprises about 3.1% (w/v) sucrose. In some embodiments, the pharmaceutical composition comprises about 3.2% (w/v) sucrose. In some embodiments, the pharmaceutical composition comprises about 3.3% (w/v) sucrose. In some embodiments, the pharmaceutical composition comprises about 3.4% (w/v) sucrose. In some embodiments, the pharmaceutical composition comprises about 3.5% (w/v) sucrose. In some embodiments, the pharmaceutical composition comprises about 3.6% (w/v) sucrose. In some embodiments, the pharmaceutical composition comprises about 3.7% (w/v) sucrose. In some embodiments, the pharmaceutical composition comprises about 3.8% (w/v) sucrose. In some embodiments, the pharmaceutical composition comprises about 3.9% (w/v) sucrose. In some embodiments, the pharmaceutical composition comprises about 4.0% (w/v) sucrose. In some embodiments, the pharmaceutical composition comprises about 4.1% (w/v) sucrose. In some embodiments, the pharmaceutical composition comprises about 4.2% (w/v) sucrose. In some embodiments, the pharmaceutical composition comprises about 4.3% (w/v) sucrose. In some embodiments, the pharmaceutical composition comprises about 4.4% (w/v) sucrose. In some embodiments, the pharmaceutical composition comprises about 4.5% (w/v) sucrose. In some embodiments, the pharmaceutical composition comprises about 4.4% (w/v) sucrose. In some embodiments, the pharmaceutical composition comprises about 4.7% (w/v) sucrose. In some embodiments, the pharmaceutical composition comprises about 4.8% (w/v) sucrose. In some embodiments, the pharmaceutical composition comprises about 4.9% (w/v) sucrose. In some embodiments, the pharmaceutical composition comprises about 5% (w/v) sucrose. In some embodiments, the pharmaceutical composition comprises about 5.1% (w/v) sucrose. In some embodiments, the pharmaceutical composition comprises about 5.2% (w/v) sucrose. In some embodiments, the pharmaceutical composition comprises about 5.3% (w/v) sucrose. In some embodiments, the pharmaceutical composition comprises about 5.4% (w/v) sucrose. In some embodiments, the pharmaceutical composition comprises about 5.5% (w/v) sucrose. In some embodiments, the pharmaceutical composition comprises about 5.6% (w/v) sucrose. In some embodiments, the pharmaceutical composition comprises about 5.7% (w/v) sucrose. In some embodiments, the pharmaceutical composition comprises about 5.8% (w/v) sucrose. In some embodiments, the pharmaceutical composition comprises about 5.9% (w/v) sucrose. In some embodiments, the pharmaceutical composition comprises about 6% (w/v) sucrose. In some embodiments, the pharmaceutical composition comprises about 6.1% (w/v) sucrose. In some embodiments, the pharmaceutical composition comprises about 6.2% (w/v) sucrose. In some embodiments, the pharmaceutical composition comprises about 6.3% (w/v) sucrose. In some embodiments, the pharmaceutical composition comprises about 6.4% (w/v) sucrose. In some embodiments, the pharmaceutical composition comprises about 6.5% (w/v) sucrose. In some embodiments, the pharmaceutical composition comprises about 6.6% (w/v) sucrose. In some embodiments, the pharmaceutical composition comprises about 6.7% (w/v) sucrose. In some embodiments, the pharmaceutical composition comprises about 6.8% (w/v) sucrose. In some embodiments, the pharmaceutical composition comprises about 6.9% (w/v) sucrose. In some embodiments, the pharmaceutical composition comprises about 7% (w/v) sucrose. In some embodiments, the pharmaceutical composition comprises about 7.1% (w/v) sucrose. In some embodiments, the pharmaceutical composition comprises about 7.2% (w/v) sucrose. In some embodiments, the pharmaceutical composition comprises about 7.3% (w/v) sucrose. In some embodiments, the pharmaceutical composition comprises about 7.4% (w/v) sucrose. In some embodiments, the pharmaceutical composition comprises about 7.5% (w/v) sucrose. In some embodiments, the pharmaceutical composition comprises about 7.6% (w/v) sucrose. In some embodiments, the pharmaceutical composition comprises about 7.7% (w/v) sucrose. In some embodiments, the pharmaceutical composition comprises about 7.8% (w/v) sucrose. In some embodiments, the pharmaceutical composition comprises about 7.9% (w/v) sucrose. In some embodiments, the pharmaceutical composition comprises about 8% (w/v) sucrose.

In some embodiments, the pharmaceutical composition comprises about 33.67 mg sucrose. In some embodiments, the pharmaceutical composition comprises about 67.34 mg sucrose. In some embodiments, the pharmaceutical composition comprises about 168.35 mg sucrose. In some embodiments, the amount of sucrose can vary up to 10% of a specific amount. In some embodiments, the amount of sucrose can vary up to 5% of a specific amount. In some embodiments, the amount of sucrose can vary up to 1% of a specific amount. In some embodiments, the specific amount of sucrose is 33.67 mg, 67.34 mg, or 168.35 mg.

Pharmaceutical compositions disclosed herein may include a buffer. In some embodiments, the pharmaceutical compositions disclosed herein include specified amounts or concentrations of histidine. In some embodiments, the histidine included in the pharmaceutical composition is L-histidine. In some embodiments, the pharmaceutical composition comprises about 5 mM to about 15 mM histidine.

In some embodiments, the pharmaceutical composition comprises about 5 mM histidine. In some embodiments, the pharmaceutical composition comprises about 5.5 mM histidine. In some embodiments, the pharmaceutical composition comprises about 6 mM histidine. In some embodiments, the pharmaceutical composition comprises about 6.5 mM histidine. In some embodiments, the pharmaceutical composition comprises about 7 mM histidine. In some embodiments, the pharmaceutical composition comprises about 7.5 mM histidine. In some embodiments, the pharmaceutical composition comprises about 8 mM histidine. In some embodiments, the pharmaceutical composition comprises about 8.5 mM histidine. In some embodiments, the pharmaceutical composition comprises about 9 mM histidine. In some embodiments, the pharmaceutical composition comprises about 9.5 mM histidine. In some embodiments, the pharmaceutical composition comprises about 10 mM histidine. In some embodiments, the pharmaceutical composition comprises about 10.5 mM histidine. In some embodiments, the pharmaceutical composition comprises about 11 mM histidine. In some embodiments, the pharmaceutical composition comprises about 11.5 mM histidine. In some embodiments, the pharmaceutical composition comprises about 12 mM histidine. In some embodiments, the pharmaceutical composition comprises about 12.5 mM histidine. In some embodiments, the pharmaceutical composition comprises about 13 mM histidine. In some embodiments, the pharmaceutical composition comprises about 13.5 mM histidine. In some embodiments, the pharmaceutical composition comprises about 14 mM histidine. In some embodiments, the pharmaceutical composition comprises about 14.5 mM histidine. In some embodiments, the pharmaceutical composition comprises about 15 mM histidine. In some embodiments, the histidine is L-histidine.

In some embodiments, the pharmaceutical compositions disclosed herein include specified amounts or concentrations of arginine. In some embodiments, the the pharmaceutical composition comprises arginine hydrochloride (HCl). In some embodiments, the arginine is L-arginine. In some embodiments, the composition comprises L-arginine-HCI.

In some embodiments, the pharmaceutical composition comprises about 200 mM to about 300 mM arginine.

In some embodiments, the pharmaceutical composition comprises about 200 mM arginine. In some embodiments, the pharmaceutical composition comprises about 210 mM arginine. In some embodiments, the pharmaceutical composition comprises about 220 mM arginine. In some embodiments, the pharmaceutical composition comprises about 230 mM arginine. In some embodiments, the pharmaceutical composition comprises about 240 mM arginine. In some embodiments, the pharmaceutical composition comprises about 250 mM arginine. In some embodiments, the pharmaceutical composition comprises about 260 mM arginine. In some embodiments, the pharmaceutical composition comprises about 270 mM arginine. In some embodiments, the pharmaceutical composition comprises about 280 mM arginine. In some embodiments, the pharmaceutical composition comprises about 290 mM arginine. In some embodiments, the pharmaceutical composition comprises about 300 mM arginine. In some embodiments, the arginine is L-arginine. In some embodiments, the composition comprises L-arginine-HCI.

Pharmaceutical compositions disclosed herein may include a bulking agent. In some embodiments, the pharmaceutical compositions disclosed herein include specified amounts or concentrations of calcium chloride (CaCl₂). In some embodiments, the composition comprises CaCl₂●2H₂O, CaCl₂ (anhydrous), CaCl₂●4H₂O, or CaCl₂●6H₂O. In some embodiments, the composition comprises calcium chloride dihydrate. In some embodiments, the pharmaceutical composition comprises about 2.5 mM to about 10 mM calcium chloride. In some embodiments, the composition comprises calcium chloride dihydrate.

In some embodiments, the pharmaceutical composition comprises about 2.5 mM calcium chloride. In some embodiments, the pharmaceutical composition comprises about 3 mM calcium chloride. In some embodiments, the pharmaceutical composition comprises about 3.5 mM calcium chloride. In some embodiments, the pharmaceutical composition comprises about 4 mM calcium chloride. In some embodiments, the pharmaceutical composition comprises about 4.5 mM calcium chloride. In some embodiments, the pharmaceutical composition comprises about 5 mM calcium chloride. In some embodiments, the pharmaceutical composition comprises about 5.5 mM calcium chloride. In some embodiments, the pharmaceutical composition comprises about 6 mM calcium chloride. In some embodiments, the pharmaceutical composition comprises about 6.5 mM calcium chloride. In some embodiments, the pharmaceutical composition comprises about 7 mM calcium chloride. In some embodiments, the pharmaceutical composition comprises about 7.5 mM calcium chloride. In some embodiments, the pharmaceutical composition comprises about 8 mM calcium chloride. In some embodiments, the pharmaceutical composition comprises about 8.5 mM calcium chloride. In some embodiments, the pharmaceutical composition comprises about 9 mM calcium chloride. In some embodiments, the pharmaceutical composition comprises about 9.5 mM calcium chloride. In some embodiments, the pharmaceutical composition comprises about 10 mM calcium chloride. In some embodiments, the composition comprises calcium chloride dihydrate.

In some embodiments, the pharmaceutical compositions disclosed herein do not include bulking agents other than calcium chloride. In some embodiments, calcium chloride is the sole bulking agent. In some embodiments, the pharmaceutical composition comprises less than 8.8 mg/mL sodium chloride (NaCl). In some embodiments, the pharmaceutical composition is substantially free of sodium chloride. In some embodiments, the pharmaceutical composition is free of sodium chloride.

In some embodiments, the pharmaceutical compositions disclosed herein include specified amounts or concentrations of poloxamer 188 (P188). In some embodiments, the poloxamer is poloxamer 101, 105, 108, 122, 123, 124, 181, 182, 183, 184, 185, 188, 212, 215, 217, 231, 234, 235, 237, 238, 282, 284, 288, 331, 333, 334, 335, 338, 401, 402, 403, or 407. In some embodiments, the poloxamer is poloxamer 407. In some embodiments, the pharmaceutical composition comprises about .01 mg/ml to about 10 mg/ml of a poloxamer. In some embodiments, the pharmaceutical composition comprises at least about 1 mg/ml of a poloxamer.

In some embodiments, the pharmaceutical compositions disclosed herein include specified amounts or concentrations of poloxamer 188 (P188). Non-limiting disclosures relating to P188 may be found in Strickley and Lambert (2021) Journal of Pharmaceutical Sciences 110 2590-2608, the entire contents of each of which are incorporated herein by reference. In some embodiments, the pharmaceutical composition comprises about 0.01% (w/v) to about 1.0% (w/v) poloxamer 188. In some embodiments, the pharmaceutical composition comprises at least about 0.1% poloxamer 188. In some embodiments, the pharmaceutical composition comprises about 0.1% poloxamer 188.

In some embodiments, the pharmaceutical composition comprises about 0.01% (w/v) poloxamer 188. In some embodiments, the pharmaceutical composition comprises about 0.02% (w/v) poloxamer 188. In some embodiments, the pharmaceutical composition comprises about 0.03% (w/v) poloxamer 188. In some embodiments, the pharmaceutical composition comprises about 0.04% (w/v) poloxamer 188. In some embodiments, the pharmaceutical composition comprises about 0.05% (w/v) poloxamer 188. In some embodiments, the pharmaceutical composition comprises about 0.06% (w/v) poloxamer 188. In some embodiments, the pharmaceutical composition comprises about 0.07% (w/v) poloxamer 188. In some embodiments, the pharmaceutical composition comprises about 0.08% (w/v) poloxamer 188. In some embodiments, the pharmaceutical composition comprises about 0.09% (w/v) poloxamer 188. In some embodiments, the pharmaceutical composition comprises about 0.1% (w/v) poloxamer 188.

In some embodiments, the pharmaceutical composition comprises about 0.11% (w/v) poloxamer 188. In some embodiments, the pharmaceutical composition comprises about 0.12% (w/v) poloxamer 188. In some embodiments, the pharmaceutical composition comprises about 0.13% (w/v) poloxamer 188. In some embodiments, the pharmaceutical composition comprises about 0.14% (w/v) poloxamer 188. In some embodiments, the pharmaceutical composition comprises about 0.15% (w/v) poloxamer 188. In some embodiments, the pharmaceutical composition comprises about 0.16% (w/v) poloxamer 188. In some embodiments, the pharmaceutical composition comprises about 0.17% (w/v) poloxamer 188. In some embodiments, the pharmaceutical composition comprises about 0.18% (w/v) poloxamer 188. In some embodiments, the pharmaceutical composition comprises about 0.19% (w/v) poloxamer 188. In some embodiments, the pharmaceutical composition comprises about 0.2% (w/v) poloxamer 188.

In some embodiments, the pharmaceutical composition comprises about 0.3% (w/v) poloxamer 188. In some embodiments, the pharmaceutical composition comprises about 0.4% (w/v) poloxamer 188. In some embodiments, the pharmaceutical composition comprises about 0.5% (w/v) poloxamer 188. In some embodiments, the pharmaceutical composition comprises about 0.6% (w/v) poloxamer 188. In some embodiments, the pharmaceutical composition comprises about 0.7% (w/v) poloxamer 188. In some embodiments, the pharmaceutical composition comprises about 0.8% (w/v) poloxamer 188. In some embodiments, the pharmaceutical composition comprises about 0.9% (w/v) poloxamer 188. In some embodiments, the pharmaceutical composition comprises about 1.0% (w/v) poloxamer 188.

In some embodiments, the pharmaceutical composition is a pre-lyophilization solution. In some embodiments, pre-lyophilization solution does not comprise NaCl.

In some embodiments, the pharmaceutical composition comprises
(a) about 1% (w/v) to about 8% (w/v) sucrose;
(b) about 5 mM to about 15 mM histidine;
(c) about 200 mM to about 300 mM arginine;
(d) about 2.5 mM to about 10 mM calcium chloride; and
(e) about 0.1% (w/v) to about 1.0% (w/v) poloxamer. In some embodiments, the poloxamer is poloxamer 188 (P188). In some embodiments, the composition comprises poloxamer 188. In some embodiments, the histidine is L-histidine. In some embodiments, the arginine is L-arginine. In some embodiments the composition comprises L-arginine-HCI. In some embodiments, the composition comprises calcium chloride dihydrate. In some embodiments, the pharmaceutical composition is a pre-lyophilization solution.

In some embodiments, when the lyophilized pharmaceutical composition and the sterile water are combined, then the resulting solution does not comprise NaCl.

In some embodiments, the pharmaceutical composition comprises
(a) about 1% (w/v) sucrose;
(b) about 10 mM histidine;
(c) about 250 mM arginine;
(d) about 5 mM calcium chloride; and
(e) about 0.1% poloxamer 188. In some embodiments, the histidine is L-histidine. In some embodiments, the arginine is L-arginine. In some embodiments the composition comprises L-arginine-HCl. In some embodiments, the composition comprises calcium chloride dihydrate. In some embodiments, the pharmaceutical composition is a pre-lyophilization solution.

In some embodiments, the pharmaceutical composition comprises
(a) about 2% (w/v) sucrose;
(b) about 10 mM histidine;
(c) about 250 mM arginine;
(d) about 5 mM calcium chloride; and
(e) about 0.1% poloxamer 188. In some embodiments, the histidine is L-histidine. In some embodiments, the arginine is L-arginine. In some embodiments the composition comprises L-arginine-HCl. In some embodiments, the composition comprises calcium chloride dihydrate. In some embodiments, the pharmaceutical composition is a pre-lyophilization solution.

In some embodiments, the pharmaceutical composition comprises
(a) about 5% (w/v) sucrose;
(b) about 10 mM histidine;
(c) about 250 mM arginine;
(d) about 5 mM calcium chloride; and
(e) about 0.1% poloxamer 188. In some embodiments, the histidine is L-histidine. In some embodiments, the arginine is L-arginine. In some embodiments the composition comprises L-arginine-HCI. In some embodiments, the composition comprises calcium chloride dihydrate. In some embodiments, the pharmaceutical composition is a pre-lyophilization solution.In some embodiments, the pharmaceutical composition comprises
   (a) about 1% (w/v) to about 5% (w/v) sucrose;
   (b) about 10 mM histidine;
   (c) about 250 mM arginine;
   (d) about 5 mM calcium chloride; and
   (e) about 0.1% poloxamer 188. In some embodiments, the histidine is L-histidine. In some embodiments, the arginine is L-arginine. In some embodiments the composition comprises L-arginine-HCl. In some embodiments, the composition comprises calcium chloride dihydrate. In some embodiments, the pharmaceutical composition is a pre-lyophilization solution.

In some embodiments, the pharmaceutical composition comprises
(a) about 1%, 2%, or 5% (w/v) sucrose;
(b) about 5 mM to about 15 mM histidine;
(c) about 250 mM arginine;
(d) about 5 mM calcium chloride; and
(e) about 0.1% poloxamer 188. In some embodiments, the histidine is L-histidine. In some embodiments, the arginine is L-arginine. In some embodiments the composition comprises L-arginine-HCl. In some embodiments, the composition comprises calcium chloride dihydrate. In some embodiments, the pharmaceutical composition is a pre-lyophilization solution.

In some embodiments, the pharmaceutical composition comprises
(a) about 1%, 2%, or 5% (w/v) sucrose;
(b) about 10 mM histidine;
(c) about 200 mM to about 300 mM arginine;
(d) about 5 mM calcium chloride; and
(e) about 0.1% poloxamer 188. In some embodiments, the histidine is L-histidine. In some embodiments, the arginine is L-arginine. In some embodiments the composition comprises L-arginine-HCl. In some embodiments, the composition comprises calcium chloride dihydrate. In some embodiments, the pharmaceutical composition is a pre-lyophilization solution.

In some embodiments, the pharmaceutical composition comprises
(a) about 1%, 2%, or 5% (w/v) sucrose;
(b) about 10 mM histidine;
(c) about 250 mM arginine;
(d) about 2.5 mM to about 10 mM calcium chloride; and
(e) about 0.1% poloxamer 188. In some embodiments, the histidine is L-histidine. In some embodiments, the arginine is L-arginine. In some embodiments the composition comprises L-arginine-HCl. In some embodiments, the composition comprises calcium chloride dihydrate. In some embodiments, the pharmaceutical composition is a pre-lyophilization solution.

In some embodiments, the pharmaceutical composition comprises
(a) about 1%, 2%, or 5% (w/v) sucrose;
(b) about 10 mM histidine;
(c) about 250 mM arginine;
(d) about 5 mM calcium chloride; and
(e) about 0.008% (w/v) to about 0.1% (w/v) poloxamer 188. In some embodiments, the histidine is L-histidine. In some embodiments, the arginine is L-arginine. In some embodiments the composition comprises L-arginine-HCl. In some embodiments, the composition comprises calcium chloride dihydrate. In some embodiments, the pharmaceutical composition is a pre-lyophilization solution.

In some embodiments, the pharmaceutical composition comprises:
(a) about 10, 20, or 50 mg/ml sucrose;
(b) about 1.552 mg/ml L-histidine;
(c) about 52.665 mg/ml L-arginine-HCl;
(d) about 0.735 mg/ml calcium chloride; and
(e) about 1 mg/ml poloxamer 188.

In some embodiments, the pharmaceutical composition comprises:
(a) about 10, 20, or 50 mg/ml sucrose;
(b) about 1.552 mg/ml L-histidine;
(c) about 52.665 mg/ml L-arginine-HCl;
(d) about 0.735 mg/ml calcium chloride dihydrate; and
(e) about 1 mg/ml poloxamer 188.

In some embodiments, the pharmaceutical composition comprises:
(a) about 10, 20, or 50 mg/ml sucrose;
(b) about 1.552 mg/ml L-histidine;
(c) about 52.665 mg/ml L-arginine-HCl;
(d) about 0.555 mg/ml calcium chloride; and
(e) about 1 mg/ml poloxamer 188. In some embodiments, the composition comprises calcium chloride dihydrate. In some embodiments, the pharmaceutical composition is a pre-lyophilization solution.

In some embodiments, the pharmaceutical composition comprises:
(a) about 10, 20, or 50 mg/ml sucrose;
(b) about 1.552 mg/ml L-histidine;
(c) about 43.550 mg/ml L-arginine;
(d) about 0.735 mg/ml calcium chloride dihydrate; and
(e) about 1 mg/ml poloxamer 188. In some embodiments the composition comprises L-arginine-HCl. In some embodiments, the composition comprises calcium chloride dihydrate. In some embodiments, the pharmaceutical composition is a pre-lyophilization solution.

In some embodiments, the pharmaceutical composition comprises:
(a) about 10, 20, or 50 mg/ml sucrose;
(b) about 1.552 mg/ml L-histidine;
(c) about 43.550 mg/ml L-arginine;
(d) about 0.735 mg/ml calcium chloride; and
(e) about 1 mg/ml poloxamer 188. In some embodiments the composition comprises L-arginine-HCl. In some embodiments, the composition comprises calcium chloride dihydrate. In some embodiments, the pharmaceutical composition is a pre-lyophilization solution.

In some embodiments, the pharmaceutical composition comprises:
(a) about 10, 20, or 50 mg/ml sucrose;
(b) about 1.552 mg/ml L-histidine;
(c) about 43.550 mg/ml L-arginine;
(d) about 0.555 mg/ml calcium chloride; and
(e) about 1 mg/ml poloxamer 188. In some embodiments the composition comprises L-arginine-HCl. In some embodiments, the composition comprises calcium chloride dihydrate. In some embodiments, the pharmaceutical composition is a pre-lyophilization solution.

In some embodiments, the pharmaceutical composition comprises:
(a) 11.23, 22.45, or 56.12 mg/ml sucrose;
(b) 1.74 mg/ml L-histidine;
(c) 59.11 mg/ml L-arginine-HCl;
(d) 0.82 mg/ml calcium chloride dihydrate; and
(e) 1.12 mg/ml poloxamer 188.

In some embodiments, the pharmaceutical composition comprises:
(a) 11.23, 22.45, or 56.12 mg/ml sucrose;
(b) 1.74 mg/ml L-histidine;
(c) 59.11 mg/ml L-arginine-HCl;
(d) 0.62 mg/ml calcium chloride; and
(e) 1.12 mg/ml poloxamer 188. In some embodiments, the composition comprises calcium chloride dihydrate.

In some embodiments, the pharmaceutical composition comprises:
(a) 11.23, 22.45, or 56.12 mg/ml sucrose;
(b) 1.74 mg/ml L-histidine;
(c) 48.88 mg/ml L-arginine;
(d) 0.82 mg/ml calcium chloride dihydrate; and
(e) 1.12 mg/ml poloxamer 188.

In some embodiments, the pharmaceutical composition comprises:
(a) 11.23, 22.45, or 56.12 mg/ml sucrose;
(b) 1.74 mg/ml L-histidine;
(c) 48.88 mg/ml L-arginine;
(d) 0.62 mg/ml calcium chloride; and
(e) 1.12 mg/ml poloxamer 188. In some embodiments the composition comprises L-arginine-HCl. In some embodiments, the composition comprises calcium chloride dihydrate.

In some embodiments, the pharmaceutical composition has a pH of about 6.5 to about 7.5. In some embodiments, the pharmaceutical composition has a pH of about 7.0. In some embodiments, the pharmaceutical composition has a pH of about 6.8.

In some embodiments, the pharmaceutical composition has a pH of about 6.5. In some embodiments, the pharmaceutical composition has a pH of about 6.6. In some embodiments, the pharmaceutical composition has a pH of about 6.7. In some embodiments, the pharmaceutical composition has a pH of about 6.8. In some embodiments, the pharmaceutical composition has a pH of about 6.9. In some embodiments, the pharmaceutical composition has a pH of about 7.0. In some embodiments, the pharmaceutical composition has a pH of about 7.1. In some embodiments, the pharmaceutical composition has a pH of about 7.2. In some embodiments, the pharmaceutical composition has a pH of about 7.3. In some embodiments, the pharmaceutical composition has a pH of about 7.4. In some embodiments, the pharmaceutical composition has a pH of about 7.5.

In some embodiments, the pharmaceutical composition has a pH of 6.5. In some embodiments, the pharmaceutical composition has a pH of 6.6. In some embodiments, the pharmaceutical composition has a pH of 6.7. In some embodiments, the pharmaceutical composition has a pH of 6.8. In some embodiments, the pharmaceutical composition has a pH of 6.9. In some embodiments, the pharmaceutical composition has a pH of 7.0. In some embodiments, the pharmaceutical composition has a pH of 7.1. In some embodiments, the pharmaceutical composition has a pH of 7.2. In some embodiments, the pharmaceutical composition has a pH of 7.3. In some embodiments, the pharmaceutical composition has a pH of 7.4. In some embodiments, the pharmaceutical composition has a pH of 7.5.

In some embodiments, a volume of 3.367 mL of the pre-lyophilization solution is added to a container or vial. In some embodiments, the pre-lyophilized solution is subjected to lyophilization, resulting in a lyophilized pharmaceutical composition.

In some embodiments, the lyophilized pharmaceutical composition comprises:
(a) about 10 mg to about 200 mg sucrose;
(b) about 2.5 mg to about 7.5 mg histidine;
(c) about 140 mg to about 200 mg arginine;
(d) about 1.5 mg to about 5 mg calcium chloride; and
(e) about 1 mg to about 10 mg poloxamer 188.

In some embodiments, the lyophilized pharmaceutical composition comprises:
(a) about 10 mg to about 200 mg sucrose;
(b) about 2.5 mg to about 7.5 mg L-histidine;
(c) about 140 mg to about 200 mg L-arginine;
(d) about 1.5 mg to about 5 mg calcium chloride; and
(e) about 1 mg to about 10 mg poloxamer 188. In some embodiments the composition comprises L-arginine-HCl. In some embodiments, the composition comprises calcium chloride dihydrate.

In some embodiments, the lyophilized pharmaceutical composition comprises:
(a) 10 mg to 200 mg sucrose;
(b) 2.5 mg to 7.5 mg histidine;
(c) 140 mg to 200 mg arginine;
(d) 1.5 mg to 5 mg calcium chloride; and
(e) 1 mg to 10 mg poloxamer 188.

In some embodiments, the lyophilized pharmaceutical composition comprises:
(a) 10 mg to 200 mg sucrose;
(b) 2.5 mg to 7.5 mg L-histidine;
(c) 140 mg to 200 mg L-arginine-HCl;
(d) 1.5 mg to 5 mg calcium chloride; and
(e) 1 mg to 10 mg poloxamer 188. In some embodiments, the composition comprises calcium chloride dihydrate.

In some embodiments, the lyophilized pharmaceutical composition comprises:
(a) 10 mg to 200 mg sucrose;
(b) 2.5 mg to 7.5 mg L-histidine;
(c) 140 mg to 200 mg L-arginine;
(d) 1.5 mg to 5 mg calcium chloride; and
(e) 1 mg to 10 mg poloxamer 188. In some embodiments the composition comprises L-arginine-HCl. In some embodiments, the composition comprises calcium chloride dihydrate.

In some embodiments, the lyophilized pharmaceutical composition comprises:
(a) about 33.7, 67.3, or 168.4 mg sucrose;
(b) about 5.2 mg L-histidine;
(c) about 177.3 mg L-arginine-HCl;
(d) about 2.5 mg calcium chloride; and
(e) about 3.4 mg poloxamer 188.

In some embodiments, the lyophilized pharmaceutical composition comprises:
(a) about 33.7, 67.3, or 168.4 mg sucrose;
(b) about 5.2 mg L-histidine;
(c) about 146.6 mg L-arginine;
(d) about 2.5 mg calcium chloride; and
(e) about 3.4 mg poloxamer 188.

In some embodiments, the lyophilized pharmaceutical composition comprises:
(a) 33.7, 67.3, or 168.4 mg sucrose;
(b) 5.2 mg L-histidine;
(c) 177.3 mg L-arginine-HCl;
(d) 2.5 mg calcium chloride; and
(e) 3.4 mg poloxamer 188.

In some embodiments, the lyophilized pharmaceutical composition comprises:
(a) 33.7, 67.3, or 168.4 mg sucrose;
(b) 5.2 mg L-histidine;
(c) 146.6 mg L-arginine;
(d) 2.5 mg calcium chloride; and
(e) 3.4 mg poloxamer 188.

In some embodiments, the lyophilized pharmaceutical composition comprises:
(a) 33.67, 67.34, or 168.35 mg sucrose;
(b) 5.23 mg L-histidine;
(c) 177.32 mg L-arginine-HCl;
(d) 2.47 mg calcium chloride dihydrate; and
(e) 3.37 mg poloxamer 188. In some embodiments, the lyophilized pharmaceutical composition comprises:
   (a) 33.67, 67.34, or 168.35mg sucrose;
   (b) 5.23 mg L-histidine;
   (c) 177.32 mg L-arginine-HCl;
   (d) 1.87 mg calcium chloride; and
   (e) 3.37 mg poloxamer 188.

In some embodiments, the lyophilized pharmaceutical composition comprises:
(a) 33.67, 67.34, or 168.35mg sucrose;
(b) 5.23 mg L-histidine;
(c) 146.63 mg L-arginine;
(d) 2.47 mg calcium chloride dihydrate; and
(e) 3.37 mg poloxamer 188.

In some embodiments, the lyophilized pharmaceutical composition comprises:
(a) 33.67, 67.34, or 168.35mg sucrose;
(b) 5.23 mg L-histidine;
(c) 146.63 mg L-arginine;
(d) 1.87 mg calcium chloride; and
(e) 3.37 mg poloxamer 188.

In some embodiments, the lyophilized pharmaceutical composition comprises:
(a) 10 mg to 200 mg sucrose;
(b) 5.2 mg L-histidine;
(c) 177.3 mg L-arginine-HCl;
(d) 2.5 mg calcium chloride dihydrate; and
(e) 3.4 mg poloxamer 188.

In some embodiments, the lyophilized pharmaceutical composition comprises:
(a) 10 mg to 200 mg sucrose;
(b) 5.2 mg L-histidine;
(c) 177.3 mg L-arginine-HCl;
(d) 1.9 mg calcium chloride; and
(e) 3.4 mg poloxamer 188.

In some embodiments, the lyophilized pharmaceutical composition comprises:
(a) 10 mg to 200 mg sucrose;
(b) 5.2 mg L-histidine;
(c) 146.6 mg L-arginine;
(d) 2.5 mg calcium chloride dihydrate; and
(e) 3.4 mg poloxamer 188.

In some embodiments, the lyophilized pharmaceutical composition comprises:
(a) 10 mg to 200 mg sucrose;
(b) 5.2 mg L-histidine;
(c) 146.6 mg L-arginine;
(d) 1.9 mg calcium chloride; and
(e) 3.4 mg poloxamer 188.

In some embodiments, the lyophilized pharmaceutical composition comprises:
(a) 33.7, 67.3, or 168.4mg sucrose;
(b) 2.5 mg to 7.5 mg L-histidine;
(c) 177.3 mg L-arginine-HCl;
(d) 2.5 mg calcium chloride dihydrate; and
(e) 3.4 mg poloxamer 188.

In some embodiments, the lyophilized pharmaceutical composition comprises:
(a) 33.7, 67.3, or 168.4mg sucrose;
(b) 2.5 mg to 7.5 mg L-histidine;
(c) 177.3 mg L-arginine-HCl;
(d) 1.9 mg calcium chloride; and
(e) 3.4 mg poloxamer 188.

In some embodiments, the lyophilized pharmaceutical composition comprises:
(a) 33.7, 67.3, or 168.4mg sucrose;
(b) 2.5 mg to 7.5 mg L-histidine;
(c) 146.6 mg L-arginine;
(d) 2.5 mg calcium chloride dihydrate; and
(e) 3.4 mg poloxamer 188.

In some embodiments, the lyophilized pharmaceutical composition comprises:
(a) 33.7, 67.3, or 168.4mg sucrose;
(b) 2.5 mg to 7.5 mg L-histidine;
(c) 146.6 mg L-arginine;
(d) 1.9 mg calcium chloride; and
(e) 3.4 mg poloxamer 188.

In some embodiments, the lyophilized pharmaceutical composition comprises:
(a) 33.67, 67.34, or 168.35mg sucrose;
(b) 5.23 mg L-histidine;
(c) 140 mg to 200 mg L-arginine;
(d) 1.5 mg to 5 mg calcium chloride; and
(e) 3.37 mg poloxamer 188. In some embodiments, the composition compriuses 140 mg to 200 mg L-arginine-HCl.

In some embodiments, the lyophilized pharmaceutical composition comprises:
(a) 33.67, 67.34, or 168.35mg sucrose;
(b) 5.23 mg L-histidine;
(c) 177.32 mg L-arginine-HCl;
(d) 1.5 mg to 5 mg calcium chloride; and
(e) 3.37 mg poloxamer 188.

In some embodiments, the lyophilized pharmaceutical composition comprises:
(a) 33.67, 67.34, or 168.35mg sucrose;
(b) 5.23 mg L-histidine;
(c) 146.63 mg L-arginine;
(d) 1.5 mg to 5 mg calcium chloride; and
(e) 3.37 mg poloxamer 188.

In some embodiments, the lyophilized pharmaceutical composition comprises:
(a) 33.67, 67.34, or 168.35mg sucrose;
(b) 5.23 mg L-histidine;
(c) 177.32 mg L-arginine-HCl;
(d) 2.47 mg calcium chloride dihydrate; and
(e) 1 mg to 10 mg poloxamer 188.

In some embodiments, the lyophilized pharmaceutical composition comprises:
(a) 33.67, 67.34, or 168.35mg sucrose;
(b) 5.23 mg L-histidine;
(c) 177.32 mg L-arginine-HCl;
(d) 1.87 mg calcium chloride; and
(e) 1 mg to 10 mg poloxamer 188.

In some embodiments, the lyophilized pharmaceutical composition comprises:
(a) 33.67, 67.34, or 168.35mg sucrose;
(b) 5.23 mg L-histidine;
(c) 146.63 mg L-arginine;
(d) 2.47 mg calcium chloride dihydrate; and
(e) 1 mg to 10 mg poloxamer 188.

In some embodiments, the lyophilized pharmaceutical composition comprises:
(a) 33.67, 67.34, or 168.35mg sucrose;
(b) 5.23 mg L-histidine;
(c) 146.63 mg L-arginine;
(d) 1.87 mg calcium chloride; and
(e) 1 mg to 10 mg poloxamer 188.

In some embodiments, the lyophilized pharmaceutical composition has a moisture content of less than 2%. In some embodiments, the lyophilized pharmaceutical composition has a moisture content of less than 1.8%. In some embodiments, the lyophilized pharmaceutical composition has a moisture content of less than 1.6%.

In some embodiments, the lyophilized pharmaceutical composition is in a lyophilized cake. In some embodiments, the lyophilized cake is white. In some embodiments, the lyophilized cake is less than Y4 in the European Pharmacopoeia color scale. See Degree of Coloration of Liquids (Method 2.2.2), European Pharmacopoeia, 10th Ed. (2021).

In some embodiments, the lyophilized pharmaceutical composition and sterile water are combined to produce an injectable solution. In some embodiments, the lyophilized pharmaceutical composition is combined with about 2 mL to about 5 mL of sterile water. In some embodiments, the lyophilized pharmaceutical composition is combined with about 3 mL of sterile water. In some embodiments, the lyophilized pharmaceutical composition is combined with 3 mL of sterile water. In some embodiments, the sterile water is USP grade sterile water. In some embodiments, the sterile water is USP grade sterile water for injection. In some embodiments, the sterile water is pyrogen-free or nonpyrogenic. In some embodiments, the sterile water does not contain a bacteriostatic or antimicrobial agent. In some embodiments, the sterile water contains a bacteriostatic or antimicrobial agent. In some embodiments, the sterile water is sterilized using a filter. In some embodiments, the sterile water is sterilized using a 0.1µm filter. In some embodiments, the sterile water is distilled water. In some embodiments, the sterile water is sterile, nonpyrogenic, distilled water, hypotonic, with an osmolarity of zero mOsmol/L, and does not contain a bacteriostatic or antimicrobial agent.

In some embodiments, when the lyophilized pharmaceutical composition and the sterile water are combined, then the resulting solution comprises:
(a) 10 mg/mL to 60 mg/mL sucrose;
(b) 1.5 mg/mL to 2.0 mg/mL L-histidine;
(c) 50 mg/mL to 70 mg/mL L-arginine;
(d) 0.7 mg/mL to 0.9 mg/mL calcium chloride dihydrate; and
(e) 0.6 mg/mL to 1.6 mg/mL poloxamer 188. In some embodiments the composition comprises L-arginine-HCl.

In some embodiments, when the lyophilized pharmaceutical composition and the sterile water are combined, then the resulting solution comprises:
(a) 10 mg/mL to 60 mg/mL sucrose;
(b) 1.5 mg/mL to 2.0 mg/mL L-histidine;
(c) 50 mg/mL to 70 mg/mL L-arginine;
(d) 0.5 mg/mL to 0.8 mg/mL calcium chloride; and
(e) 0.6 mg/mL to 1.6 mg/mL poloxamer 188. In some embodiments the composition comprises L-arginine-HCl. In some embodiments, the composition comprises calcium chloride dihydrate

In some embodiments, when the lyophilized pharmaceutical composition and the sterile water are combined, then the resulting solution comprises:
(a) about 11.23, 22.45, or 56.12 mg/ml sucrose;
(b) about 1.74 mg/ml L-histidine;
(c) about 59.11 mg/ml L-arginine-HCl;
(d) about 0.82 mg/ml calcium chloride dihydrate; and
(e) about 1.12 mg/ml poloxamer 188.

In some embodiments, when the lyophilized pharmaceutical composition and the sterile water are combined, then the resulting solution comprises:
(a) about 11.23, 22.45, or 56.12 mg/ml sucrose;
(b) about 1.74 mg/ml L-histidine;
(c) about 59.11 mg/ml L-arginine-HCl;
(d) about 0.62 mg/ml calcium chloride; and
(e) about 1.12 mg/ml poloxamer 188. In some embodiments, the composition comprises calcium chloride dihydrate.

In some embodiments, when the lyophilized pharmaceutical composition and the sterile water are combined, then the resulting solution comprises:
(a) about 11.23, 22.45, or 56.12 mg/ml sucrose;
(b) about 1.74 mg/ml L-histidine;
(c) about 48.88 mg/ml L-arginine;
(d) about 0.82 mg/ml calcium chloride dihydrate; and
(e) about 1.12 mg/ml poloxamer 188.

In some embodiments, when the lyophilized pharmaceutical composition and the sterile water are combined, then the resulting solution comprises:
(a) about 11.23, 22.45, or 56.12 mg/ml sucrose;
(b) about 1.74 mg/ml L-histidine;
(c) about 48.88 mg/ml L-arginine;
(d) about 0.62 mg/ml calcium chloride; and
(e) about 1.12 mg/ml poloxamer 188. In some embodiments the composition comprises L-arginine-HCl. In some embodiments, the composition comprises calcium chloride dihydrate.

In some embodiments, when the lyophilized pharmaceutical composition and the sterile water are combined, then the resulting solution comprises:
(a) about 10 mg/mL to about 60 mg/ml sucrose;
(b) about 1.74 mg/ml L-histidine;
(c) about 59.11 mg/ml L-arginine-HCl;
(d) about 0.82 mg/ml calcium chloride dihydrate; and
(e) about 1.12 mg/ml poloxamer 188.

In some embodiments, when the lyophilized pharmaceutical composition and the sterile water are combined, then the resulting solution comprises:
(a) about 10 mg/mL to about 60 mg/ml sucrose;
(b) about 1.74 mg/ml L-histidine;
(c) about 59.11 mg/ml L-arginine-HCl;
(d) about 0.62 mg/ml calcium chloride; and
(e) about 1.12 mg/ml poloxamer 188. In some embodiments, the composition comprises calcium chloride dihydrate.

In some embodiments, when the lyophilized pharmaceutical composition and the sterile water are combined, then the resulting solution comprises:
(a) about 10 mg/mL to about 60 mg/ml sucrose;
(b) about 1.74 mg/ml L-histidine;
(c) about 44.88 mg/ml L-arginine;
(d) about 0.82 mg/ml calcium chloride dihydrate; and
(e) about 1.12 mg/ml poloxamer 188

In some embodiments, when the lyophilized pharmaceutical composition and the sterile water are combined, then the resulting solution comprises:
(a) about 10 mg/mL to about 60 mg/ml sucrose;
(b) about 1.74 mg/ml L-histidine;
(c) about 44.88 mg/ml L-arginine;
(d) about 0.62 mg/ml calcium chloride; and
(e) about 1.12 mg/ml poloxamer 188. In some embodiments the composition comprises L-arginine-HCl. In some embodiments, the composition comprises calcium chloride dihydrate.

In some embodiments, when the lyophilized pharmaceutical composition and the sterile water are combined, then the resulting solution comprises:
(a) about 11.23, 22.45, or 56.12 mg/ml sucrose;
(b) about 1.5 mg/mL to about 2.0 mg/mL L-histidine;
(c) about 59.11 mg/ml L-arginine-HCl;
(d) about 0.82 mg/ml calcium chloride dihydrate; and
(e) about 1.12 mg/ml poloxamer 188.

In some embodiments, when the lyophilized pharmaceutical composition and the sterile water are combined, then the resulting solution comprises:
(a) about 11.23, 22.45, or 56.12 mg/ml sucrose;
(b) about 1.5 mg/mL to about 2.0 mg/mL L-histidine;
(c) about 59.11 mg/ml L-arginine-HCl;
(d) about 0.62 mg/ml calcium chloride; and
(e) about 1.12 mg/ml poloxamer 188. In some embodiments, the composition comprises calcium chloride dihydrate.

In some embodiments, when the lyophilized pharmaceutical composition and the sterile water are combined, then the resulting solution comprises:
(a) about 11.23, 22.45, or 56.12 mg/ml sucrose;
(b) about 1.5 mg/mL to about 2.0 mg/mL L-histidine;
(c) about 48.88 mg/ml L-arginine;
(d) about 0.82 mg/ml calcium chloride dihydrate; and
(e) about 1.12 mg/ml poloxamer 188.

In some embodiments, when the lyophilized pharmaceutical composition and the sterile water are combined, then the resulting solution comprises:
(a) about 22.45 mg/ml sucrose;
(b) about 1.5 mg/mL to about 2.0 mg/mL L-histidine;
(c) about 48.88 mg/ml L-arginine;
(d) about 0.62 mg/ml calcium chloride; and
(e) about 1.12 mg/ml poloxamer 188. In some embodiments the composition comprises L-arginine-HCl. In some embodiments, the composition comprises calcium chloride dihydrate.

In some embodiments, when the lyophilized pharmaceutical composition and the sterile water are combined, then the resulting solution comprises:
(a) about 11.23, 22.45, or 56.12 mg/ml sucrose;
(b) about 1.74 mg/ml L-histidine;
(c) about 50 mg/mL to about 70 mg/mL L-arginine-HCl;
(d) about 0.62 mg/ml calcium chloride dihydrate; and
(e) about 1.12 mg/ml poloxamer 188.

In some embodiments, when the lyophilized pharmaceutical composition and the sterile water are combined, then the resulting solution comprises:
(a) about 11.23, 22.45, or 56.12 mg/ml sucrose;
(b) about 1.74 mg/ml L-histidine;
(c) about 50 mg/mL to about 70 mg/mL L-arginine-HCl;
(d) about 0.62 mg/ml calcium chloride; and
(e) about 1.12 mg/ml poloxamer 188. In some embodiments, the composition comprises calcium chloride dihydrate.

In some embodiments, when the lyophilized pharmaceutical composition and the sterile water are combined, then the resulting solution comprises:
(a) about 11.23, 22.45, or 56.12 mg/ml sucrose;
(b) about 1.74 mg/ml L-histidine;
(c) about 40 mg/mL to about 60 mg/mL L-arginine;
(d) about 0.82 mg/ml calcium chloride dihydrate; and
(e) about 1.12 mg/ml poloxamer 188.

In some embodiments, when the lyophilized pharmaceutical composition and the sterile water are combined, then the resulting solution comprises:
(a) about 11.23, 22.45, or 56.12 mg/ml sucrose;
(b) about 1.74 mg/ml L-histidine;
(c) about 40 mg/mL to about 60 mg/mL L-arginine;
(d) about 0.62 mg/ml calcium chloride; and
(e) about 1.12 mg/ml poloxamer 188. In some embodiments the composition comprises L-arginine-HCl. In some embodiments, the composition comprises calcium chloride dihydrate.

In some embodiments, when the lyophilized pharmaceutical composition and the sterile water are combined, then the resulting solution comprises:
(a) about 11.23, 22.45, or 56.12 mg/ml sucrose;
(b) about 1.74 mg/ml L-histidine;
(c) about 59.11 mg/ml L-arginine-HCl;
(d) about 0.7 mg/mL to about 0.9 mg/mL calcium chloride dihydrate; and
(e) about 1.12 mg/ml poloxamer 188.

In some embodiments, when the lyophilized pharmaceutical composition and the sterile water are combined, then the resulting solution comprises:
(a) about 11.23, 22.45, or 56.12 mg/ml sucrose;
(b) about 1.74 mg/ml L-histidine;
(c) about 59.11 mg/ml L-arginine-HCl;
(d) about 0.5 mg/mL to about 0.9 mg/mL calcium chloride; and
(e) about 1.12 mg/ml poloxamer 188. In some embodiments, the composition comprises calcium chloride dihydrate.

In some embodiments, when the lyophilized pharmaceutical composition and the sterile water are combined, then the resulting solution comprises:
(a) about 11.23, 22.45, or 56.12 mg/ml sucrose;
(b) about 1.74 mg/ml L-histidine;
(c) about 48.88 mg/ml L-arginine;
(d) about 0.7 mg/mL to about 0.9 mg/mL calcium chloride dihydrate; and
(e) about 1.12 mg/ml poloxamer 188. In some embodiments the composition comprises L-arginine-HCl.

In some embodiments, when the lyophilized pharmaceutical composition and the sterile water are combined, then the resulting solution comprises:
(a) about 11.23, 22.45, or 56.12 mg/ml sucrose;
(b) about 1.74 mg/ml L-histidine;
(c) about 48.88 mg/ml L-arginine;
(d) about 0.5 mg/mL to about 0.7 mg/mL calcium chloride; and
(e) about 1.12 mg/ml poloxamer 188. In some embodiments the composition comprises L-arginine-HCl. In some embodiments, the composition comprises calcium chloride dihydrate.

In some embodiments, when the lyophilized pharmaceutical composition and the sterile water are combined, then the resulting solution comprises:
(a) about 11.23, 22.45, or 56.12 mg/ml sucrose;
(b) about 1.74 mg/ml L-histidine;
(c) about 59.11 mg/ml L-arginine-HCl;
(d) about 0.82 mg/ml calcium chloride dihydrate; and
(e) about 0.6 mg/mL to about 1.6 mg/mL poloxamer 188.

In some embodiments, when the lyophilized pharmaceutical composition and the sterile water are combined, then the resulting solution comprises:
(a) about 11.23, 22.45, or 56.12 mg/ml sucrose;
(b) about 1.74 mg/ml L-histidine;
(c) about 59.11 mg/ml L-arginine-HCl;
(d) about 0.62 mg/ml calcium chloride; and
(e) about 0.6 mg/mL to about 1.6 mg/mL poloxamer 188. In some embodiments the composition comprises L-arginine-HCl. In some embodiments, the composition comprises calcium chloride dihydrate.

In some embodiments, when the lyophilized pharmaceutical composition and the sterile water are combined, then the resulting solution comprises:
(a) about 11.23, 22.45, or 56.12 mg/ml sucrose;
(b) about 1.74 mg/ml L-histidine;
(c) about 48.88 mg/ml L-arginine;
(d) about 0.82 mg/ml calcium chloride dihydrate; and
(e) about 0.6 mg/mL to about 1.6 mg/mL poloxamer 188.

In some embodiments, when the lyophilized pharmaceutical composition and the sterile water are combined, then the resulting solution comprises:
(a) about 11.23, 22.45, or 56.12 mg/ml sucrose;
(b) about 1.74 mg/ml L-histidine;
(c) about 48.88 mg/ml L-arginine;
(d) about 0.62 mg/ml calcium chloride; and
(e) about 0.6 mg/mL to about 1.6 mg/mL poloxamer 188. In some embodiments the composition comprises L-arginine-HCl. In some embodiments, the composition comprises calcium chloride dihydrate.

In some embodiments, when the lyophilized pharmaceutical composition and the sterile water are combined, then the resulting solution comprises:
(a) about 11.23, 22.45, or 56.12 mg/ml sucrose;
(b) about 1.74 mg/ml L-histidine;
(c) about 59.11 mg/ml L-arginine-HCl;
(d) about 0.82 mg/ml calcium chloride dihydrate; and
(e) about 1.12 mg/ml poloxamer 188.

In some embodiments, when the lyophilized pharmaceutical composition and the sterile water are combined, then the resulting solution comprises:
(a) about 11.23, 22.45, or 56.12 mg/ml sucrose;
(b) about 1.74 mg/ml L-histidine;
(c) about 59.11 mg/ml L-arginine-HCl;
(d) about 0.62 mg/ml calcium chloride; and
(e) about 1.12 mg/ml poloxamer 188.

In some embodiments, when the lyophilized pharmaceutical composition and the sterile water are combined, then the resulting solution comprises:
(a) about 11.23, 22.45, or 56.12 mg/ml sucrose;
(b) about 1.74 mg/ml L-histidine;
(c) about 48.88 mg/ml L-arginine;
(d) about 0.82 mg/ml calcium chloride dihydrate; and
(e) about 1.12 mg/ml poloxamer 188.

In some embodiments, when the lyophilized pharmaceutical composition and the sterile water are combined, then the resulting solution comprises:
(a) about 11.23, 22.45, or 56.12 mg/ml sucrose;
(b) about 1.74 mg/ml L-histidine;
(c) about 48.88 mg/ml L-arginine;
(d) about 0.62 mg/ml calcium chloride; and
(e) about 1.12 mg/ml poloxamer 188.

In some embodiments, when the lyophilized pharmaceutical composition and the sterile water are combined, then the resulting solution comprises:
(a) about 33.67, 67.34, or 168.35 mg sucrose;
(b) about 5.23 mg L-histidine;
(c) about 177.32 mg L-arginine-HCl;
(d) about 2.47 mg calcium chloride; and
(e) about 3.37 mg poloxamer 188 in 3 mL of sterile water.

In some embodiments, when the lyophilized pharmaceutical composition and the sterile water are combined, then the resulting solution comprises:
(a) about 33.67, 67.34, or 168.35 mg sucrose;
(b) about 5.23 mg L-histidine;
(c) about 146.63 mg L-arginine;
(d) about 2.47 mg calcium chloride dihydrate; and
(e) about 3.37 mg poloxamer 188 in 3 mL of sterile water.

In some embodiments, when the lyophilized pharmaceutical composition and the sterile water are combined, then the resulting solution comprises:
(a) about 33.67, 67.34, or 168.35 mg sucrose;
(b) about 5.23 mg L-histidine;
(c) about 146.63 mg L-arginine;
(d) about 1.87 mg calcium chloride; and
(e) about 3.37 mg poloxamer 188 in 3 mL of sterile water.

In some embodiments, when the lyophilized pharmaceutical composition and the sterile water are combined, then the resulting solution comprises:
(a) 33.67, 67.34, or 168.35 mg sucrose;
(b) 5.23 mg L-histidine;
(c) 177.32 mg L-arginine-HCl;
(d) 2.47 mg calcium chloride dihydrate; and
(e) 3.37 mg poloxamer 188
in 3 mL of sterile water.

In some embodiments, when the lyophilized pharmaceutical composition and the sterile water are combined, then the resulting solution comprises:
(a) 33.67, 67.34, or 168.35 mg sucrose;
(b) 5.23 mg L-histidine;
(c) 177.32 mg L-arginine-HCl;
(d) 1.87 mg calcium chloride; and
(e) 3.37 mg poloxamer 188
in 3 mL of sterile water.

In some embodiments, when the lyophilized pharmaceutical composition and the sterile water are combined, then the resulting solution comprises:
(a) 33.67, 67.34, or 168.35 mg sucrose;
(b) 5.23 mg L-histidine;
(c) 146.63 mg L-arginine;
(d) 2.47 mg calcium chloride dihydrate; and
(e) 3.37 mg poloxamer 188
in 3 mL of sterile water.

In some embodiments, when the lyophilized pharmaceutical composition and the sterile water are combined, then the resulting solution comprises:
(a) 33.67, 67.34, or 168.35 mg sucrose;
(b) 5.23 mg L-histidine;
(c) 146.63 mg L-arginine;
(d) 1.87 mg calcium chloride; and
(e) 3.37 mg poloxamer 188
in 3 mL of sterile water.

In some embodiments, when the lyophilized pharmaceutical composition and the sterile water are combined, then the lyophilized pharmaceutical composition is reconstituted within 7 to 12 seconds.

In some embodiments, when the lyophilized pharmaceutical composition and the sterile water are combined, then the osmolality of the resulting solution is about 525 to about 725 mOsm/kg. In some embodiments, the pharmaceutical composition disclosed herein has an osmolality about 500 to about 650 mOsm/kg. In some embodiments, when the lyophilized pharmaceutical composition and the sterile water are combined, then the osmolality of the resulting solution is about 600 to about 650 mOsm/kg.

In some embodiments, the pharmaceutical composition has an osmolality of about 525 mOsm/kg. In some embodiments, the pharmaceutical composition has an osmolality of about 550 mOsm/kg. In some embodiments, the pharmaceutical composition has an osmolality of about 575 mOsm/kg. In some embodiments, the pharmaceutical composition has an osmolality of about 600 mOsm/kg. In some embodiments, the pharmaceutical composition has an osmolality of about 625 mOsm/kg. In some embodiments, the pharmaceutical composition has an osmolality of about 650 mOsm/kg. In some embodiments, the pharmaceutical composition has an osmolality of about 675 mOsm/kg. In some embodiments, the pharmaceutical composition has an osmolality of about 700 mOsm/kg. In some embodiments, the pharmaceutical composition has an osmolality of about 725 mOsm/kg.

In some embodiments, when the lyophilized pharmaceutical composition and the sterile water are combined, then the pH of the resulting solution is about 6.5 to about 7.5. In some embodiments, the pharmaceutical composition has a pH of about 7.0. In some embodiments, the pharmaceutical composition has a pH of about 6.8. In some embodiments, the pharmaceutical composition has a pH of 6.8.

In some embodiments, when the lyophilized pharmaceutical composition and the sterile water are combined, then the pH of the resulting solution is about 6.5. In some embodiments, when the lyophilized pharmaceutical composition and the sterile water are combined, then the pH of the resulting solution is about 6.6. In some embodiments, when the lyophilized pharmaceutical composition and the sterile water are combined, then the pH of the resulting solution is about 6.7. In some embodiments, when the lyophilized pharmaceutical composition and the sterile water are combined, then the pH of the resulting solution is about 6.8. In some embodiments, when the lyophilized pharmaceutical composition and the sterile water are combined, then the pH of the resulting solution is about 6.9. In some embodiments, when the lyophilized pharmaceutical composition and the sterile water are combined, then the pH of the resulting solution is about 7.0. In some embodiments, when the lyophilized pharmaceutical composition and the sterile water are combined, then the pH of the resulting solution is about 7.1. In some embodiments, when the lyophilized pharmaceutical composition and the sterile water are combined, then the pH of the resulting solution is about 7.2. In some embodiments, when the lyophilized pharmaceutical composition and the sterile water are combined, then the pH of the resulting solution is about 7.3. In some embodiments, when the lyophilized pharmaceutical composition and the sterile water are combined, then the pH of the resulting solution is about 7.4. In some embodiments, when the lyophilized pharmaceutical composition and the sterile water are combined, then the pH of the resulting solution is about 7.5.

In some embodiments, when the lyophilized pharmaceutical composition and the sterile water are combined, then the protein concentration of the resulting solution is about 0.8 to about 1.2 mg/mL.

In some embodiments, when the lyophilized pharmaceutical composition and the sterile water are combined, then the protein concentration of the resulting solution is about 0.8 mg/mL. In some embodiments, when the lyophilized pharmaceutical composition and the sterile water are combined, then the protein concentration of the resulting solution is about 0.9 mg/mL. In some embodiments, when the lyophilized pharmaceutical composition and the sterile water are combined, then the protein concentration of the resulting solution is about 1.0 mg/mL. In some embodiments, when the lyophilized pharmaceutical composition and the sterile water are combined, then the protein concentration of the resulting solution is about 1.1 mg/mL. In some embodiments, when the lyophilized pharmaceutical composition and the sterile water are combined, then the protein concentration of the resulting solution is about 1.2 mg/mL.

In some embodiments, when the lyophilized pharmaceutical composition and the sterile water are combined, then the protein concentration of the resulting solution is 0.8 mg/mL. In some embodiments, when the lyophilized pharmaceutical composition and the sterile water are combined, then the protein concentration of the resulting solution is 0.9 mg/mL. In some embodiments, when the lyophilized pharmaceutical composition and the sterile water are combined, then the protein concentration of the resulting solution is 1.0 mg/mL. In some embodiments, when the lyophilized pharmaceutical composition and the sterile water are combined, then the protein concentration of the resulting solution is 1.1 mg/mL. In some embodiments, when the lyophilized pharmaceutical composition and the sterile water are combined, then the protein concentration of the resulting solution is 1.2 mg/mL.

In some embodiments, when the lyophilized pharmaceutical composition and the sterile water are combined, then the turbidity of the resulting solution is less than about 7 Nephelometric Turbidity Units.

In some embodiments, when the lyophilized pharmaceutical composition and the sterile water are combined, less than 3% of the chimeric protein is aggregated.

Disclosed herein is a method of storing a pharmaceutical composition, comprising maintaining a pharmaceutical composition disclosed herein at a temperature of from about -20°C to about -40°C for at least about 1 to about 36 months. In some embodiments, the pharmaceutical composition is stored at a temperature of about -20°C, about -25°C, about -30°C, about -35°C, or about -40°C for at least about 1 to about 36 months. In some embodiments, the pharmaceutical composition is stored at a temperature of about -20°C, about -25°C, about -30°C, about -35°C, or about -40°C for at least about 3, 6, 9, 12, 18, 21, 24, 27, 30, 33, or 36 months. In some embodiments, the pharmaceutical composition is stored at a temperature of about -30°C for at least about 1, 2, 3, 4, 5, 6, 7, 8, 9, 11, or 12 months.

In some embodiments, the pharmaceutical composition has been stored at a temperature of from about -20°C to about -40°C for at least about 1 to about 36 months. In some embodiments, the pharmaceutical composition has been stored at a temperature of about -20°C, about -25°C, about -30°C, about -35°C, or about -40°C for at least about 1 to about 36 months. In some embodiments, the pharmaceutical composition has been stored at a temperature of about -20°C, about -25°C, about -30°C, about -35°C, or about -40°C for at least about 3, 6, 9, 12, 18, 21, 24, 27, 30, 33, or 36 months. In some embodiments, the pharmaceutical composition has been stored at a temperature of about -30°C for at least about 1, 2, 3, 4, 5, 6, 7, 8, 9, 11, or 12 months.

In some embodiments, the pharmaceutical composition is a liquid pharmaceutical composition that has been frozen and then thawed 2-5 times. In some embodiments, the pharmaceutical composition is a liquid pharmaceutical composition that has been frozen and then thawed 2 times. In some embodiments, the pharmaceutical composition is a liquid pharmaceutical composition that has been frozen and then thawed 3 times. In some embodiments, the pharmaceutical composition is a liquid pharmaceutical composition that has been frozen and then thawed 4 times. In some embodiments, the pharmaceutical composition is a liquid pharmaceutical composition that has been frozen and then thawed 5 times.

In some embodiments, the pharmaceutical composition comprises about 0.5 mg/ml to about 10 mg/ml of the chimeric protein. In some embodiments, the pharmaceutical composition comprises about 0.5 mg/ml to about 5 mg/ml of the chimeric protein. In some embodiments, the pharmaceutical composition comprises about 0.5 mg/ml to about 2 mg/ml of the chimeric protein. In some embodiments, the pharmaceutical composition comprises about 0.5 mg/ml of the chimeric protein. In some embodiments, the pharmaceutical composition comprises about 1 mg/ml of the chimeric protein. In some embodiments, the pharmaceutical composition comprises about 1.5 mg/ml of the chimeric protein. In some embodiments, the pharmaceutical composition comprises about 2 mg/ml of the chimeric protein.

### V. Pharmaceutical Kits

In some embodiments, the pharmaceutical composition is provided with a second container comprising sterile water. Disclosed herein are pharmaceutical kits which comprise a first container containing the pharmaceutical composition and a second container containing sterile water.

Disclosed herein is a pharmaceutical kit comprising:
(i) a first container comprising a lyophilized pharmaceutical composition comprising
   (a) a chimeric protein comprising a first polypeptide chain which comprises a Factor VIII ("FVIII") protein or a portion thereof and a first immunoglobulin ("Ig") constant region or a portion thereof, and a second polypeptide chain which comprises a von Willebrand Factor ("VWF") protein and a second Ig constant region or a portion thereof;
   (b) about 10 mg to about 200 mg sucrose;
   (c) about 2.5 mg to about 7.5 mg histidine;
   (d) about 140 mg to about 200 mg arginine;
   (e) about 1.5 mg to about 5 mg calcium chloride; and
   (f) about 0.5 mg to about 10 mg poloxamer 188, and
(ii) a second container comprising sterile water.

In some embodiments, the first container comprises 100 IU to 10,000 IU of the chimeric protein.

In some embodiments, the first container comprises 250 IU, 500 IU, 1000 IU, 2000 IU, 3000 IU, or 4,000 IU of the chimeric protein. In some embodiments, the first container comprises 250 IU of the chimeric protein. In some embodiments, the first container comprises 500 IU of the chimeric protein. In some embodiments, the first container comprises 1000 IU of the chimeric protein. In some embodiments, the first container comprises 2000 IU of the chimeric protein. In some embodiments, the first container comprises 3000 IU of the chimeric protein. In some embodiments, the first container comprises 4,000 IU of the chimeric protein. In some embodiments, the first container comprises more than about 250 IU of the chimeric protein. In some embodiments, the first container comprises more than about 300 IU of the chimeric protein. In some embodiments, the first container comprises more than about 500 IU of the chimeric protein. In some embodiments, the first container comprises at least about 500 IU of the chimeric protein.

In some embodiments, the second container comprises about 2 mL to about 5 mL of sterile water. In some embodiments, the second container comprises 2 mL to 5 mL of sterile water. In some embodiments, the second container comprises about 3 mL of sterile water. In some embodiments, the second container comprises 3 mL of sterile water.

In some embodiments, the second container comprises about 2 mL of sterile water. In some embodiments, the second container comprises about 2.1 mL of sterile water. In some embodiments, the second container comprises about 2.2 mL of sterile water. In some embodiments, the second container comprises about 2.3 mL of sterile water. In some embodiments, the second container comprises about 2.4 mL of sterile water. In some embodiments, the second container comprises about 2.5 mL of sterile water. In some embodiments, the second container comprises about 2.6 mL of sterile water. In some embodiments, the second container comprises about 2.7 mL of sterile water. In some embodiments, the second container comprises about 2.8 mL of sterile water. In some embodiments, the second container comprises about 2.9 mL of sterile water. In some embodiments, the second container comprises about 3 mL of sterile water. In some embodiments, the second container comprises about 3.1 mL of sterile water. In some embodiments, the second container comprises about 3.2 mL of sterile water. In some embodiments, the second container comprises about 3.3 mL of sterile water. In some embodiments, the second container comprises about 3.4 mL of sterile water. In some embodiments, the second container comprises about 3.5 mL of sterile water. In some embodiments, the second container comprises about 3.6 mL of sterile water. In some embodiments, the second container comprises about 3.7 mL of sterile water. In some embodiments, the second container comprises about 3.8 mL of sterile water. In some embodiments, the second container comprises about 3.9 mL of sterile water. In some embodiments, the second container comprises about 4 mL of sterile water. In some embodiments, the second container comprises about 4.1 mL of sterile water. In some embodiments, the second container comprises about 4.2 mL of sterile water. In some embodiments, the second container comprises about 4.3 mL of sterile water. In some embodiments, the second container comprises about 4.4 mL of sterile water. In some embodiments, the second container comprises about 4.5 mL of sterile water. In some embodiments, the second container comprises about 4.6 mL of sterile water. In some embodiments, the second container comprises about 4.7 mL of sterile water. In some embodiments, the second container comprises about 4.8 mL of sterile water. In some embodiments, the second container comprises about 4.9 mL of sterile water. In some embodiments, the second container comprises about 5 mL of sterile water.

In some embodiments, the pharmaceutical kit further comprises instructions for combining the lyophilized pharmaceutical composition and the sterile water.

In some embodiments, the first container is a glass vial comprising a rubber stopper.

In some embodiments, the second container is a syringe body. In some embodiments, the syringe body is associated with a plunger. In some embodiments, the pharmaceutical kit further comprises an adaptor to connect the glass vial to the syringe body. In some embodiments, the pharmaceutical kit further comprises infusion tubing associated with a needle to be connected to the syringe body, suitable for intravenous infusion. In some embodiments, the second container is a pre-filled syringe.

Disclosed herein is a method of storing a pharmaceutical kit, comprising maintaining a pharmaceutical kit disclosed herein at a temperature of from about -20°C to about -40°C for at least about 1 to about 36 months. In some embodiments, the pharmaceutical kit is stored at a temperature of about -20°C, about -25°C, about -30°C, about -35°C, or about - 40°C for at least about 1 to about 36 months. In some embodiments, the pharmaceutical kit is stored at a temperature of about -20°C, about -25°C, about -30°C, about -35°C, or about -40°C for at least about 3, 6, 9, 12, 18, 21, 24, 27, 30, 33, or 36 months. In some embodiments, the pharmaceutical kit is stored at a temperature of about -30°C for at least about 1, 2, 3, 4, 5, 6, 7, 8, 9, 11, or 12 months.

In some embodiments, the pharmaceutical kit has been stored at a temperature of from about -20°C to about -40°C for at least about 1 to about 36 months. In some embodiments, the pharmaceutical kit has been stored at a temperature of about -20°C, about -25°C, about -30°C, about -35°C, or about -40°C for at least about 1 to about 36 months. In some embodiments, the pharmaceutical kit has been stored at a temperature of about -20°C, about -25°C, about -30°C, about -35°C, or about -40°C for at least about 3, 6, 9, 12, 18, 21, 24, 27, 30, 33, or 36 months. In some embodiments, the pharmaceutical kit has been stored at a temperature of about -30°C for at least about 1, 2, 3, 4, 5, 6, 7, 8, 9, 11, or 12 months.

### VI. Methods and Uses of the Pharmaceutical Composition

Also disclosed herein is a use of the pharmaceutical composition or a method for treating hemophilia A in a subject in need thereof, comprising administering to the subject an effective amount of the pharmaceutical composition of the disclosure. In some embodiments, the treatment of hemophilia A comprises preventing a bleeding episode in a human subject in need thereof. In some embodiments, the treatment of hemophilia A comprises treating a bleeding episode in a human subject in need thereof. In some embodiments, the treatment of hemophilia A comprises controlling the incidence or frequency of a bleeding episode in a human subject in need thereof. In some embodiments, the treatment of hemophilia A comprises decreasing the incidence or frequency of a bleeding episode in a human subject in need thereof.

In some embodiments, the composition is used to treat a bleeding disease or condition in a subject in need thereof. The bleeding disease or condition is selected from the group consisting of a bleeding coagulation disorder, hemarthrosis, muscle bleed, oral bleed, hemorrhage, hemorrhage into muscles, oral hemorrhage, trauma, trauma capitis, gastrointestinal bleeding, intracranial hemorrhage, intra-abdominal hemorrhage, intrathoracic hemorrhage, bone fracture, central nervous system bleeding, bleeding in the retropharyngeal space, bleeding in the retroperitoneal space, bleeding in the illiopsoas sheath and any combinations thereof. In still other embodiments, the subject is scheduled to undergo a surgery. In some embodiments, the treatment is prophylactic or on-demand.

In some embodiments, the use or method comprises combining the lyophilized pharmaceutical composition and the sterile water of a kit of the disclosure, and administering to the subject an effective amount of the resulting combination. In some embodiments, the subject combines the lyophilized pharmaceutical composition and the sterile water of the kit. In some embodiments, the combination is self-administered by the subject.

In some embodiments, the multiple doses comprise at least two doses, at least three doses, at least four doses, at least five doses, at least six doses, at least seven doses, at least eight doses, at least nine doses, at least ten doses, at least eleven doses, at least twelve doses, at least thirteen doses, at least fourteen doses, at least fifteen doses, at least sixteen doses, at least seventeen doses, at least eighteen doses, at least nineteen doses, at least twenty doses, or more. In some embodiments, the multiple doses are administered for at least about 1 month, at least about 2 months, at least about 3 months, at least about 4 months, at least about 5 months, at least about 6 months, at least about 12 months, at least about 18 months, at least about 2 years, at least about 3 years, at least about 4 years, at least about 5 years, at least about 10 years, at least about 15 years, at least about 20 years, or for at least about 25 years.

A chimeric protein described herein can be administered by any means known in the art. In some embodiments, the chimeric protein is administered by a route selected from the group consisting of intravenous injection, intravenous infusion, subcutaneous administration, intramuscular administration, oral administration, nasal administration, and pulmonary administration. In some embodiments, the chimeric protein is administered intravenously. In some embodiments, the chimeric protein is administered subcutaneously.

Having now described the present disclosure in detail, the same will be more clearly understood by reference to the following examples, which are included herewith for purposes of illustration only and are not intended to be limiting of the disclosure. All patents, publications, and articles referred to herein are expressly and specifically incorporated herein by reference.

### EXAMPLES

### Example 1: Evaluation of aggregation of efanesoctocog alfa compositions comprising different surfactants

The experiments described in this example assess the stability of efanesoctocog alfa in pharmaceutical compositions comprising a different surfactant (poloxamer 188) formulated with different concentrations of sucrose. In addition to sucrose, each composition also contained 10 mM L-histidine, 250 mM L-arginine-HCl, 5 mM CaCl₂, and 0.1% (w/v) poloxamer 188 (poloxamer 188), at a pH of 6.8. Stability attributes for poloxamer 188 compositions were also compared to stability attributes for otherwise identical PS80 compositions to evaluate the effect of selected surfactant.

To further analyze the impact on sucrose concentration on efanesoctocog alfa compositions containing poloxamer 188, pharmaceutical compositions of efanesoctocog alfa at 0, 1, 2, and 5% w/v sucrose were subjected to different stability conditions and stability was assessed using several methods. Compositions were tested at the following product stages: drug substance (DS, which was had not been lyophilized or adjusted for any particular IU strength) frozen storage stability, DS freeze/thaw (FT) stability, liquid DS post-thaw stability, diluted DS and drug product (DP) stability, DP lyophilization stability, and DP-post reconstitution.

Aggregation of efanesoctocog alfa compositions was measured by SEC assay (%HMWS). The SEC assay uses an HPLC instrument equipped with a pump, a temperature controlled autosampler, a column heater, and fluorescence detector. The sample solutions are analyzed using the following instrumentation and method parameters:
▪ Mobile Phase: Dulbecco's Phosphate- Buffered Saline (D-PBS) containing Calcium and Magnesium adding 0.36 M Sodium Chloride (0.9 mM Calcium Chloride, 0.5 mM Magnesium Chloride, 2.7 mM Potassium Chloride, 1.5 mM Potassium Phosphate Mono basic, 496 mM Sodium Chloride, 8.1 mM Sodium Phosphate Dibasic, pH 7. 0 ± 0.1)
▪ Column Heater: 26°C
▪ Run Time: 40 minutes
▪ FL Detector: Ex/Em = 280/350 nm, PMT Gain = 5
▪ Injection Mass Load: 2 µg
▪ Autosampler Temperature: 5°C
▪ Flow Rate: 0.5 mL/min, Isocratic

Aggregation of efanesoctocog alfa drug substance (DS) compositions (1 mg/mL) containing either 0.5% (w/v) PS80 or 0.1% (w/v) poloxamer 188 as a surfactant and and either 1% or 5% (w/v) sucrose were tested at room temperature (RT)/room light (RL) conditions or at 2-8 °C. Aggregation results for RT/RL conditions over time (hours) are shown in FIG. 2. At RT/RL conditions, reduction of sucrose from 5% to 1% increased aggregation by 2X for compositions containing either surfactant. At RT/RL conditions, aggregation rate increases were about the same for both poloxamer 188 compositions and PS80 compositions.

Aggregation results at 2-8°C over time (hours) are shown in FIG. 3. At 2-8°C conditions, reduction of sucrose from 5% to 1% increased aggregation by 2X in PS80 compositions, which was similar to the results at RT/RL conditions. However, at 2-8°C, reduction of sucrose from 5% to 1% increased aggregation by 10X for PS80 compositions. At 2-8°C, aggregation rate increases were lower in poloxamer 188 compositions than PS80 compositions.

Aggregation of efanesoctocog alfa drug substance (DS) liquid compositions at 4000 IU and containing either 0.5% (w/v) PS80 or 0.1% (w/v) poloxamer 188 as a surfactant were assessed in a cumulative hold time assay. Efanesoctocog alfa liquid compositions were held at RT and tested after 0, 5, 7, 25, 43, and 55 hours. Samples were also tested after lyophilization.

Results of the cumulative hold time assay are shown in FIG. 4. Efanesoctocog alfa drug substance (DS) liquid compositions followed by subsequent dilutions to 4000 IU comprising 1% sucrose and 0.05% PS80 showed the highest increase in aggregation (see FIG. 4, square plot). The results of this cumulative hold time assay also show that compositions comprising poloxamer 188 may be moderately more stable than PS80 compositions in liquid state at RT.

Frozen storage stability of compositions of efanesoctocog alfa DS at 1 mg/mL was assessed for compositions comprising either 0.5% (w/v) PS80 or 0.1% (w/v) poloxamer 188 as a surfactant. Compositions containing 0, 1, 2, or 5% (w/v) sucrose were held at freezing temperatures (-80°C or -30°C). Time points measured were pre-freeze, start of experiment (T0), one month (T1M), 3 months (T3M), and 6 months (T6M).

Results of the frozen storage stability assay for compositions comprising 0.5% (w/v) PS80 are shown in FIG. 5. Results of the frozen storage stability assay for compositions comprising 0.1% (w/v) poloxamer 188 are shown in FIG. 6. No trend in aggregation was observed for any of the tested compositions at -80°C. However, at at -30°C, compositions comprising PS80 surfactant showed an increase in aggregation (see FIG. 5). This effect was most evident at lower sucrose concentrations (see, e.g., FIG. 5, 0% sucrose result). In contrast, compositions comprising poloxamer 188 did not show an increase in aggregation at -30°C (see FIG. 6). Compositions comprising poloxamer 188 showed comparable aggregation levels at both -80°C and -30°C. Thus, use of poloxamer 188 as a surfactant may provide improved frozen storage stability for efanesoctocog alfa compositions, particularly if compositions are stored at -30°C.

The frozen storage stability of efanesoctocog alfa DS compositions (1 mg/mL) comprising 0.1% (w/v) poloxamer 188 was further analyzed by particle testing using high accuracy liquid particle counter (HIAC). DS compositions containing 0.1% (w/v) poloxamer 188 and 0, 1, 2, or 5% (w/v) sucrose were held at -80°C and assessed at the following time points: pre-freeze, start of experiment (T0), one month (T1M), 3 months (T3M), and 6 months (T6M). Results for ≥10 µm particles are shown in FIG. 7A. Results for 225 µm particles are shown in FIG. 7B. The ≥10 µm particles are less than 6000 particles and ≥25 µm particles are less than 600 particles, and within the limit of <USP 787>. No significant trending in particles was observed over storage.

Aggregation of compositions of efanesoctocog alfa lyophilized drug product (Lyo DP) at 250 IU or 4000 IU and containing 0.1% (w/v) poloxamer 188 were tested at 5°C, 30°C, and 40°C. Lyo DP compositions containing 1% and 5% w/v sucrose were tested. Samples were tested at T0, 1 month, 2 months, 3 months, and 6 months.

Results of Lyo DP compositions with at 250 IU are shown in FIG. 8A (5°C), FIG. 8B (30°C), and FIG. 8C (40°C). For the 250 IU compositions, higher aggregation was generally demonstrated in poloxamer 188 compositions than PS80 compositions (data not shown). The 250 IU compositions comprising 0.1% (w/v) poloxamer 188 and 1% sucrose demonstrated significant increases in aggregation at all temperatures.

Results of Lyo DP compositions at 4000 IU are shown in FIG. 9A (5°C), FIG. 9B (30°C), and FIG. 9C (40°C). For all 4000 IU compositions comprising 0.1% (w/v) poloxamer 188, no aggregation trends were observed at any temperature for both sucrose concentrations. This result is consistent with results for Lyo DP compositions at 4000 IU comprising 0.5% (w/v) PS80, which also did not demonstrate notable aggregation trends based on sucrose concentration or temperature (data not shown).

Aggregation of efanesoctocog alfa compositions following freeze/thaw (F/T) stress was also analyzed for each surfactant. Compositions of efanesoctocog alfa DS at 1 mg/mL and comprising either 0.5% (w/v) PS80 or 0.1% (w/v) poloxamer 188 as surfactant and 0, 1, 2, or 5% (w/v) sucrose were subjected to stress by F/T at either -80°C or -30°C for 5 cycles (5X)(not less than 24hrs, thaw at room temperature). Samples were tested before freezing (Pre Freeze) and after 5x F/T cycles.

Results for compositions comprising 0.5% (w/v) PS80 are shown in FIG. 10. Results for compositions comprising 0.1% (w/v) poloxamer 188 are shown in FIG. 11. DS compositions comprising 0.5% (w/v) PS80 showed a moderate increase in aggregation after 5x F/T cycles at -80°C (see FIG. 10). However, DS compositions comprising 0.1% (w/v) poloxamer 188 showed no change in aggregation after 5x F/T cycles at -80°C (see FIG. 11). Compositions comprising either surfactant showed increases in aggregation after 5x F/T cycles at -30°C. However, the increase in aggregation was significantly greater in DS compositions comprising 0.5% (w/v) PS80 than compositions comprising 0.1% (w/v) poloxamer 188 (compare FIG. 10 and FIG. 11). Thus, it appears that 0.1% (w/v) poloxamer 188 as a surfactant in efanesoctocog alfa compositions may provide slightly improved F/T stability at -80°C and significantly improved F/T stability at -30°C over 0.5% (w/v) PS80 as surfactant.

A summary of the results of the aggregation testing for efanesoctocog alfa compositions comprising 0.1% (w/v) poloxamer 188 is provided in Table 1.

**Table 1: Summary of aggregation analysis (%HMWS) for 0.1% (w/v) poloxamer 188 formulations.**

| **Sucrose conc, %** | **Frozen Storage, % (-80°C) (1 mg/mL)** | | **Liquid DS Rate %/h (1 mg/mL)** | **Liquid BDP Rate%/h (4000 IU)** | | **Cum. %/h (4000 IU)** | **Lyo DP (Δ6M) (4000 IU)** | | | **Lyo DP (Δ6M) (250 IU)** | | |
|---|---|---|---|---|---|---|---|---|---|---|---|---|
| | **Δ6M** | **Δ5XFT** | **RT** | **5°C** | **RT** | **5°C/RT** | **5°C** | **30°C** | **40°C** | **5°C** | **30°C** | **40°C** |
| 5 | 0.5 | 0.2 | 0.00 | 0.00 | 0.05 | 0.02 | 0.2 | 0.0 | 0.0 | 1.2 | 0.8 | -0.3 |
| 2 | 0.7 | 1.3 | ND | ND | ND | ND | ND | ND | ND | ND | ND | ND |
| 1 | 0.3 | 1.3 | 0.08 | 0.03 | 0.04 | 0.03 | 0.2 | 0.3 | -0.1 | -0.2 | -0.1 | 3.34 |
| 0 | 0.7 | 0.4 | ND | ND | ND | ND | ND | ND | ND | ND | ND | ND |

| | | | | | | | | | | | | |
|---|---|---|---|---|---|---|---|---|---|---|---|---|
| ND = Not Determined | | | | | | | | | | | | |

Overall, these aggregation studies suggest that efanesoctocog alfa compositions may have improved stability when formulated with 0.1% (w/v) poloxamer 188 rather than 0.5% (w/v) PS80. poloxamer 188 compositions showed a significant improvement over PS80 compositions in F/T and Frozen Storage Stability assays at -30°C across all sucrose concentrations. Accordingly, poloxamer 188 may be preferable to PS80 for any efanesoctocog alfa compositions to be stored at -30°C. Efanesoctocog alfa DS and BDP liquid compositions also showed improved stability with poloxamer 188 compositions over PS80 compositions. For compositions of efanesoctocog alfa lyophilized DP, poloxamer 188 compositions at 4000IU showed favorable stability over 6 months at 40°C. However, PS80 lyophilized drug product showed significantly increased stability at 250 IU as compared to poloxamer 188.

### Example 2: Evaluation of additional stability attributes for efanesoctocog alfa compositions comprising different surfactants

In addition to aggregation, other measures of stability of efanesoctocog alfa compositions comprising different surfactants were evaluated.

Concentration of surfactant in efanesoctocog alfa compositions following F/T stress was analyzed by high performance liquid chromatography (HPLC). Compositions of efanesoctocog alfa DS at 1 mg/mL and comprising either 0.5% (w/v) PS80 or 0.1% (w/v) poloxamer 188 as surfactant and 0, 1, 2, or 5% (w/v) sucrose were subjected to stress by F/T for 5 F/T cycles at either -80°C or -30°C (not less than 24hrs, thaw at room temperature). Samples were tested before freezing (Pre Freeze) and after 1, 3, or 5 F/T cycles. Results for DS compositions containing 0.5% (w/v) PS80 and 0, 1, 2, or 5% (w/v) sucrose are shown in FIG. 12A. Results for DS compositions containing 0.1% (w/v) poloxamer 188 and 0, 1, 2, or 5% (w/v) sucrose are shown in FIG. 12B. No change in surfactant concentration was observed over all F/T cycles for either surfactant or sucrose concentration.

Change in pH of efanesoctocog alfa compositions following F/T stress was also analyzed. Compositions of efanesoctocog alfa DS at 1 mg/mL and comprising either 0.5% (w/v) PS80 or 0.1% (w/v) poloxamer 188 as surfactant and 0, 1, 2, or 5% (w/v) sucrose were subjected to stress by F/T for 5 F/T cycles at either -80°C or -30°C (not less than 24hrs, thaw at room temperature). Samples were tested before freezing (Pre Freeze) and after 1, 3, or 5 F/T cycles. Results of pH analysis are shown in FIG. 13. No significant pH trends or changes were observed for any composition tested.

Turbidity of compositions comprising efanesoctocog alfa DS at 1 mg/mL and poloxamer 188 were tested. Compositions comprising 1% or 5% sucrose were tested, and the results were compared to the turbidity results of efanesoctocog alfa DS at 1 mg/mL compositions comprising PS80. No trends or changes in turbidity were observed for any of the tested poloxamer 188 compositions (data not shown).

The glass transition temperature (Tg) was measured for efanesoctocog alfa lyophilized drug product (Lyo DP) compositions at 250 IU or 4000 IU and containing 0.1% (w/v) poloxamer 188 as a surfactant and 0, 1, 2, or 5% (w/v) sucrose. Tg was measured by modulated differential scanning calorimetry (DSC), according to the following steps: (i) equilibration at 15°C, (ii) modulate at 1°C every 60 seconds, (iii) isothermal for 5 minutes; and (iv) ramp at 3°C/min to 130°C. Residual moisture content (%) was also measured for all samples. Tg and residual moisture content results for Lyo DP 250 IU at T0 are shown in FIG. 14. Tg and residual moisture content results for Lyo DP 4000 IU at T0 are shown in FIG. 15.

Tg was determined for efanesoctocog alfa lyophilized drug product (Lyo DP) compositions at 4000 IU or 250 IU and containing 0.1% (w/v) poloxamer 188 as a surfactant and 5% or 1% (w/v) sucrose. Samples were held at 5°C, 30°C, or 40°C and tested at T0, 1 month, 2 months, 3 months, and 6 months. Results are shown in FIG. 16A (5°C), FIG. 16B (30°C), and FIG. 16C (40°C).

The glass transition temperature (Tg) was also determined for efanesoctocog alfa liquid bulk drug product (BDP) compositions at 250 IU or 4000 IU and containing either 0.5% (w/v) PS80 or 0.1% (w/v) poloxamer 188 as a surfactant and 0, 1, 2, or 5% (w/v) sucrose. Tg results for both surfactants at all strengths and sucrose concentrations are shown in FIG. 17.

For both 250 IU or 4000 IU compositions comprising either surfactant, Tg was generally observed to correlate with sucrose concentration, with the higher sucrose concentration showing lower Tg. No significant differences in Tg were observed based on surfactant type for either 250 IU or 4000 IU concentration.

Residual moisture content was determined for efanesoctocog alfa lyophilized drug product (Lyo DP) compositions at 250 IU or 4000 IU and containing 0.1% (w/v) poloxamer 188 as a surfactant and 1% or 5% (w/v) sucrose. Samples were held at 5°C, 30°C, or 40°C and tested at T0, 1 month, 2 months, 3 months, and 6 months. Results are shown in FIG. 18A (5°C), FIG. 18B (30°C), and FIG. 18C (40°C). No significant change in residual moisture content was observed for any of the poloxamer 188 compositions at any temperatures. All residual moisture values fell below the spec of 3% after 6 months.

Upon visual inspection, concentration of sucrose had no effect on poloxamer 188 formulation cake appearance over 6 months at 2-8°C, 30°C, and 40°C (data not shown).

The results of this study show that, except for aggregation, compositions of efanesoctocog alfa formulated with either 0.5% (w/v) PS80 or 0.1% (w/v) poloxamer 188 have similar stability attributes. Evaluation of other stability attributes such as glass transition temperature, turbidity, and residual moisture did not show any significant differences based on choice of surfactant.

### Example 3: Effect of arginine concentration on stability of efanesoctocog alfa compositions

Efanesoctocog alfa has demonstrated a tendency to undergo reversible self-association following freeze/thaw (F/T) stress. Use of arginine in efanesoctocog alfa compositions may provide a protective effect against this F/T induced molecular self association. To determine the optimal concentration of arginine for efanesoctocog alfa compositions, several compositions comprising various concentrations of arginine were prepared and aggregation levels were tested by SEC. Each tested composition comprised concentrated efanesoctocog alfa (>2 mg/mL), 10 mM L-histidine, 5 mM CaCl₂, 5% (w/v) sucrose, 0.1% (w/v) poloxamer 188 (P188), and L-arginine-HCl at a concentration of 125, 150, 175, 200, or 250 mM.

To prepare the compositions for testing, efanesoctocog alfa compositions were lyophilized using the LyoStar2 development-scale lyophilizer (Protein Pharmaceutical Development Laboratories, Cambridge, MA). To test each sample, compositions were thawed and injected immediately into the HPLC for SEC analysis. Samples were analyzed immediately after thawing, and after 40, 80, 120, and 160 minutes post-thaw.

The results of the aggregation assessments (%HMWS) for all samples are shown in FIG. 19. Efanesoctocog alfa compositions comprising arginine HCl levels below 250 mM demonstrate increased aggregation levels immediately upon thawing, followed by a decrease in aggregate levels over time (FIG. 18). This aggregation trend suggests formation and disassociation of HMWS that were formed due to F/T induced molecular self association of efanesoctocog alfa. However, efanesoctocog alfa compositions comprising arginine HCl concentrations of 250 mM demonstrate relatively low aggregation levels immediately after thawing, and these levels remain consistent over time (see FIG. 18, far right columns).

The results of this study indicate that efanesoctocog alfa compositions with arginine HCl concentrations of 250 mM may be better protected from F/T induced molecular self association. Thus 250 mM appears to be the preferred concentration of arginine HCl for use in formulating efanesoctocog alfa compositions.

### Example 4: Evaluation of pharmacokinetics of efanesoctocog alfa compositions comprising different surfactants in non-human primates

The objective of this study was to compare the pharmacokinetic (PK) profile of efanesoctocog alfa compositions comprising either polysorbate 80 (PS80) or poloxamer 188 (P188) in cynomolgus monkeys.

The following two vehicles were tested:
Vehicle 1: 10mM L-Histidine, 250mM Arginine-HCl, 5mM Calcium Chloride, 5% w/v Sucrose, 0.05% Polysorbate 80
Vehicle 2: 10mM L-Histidine, 250mM Arginine-HCl, 5mM Calcium Chloride, 5% w/v Sucrose, 0.1% Poloxamer 188

The first group of subjects were administered a single intravenous (IV) bolus injection of 75 IU/kg/dose of efanesoctocog alfa in Vehicle 1. The second group of subjects were administered a single intravenous (IV) bolus injection of 75 IU/kg/dose of efanesoctocog alfa in Vehicle 2. Both compositions included efanesoctocog alfa at a concentration of 37.5 IU/mL. A volume of 2 mL was administered to each subject. Blood samples were collected for PK analysis at Predose, 0.25, 1, 3, 8, 24, 48, 96, 168, 240, and 336 hours.

For the dose formulations, the concentrations of formulation samples analyzed were 88.6% and 92.0% of their respective theoretical concentration, for groups 1 and 2, respectively. The chromogenic activity of the formulation samples analyzed (following repeat analysis) were on a lower range and were 70.5% and 64.9% of their respective theoretical concentration, for groups 1 and 2, respectively.

Efanesoctocog alfa protein concentration was determined by ELISA. Efanesoctocog alfa protein concentration over time is shown in FIG. 20. Efanesoctocog alfa protein activity was determined using the chromogenic assay. Efanesoctocog alfa activity over time is shown in FIG. 21. Both protein concentration and activity profiles were comparable for compositions comprising either PS80 or P188.

PK parameters for efanesoctocog alfa protein concentration and activity were determined and are presented in Table 2. Values are presented as mean (±SD) and were generated from 4 animals for each group. As can be seen in Table 2, PK parameters were comparable for compositions comprising either PS80 or P188.

Efanesoctocog alfa protein concentration and activity parameters were directly compared between subjects receiving P188 or PS80 compositions. For protein concentration, the Cₘₐₓ ratio and AUC₍₀₋ₜ₎ ratio were determined to be 91.8% and 89.8%, respectively. For chromogenic activity, the Cₘₐₓ ratio and AUC₍₀₋ₜ₎ ratio were determined to be 106% and 105%, respectively.

The Tₘₐₓ was observed at first time point after dosing (i.e., 0.25 hour post dose) for efanesoctocog alfa and efanesoctocog alfa activity. The mean Cₘₐₓ was 841 and 772 ng/mL for efanesoctocog alfa concentration and 2110 and 2230 mIU/mL for efanesoctocog alfa activity, with Vehicle 1 and 2, respectively. The mean AUC₍₀₋ₜ₎ was 25400 and 22800 hr*ng/mL for efanesoctocog alfa concentration and 59600 and 62800 hr*mIU/mL for efanesoctocog alfa activity, with Vehicle 1 and 2, respectively. The mean t1/2 was 36.1 and 35.8 hours for efanesoctocog alfa concentration and 34.4 and 35.3 hours for efanesoctocog alfa activity, with vehicle 1 and 2, respectively. The mean CLₚₗₐₛₘₐ was 1.33 and 1.44 mL/hr/kg for efanesoctocog alfa concentration and 1.29 and 1.20 mL/hr/kg for efanesoctocog alfa activity, with vehicle 1 and 2, respectively. The mean Vd was 69.0 and 74.4 mL/kg for efanesoctocog alfa concentration and 63.8 and 61.4 mL/kg for BIVV001 activity, with vehicle 1 and 2, respectively.

In conclusion, the PK parameters of efanesoctocog alfa compositions were relatively similar regardless of selection of vehicle between PS80 and P188. There were no test item-related clinical signs or any injection site irritation noted following administration to subjects. All toxicokinetic parameters for each group were generated from 4 animals. Values are rounded to 3 significant figures (except Tₘₐₓ).

**Table 2. PK parameters for efanesoctocog alfa compositions in cynomolgus monkeys.**

| **Analyte** | **Vehicle** | **Cₘₐₓ (ng/mL)** | **Tₘₐₓ Median (range) (hr)** | **AUC_{(0 - t)} (hr*ng/mL)** | **t_{1/2} (hr)** | **CLₚₗₐₛₘₐ (mL/hr/kg)** | **Vd (mL/kg)** |
|---|---|---|---|---|---|---|---|
| Efa concentration | PS80 | 841 ± 119 | 0.25 (0.25 - 0.25) | 25400 ± 4940 | 36.1 ± 3.53 | 1.33 ± 0.300 | 69.0 ± 14.2 |
| | P188 | 772 ± 101 | 0.25 (0.25 - 0.25) | 22800 ± 2200 | 35.8 ± 5.50 | 1.44 ± 0.133 | 74.4 ± 15.4 |

| **Analyte** | **Vehicle** | **Cₘₐₓ (mIU/mL)** | **Tₘₐₓ Median (range) (hr)** | **AUC_{(0 - t)} (hr*mIU /mL)** | **t_{1/2} (hr)** | **CLₚₗₐₛₘₐ (mL/hr/kg)** | **Vd (mL/kg)** |
|---|---|---|---|---|---|---|---|
| Efa Activity | PS80 | 2110 ± 382 | 0.25 (0.25 - 0.25) | 59600 ± 14300 | 34.4 ± 3.05 | 1.29 ± 0.276 | 63.8 ± 11.7 |
| | P188 | 2230 ± 342 | 0.25 (0.25 - 0.25) | 62800 ± 8640 | 35.3 ± 5.96 | 1.20 ± 0.153 | 61.4 ± 16.2 |

The foregoing description of the specific embodiments will so fully reveal the general nature of the disclosure that others can, by applying knowledge within the skill of the art, readily modify and/or adapt for various applications such specific embodiments, without undue experimentation, without departing from the general concept of the present disclosure. Therefore, such adaptations and modifications are intended to be within the meaning and range of equivalents of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

Other embodiments of the disclosure will be apparent to those skilled in the art from consideration of the specification and practice of the disclosure disclosed herein. It is intended that the specification and examples be considered as exemplary only, with a true scope and spirit of the disclosure being indicated by the following claims.

All patents and publications cited herein are incorporated by reference herein in their entirety.

### SEQUENCES

**Table 3: Exemplary chimeric protein sequences**

| **A-FVIII(ELNN)-Fc: SEQ ID NO: 1** |
|---|
| ATRRYYLGAV ELSWDYMQSD LGELPVDARF PPRVPKSFPF NTSVVYKKTL FVEFTDHLFN 60 IAKPRPPWMG LLGPTIQAEV YDTVVITLKN MASHPVSLHA VGVSYWKASE GAEYDDQTSQ 120 REKEDDKVFP GGSHTYVWQV LKENGPMASD PLCLTYSYLS HVDLVKDLNS GLIGALLVCR 180 EGSLAKEKTQ TLHKFILLFA VFDEGKSWHS ETKNSLMQDR DAASARAWPK MHTVNGYVNR 240 SLPGLIGCHR KSVYWHVIGM GTTPEVHSIF LEGHTFLVRN HRQASLEISP ITFLTAQTLL 300 MDLGQFLLFC HISSHQHDGM EAYVKVDSCP EEPQLRMKNN EEAEDYDDDL TDSEMDVVRF 360 DDDNSPSFIQ IRSVAKKHPK TWVHYIAAEE EDWDYAPLVL APDDRSYKSQ YLNNGPQRIG 420 RKYKKVRFMA YTDETFKTRE AIQHESGILG PLLYGEVGDT LLIIFKNQAS RPYNIYPHGI 480 TDVRPLYSRR LPKGVKHLKD FPILPGEIFK YKWTVTVEDG PTKSDPRCLT RYYSSFVNME 540 RDLASGLIGP LLICYKESVD QRGNQIMSDK RNVILFSVFD ENRSWYLTEN IQRFLPNPAG 600 VQLEDPEFQA SNIMHSINGY VFDSLQLSVC LHEVAYWYIL SIGAQTDFLS VFFSGYTFKH 660 KMVYEDTLTL FPFSGETVFM SMENPGLWIL GCHNSDFRNR GMTALLKVSS CDKNTGDYYE 720 DSYEDISAYL LSKNNAIEPR SFSQNGTSES ATPESGPGSE PATSGSETPG TSESATPESG 780 PGSEPATSGS ETPGTSESAT PESGPGTSTE PSEGSAPGSP AGSPTSTEEG TSESATPESG 840 PGSEPATSGS ETPGTSESAT PESGPGSPAG SPTSTEEGSP AGSPTSTEEG TSTEPSEGSA 900 PGTSESATPE SGPGTSESAT PESGPGTSES ATPESGPGSE PATSGSETPG SEPATSGSET 960 PGSPAGSPTS TEEGTSTEPS EGSAPGTSTE PSEGSAPGSE PATSGSETPG TSESATPESG 1020 PGTSTEPSEG SAPASSEITR TTLQSDQEEI DYDDTISVEM KKEDFDIYDE DENQSPRSFQ 1080 KKTRHYFIAA VERLWDYGMS SSPHVLRNRA QSGSVPQFKK VVFQEFTDGS FTQPLYRGEL 1140 NEHLGLLGPY IRAEVEDNIM VTFRNQASRP YSFYSSLISY EEDQRQGAEP RKNFVKPNET 1200 KTYFWKVQHH MAPTKDEFDC KAWAYFSDVD LEKDVHSGLI GPLLVCHTNT LNPAHGRQVT 1260 VQEFALFFTI FDETKSWYFT ENMERNCRAP CNIQMEDPTF KENYRFHAIN GYIMDTLPGL 1320 VMAQDQRIRW YLLSMGSNEN IHSIHFSGHV FTVRKKEEYK MALYNLYPGV FETVEMLPSK 1380 AGIWRVECLI GEHLHAGMST LFLVYSNKCQ TPLGMASGHI RDFQITASGQ YGQWAPKLAR 1440 LHYSGSINAW STKEPFSWIK VDLLAPMIIH GIKTQGARQK FSSLYISQFI IMYSLDGKKW 1500 QTYRGNSTGT LMVFFGNVDS SGIKHNIFNP PIIARYIRLH PTHYSIRSTL RMELMGCDLN 1560 SCSMPLGMES KAISDAQITA SSYFTNMFAT WSPSKARLHL QGRSNAWRPQ VNNPKEWLQV 1620 DFQKTMKVTG VTTQGVKSLL TSMYVKEFLI SSSQDGHQWT LFFQNGKVKV FQGNQDSFTP 1680 VVNSLDPPLL TRYLRIHPQS WVHQIALRME VLGCEAQDLY DKTHTCPPCP APELLGGPSV 1740 FLFPPKPKDT LMISRTPEVT CVVVDVSHED PEVKFNWYVD GVEVHNAKTK PREEQYNSTY 1800 RVVSVLTVLH QDWLNGKEYK CKVSNKALPA PIEKTISKAK GQPREPQVYT LPPSRDELTK 1860 NQVSLTCLVK GFYPSDIAVE WESNGQPENN YKTTPPVLDS DGSFFLYSKL TVDKSRWQQG 1920 NVFSCSVMHE ALHNHYTQKS LSLSPG |

| **VWF(D'D3)-ELNN-a2 Linker-Fc: SEQ ID NO: 2** |
|---|
| SLSCRPPMVK LVCPADNLRA EGLECTKTCQ NYDLECMSMG CVSGCLCPPG MVRHENRCVA 60 LERCPCFHQG KEYAPGETVK IGCNTCVCRD RKWNCTDHVC DATCSTIGMA HYLTFDGLKY 120 LFPGECQYVL VQDYCGSNPG TFRILVGNKG CSHPSVKCKK RVTILVEGGE IELFDGEVNV 180 KRPMKDETHF EVVESGRYII LLLGKALSVV WDRHLSISVV LKQTYQEKVC GLCGNFDGIQ 240 NNDLTSSNLQ VEEDPVDFGN SWKVSSQCAD TRKVPLDSSP ATCHNNIMKQ TMVDSSCRIL 300 TSDVFQDCNK LVDPEPYLDV CIYDTCSCES IGDCAAFCDT IAAYAHVCAQ HGKVVTWRTA 360 TLCPQSCEER NLRENGYEAE WRYNSCAPAC QVTCQHPEPL ACPVQCVEGC HAHCPPGKIL 420 DELLQTCVDP EDCPVCEVAG RRFASGKKVT LNPSDPEHCQ ICHCDVVNLT CEACQEPGTS 480 ESATPESGPG SEPATSGSET PGTSESATPE SGPGSEPATS GSETPGTSES ATPESGPGTS 540 TEPSEGSAPG SPAGSPTSTE EGTSESATPE SGPGSEPATS GSETPGTSES ATPESGPGSP 600 AGSPTSTEEG SPAGSPTSTE EGASSDKNTG DYYEDSYEDI SAYLLSKNNA IEPRSFSDKT 660 HTCPPCPAPE LLGGPSVFLF PPKPKDTLMI SRTPEVTCVV VDVSHEDPEV KFNWYVDGVE 720 VHNAKTKPRE EQYNSTYRVV SVLTVLHQDW LNGKEYKCKV SNKALPAPIE KTISKAKGQP 780 REPQVYTLPP SRDELTKNQV SLTCLVKGFY PSDIAVEWES NGQPENNYKT TPPVLDSDGS 840 FFLYSKLTVD KSRWQQGNVF SCSVMHEALH NHYTQKSLSL SPG 883 |

| **Amino acid sequence of FVIII protein (312A) - includes signal peptide: SEQ ID NO: 3** |
|---|
| 1 MQIELSTCFF LCLLRFCFSA TRRYYLGAVE LSWDYMQSDL GELPVDARFP |
| 51 PRVPKSFPFN TSVVYKKTLF VEFTDHLFNI AKPRPPWMGL LGPTIQAEVY 101 DTVVITLKNM ASHPVSLHAV GVSYWKASEG AEYDDQTSQR EKEDDKVFPG 151 GSHTYVWQVL KENGPMASDP LCLTYSYLSH VDLVKDLNSG LIGALLVCRE 201 GSLAKEKTQT LHKFILLFAV FDEGKSWHSE TKNSLMQDRD AASARAWPKM 251 HTVNGYVNRS LPGLIGCHRK SVYWHVIGMG TTPEVHSIFL EGHTFLVRNH 301 RQASLEISPI TFLTAQTLLM DLGQFLLFCH ISSHQHDGME AYVKVDSCPE 351 EPQLRMKNNE EAEDYDDDLT DSEMDVVRFD DDNSPSFIQI RSVAKKHPKT 401 WVHYIAAEEE DWDYAPLVLA PDDRSYKSQY LNNGPQRIGR KYKKVRFMAY 451 TDETFKTREA IQHESGILGP LLYGEVGDTL LIIFKNQASR PYNIYPHGIT 501 DVRPLYSRRL PKGVKHLKDF PILPGEIFKY KWTVTVEDGP TKSDPRCLTR 551 YYSSFVNMER DLASGLIGPL LICYKESVDQ RGNQIMSDKR NVILFSVFDE 601 NRSWYLTENI QRFLPNPAGV QLEDPEFQAS NIMHSINGYV FDSLQLSVCL 651 HEVAYWYILS IGAQTDFLSV FFSGYTFKHK MVYEDTLTLF PFSGETVFMS 701 MENPGLWILG CHNSDFRNRG MTALLKVSSC DKNTGDYYED SYEDISAYLL 751 SKNNAIEPRS FSQNGTSESA TPESGPGSEP ATSGSETPGT SESATPESGP 801 GSEPATSGSE TPGTSESATP ESGPGTSTEP SEGSAPGSPA GSPTSTEEGT 851 SESATPESGP GSEPATSGSE TPGTSESATP ESGPGSPAGS PTSTEEGSPA 901 GSPTSTEEGT STEPSEGSAP GTSESATPES GPGTSESATP ESGPGTSESA 951 TPESGPGSEP ATSGSETPGS EPATSGSETP GSPAGSPTST EEGTSTEPSE 1001 GSAPGTSTEP SEGSAPGSEP ATSGSETPGT SESATPESGP GTSTEPSEGS 1051 APASSEITRT TLQSDQEEID YDDTISVEMK KEDFDIYDED ENQSPRSFQK 1101 KTRHYFIAAV ERLWDYGMSS SPHVLRNRAQ SGSVPQFKKV VFQEFTDGSF 1151 TQPLYRGELN EHLGLLGPYI RAEVEDNIMV TFRNQASRPY SFYSSLISYE 1201 EDQRQGAEPR KNFVKPNETK TYFWKVQHHM APTKDEFDCK AWAYFSDVDL 1251 EKDVHSGLIG PLLVCHTNTL NPAHGRQVTV QEFALFFTIF DETKSWYFTE 1301 NMERNCRAPC NIQMEDPTFK ENYRFHAING YIMDTLPGLV MAQDQRIRWY 1351 LLSMGSNENI HSIHFSGHVF TVRKKEEYKM ALYNLYPGVF ETVEMLPSKA 1401 GIWRVECLIG EHLHAGMSTL FLVYSNKCQT PLGMASGHIR DFQITASGQY 1451 GQWAPKLARL HYSGSINAWS TKEPFSWIKV DLLAPMIIHG IKTQGARQKF 1501 SSLYISQFII MYSLDGKKWQ TYRGNSTGTL MVFFGNVDSS GIKHNIFNPP 1551 IIARYIRLHP THYSIRSTLR MELMGCDLNS CSMPLGMESK AISDAQITAS 1601 SYFTNMFATW SPSKARLHLQ GRSNAWRPQV NNPKEWLQVD FQKTMKVTGV 1651 TTQGVKSLLT SMYVKEFLIS SSQDGHQWTL FFQNGKVKVF QGNQDSFTPV 1701 VNSLDPPLLT RYLRIHPQSW VHQIALRMEV LGCEAQDLYD KTHTCPPCPA 1751 PELLGGPSVF LFPPKPKDTL MISRTPEVTC VVVDVSHEDP EVKFNWYVDG 1801 VEVHNAKTKP REEQYNSTYR VVSVLTVLHQ DWLNGKEYKC KVSNKALPAP 1851 IEKTISKAKG QPREPQVYTL PPSRDELTKN QVSLTCLVKG FYPSDIAVEW 1901 ESNGQPENNY KTTPPVLDSD GSFFLYSKLT VDKSRWQQGN VFSCSVMHEA 1951 LHNHYTQKSL SLSPG* |

| **Nucleotide sequence encoding FVIII 312A: SEQ ID NO: 4** |
|---|
| 1 ATGCAAATAG AGCTCTCCAC CTGCTTCTTT CTGTGCCTTT TGCGATTCTG 51 CTTTAGTGCC ACCAGAAGAT ACTACCTGGG TGCAGTGGAA CTGTCATGGG 101 ACTATATGCA AAGTGATCTC GGTGAGCTGC CTGTGGACGC AAGATTTCCT 151 CCTAGAGTGC CAAAATCTTT TCCATTCAAC ACCTCAGTCG TGTACAAAAA 201 GACTCTGTTT GTAGAATTCA CGGATCACCT TTTCAACATC GCTAAGCCAA 251 GGCCACCCTG GATGGGTCTG CTAGGTCCTA CCATCCAGGC TGAGGTTTAT 301 GATACAGTGG TCATTACACT TAAGAACATG GCTTCCCATC CTGTCAGTCT 351 TCATGCTGTT GGTGTATCCT ACTGGAAAGC TTCTGAGGGA GCTGAATATG 401 ATGATCAGAC CAGTCAAAGG GAGAAAGAAG ATGATAAAGT CTTCCCTGGT 451 GGAAGCCATA CATATGTCTG GCAGGTCCTG AAAGAGAATG GTCCAATGGC 501 CTCTGACCCA CTGTGCCTTA CCTACTCATA TCTTTCTCAT GTGGACCTGG 551 TAAAAGACTT GAATTCAGGC CTCATTGGAG CCCTACTAGT ATGTAGAGAA 601 GGGAGTCTGG CCAAGGAAAA GACACAGACC TTGCACAAAT TTATACTACT 651 TTTTGCTGTA TTTGATGAAG GGAAAAGTTG GCACTCAGAA ACAAAGAACT |
| 701 CCTTGATGCA GGATAGGGAT GCTGCATCTG CTCGGGCCTG GCCTAAAATG 751 CACACAGTCA ATGGTTATGT AAACAGGTCT CTGCCAGGTC TGATTGGATG 801 CCACAGGAAA TCAGTCTATT GGCATGTGAT TGGAATGGGC ACCACTCCTG 851 AAGTGCACTC AATATTCCTC GAAGGTCACA CATTTCTTGT GAGGAACCAT 901 CGCCAGGCGT CCTTGGAAAT CTCGCCAATA ACTTTCCTTA CTGCTCAAAC 951 ACTCTTGATG GACCTTGGAC AGTTTCTACT GTTTTGTCAT ATCTCTTCCC 1001 ACCAACATGA TGGCATGGAA GCTTATGTCA AAGTAGACAG CTGTCCAGAG 1051 GAACCCCAAC TACGAATGAA AAATAATGAA GAAGCGGAAG ACTATGATGA 1101 TGATCTTACT GATTCTGAAA TGGATGTGGT CAGGTTTGAT GATGACAACT 1151 CTCCTTCCTT TATCCAAATT CGCTCAGTTG CCAAGAAGCA TCCTAAAACT 1201 TGGGTACATT ACATTGCTGC TGAAGAGGAG GACTGGGACT ATGCTCCCTT 1251 AGTCCTCGCC CCCGATGACA GAAGTTATAA AAGTCAATAT TTGAACAATG 1301 GCCCTCAGCG GATTGGTAGG AAGTACAAAA AAGTCCGATT TATGGCATAC 1351 ACAGATGAAA CCTTTAAGAC TCGTGAAGCT ATTCAGCATG AATCAGGAAT 1401 CTTGGGACCT TTACTTTATG GGGAAGTTGG AGACACACTG TTGATTATAT 1451 TTAAGAATCA AGCAAGCAGA CCATATAACA TCTACCCTCA CGGAATCACT 1501 GATGTCCGTC CTTTGTATTC AAGGAGATTA CCAAAAGGTG TAAAACATTT 1551 GAAGGATTTT CCAATTCTGC CAGGAGAAAT ATTCAAATAT AAATGGACAG 1601 TGACTGTAGA AGATGGGCCA ACTAAATCAG ATCCTCGGTG CCTGACCCGC 1651 TATTACTCTA GTTTCGTTAA TATGGAGAGA GATCTAGCTT CAGGACTCAT 1701 TGGCCCTCTC CTCATCTGCT ACAAAGAATC TGTAGATCAA AGAGGAAACC 1751 AGATAATGTC AGACAAGAGG AATGTCATCC TGTTTTCTGT ATTTGATGAG 1801 AACCGAAGCT GGTACCTCAC AGAGAATATA CAACGCTTTC TCCCCAATCC 1851 AGCTGGAGTG CAGCTTGAGG ATCCAGAGTT CCAAGCCTCC AACATCATGC 1901 ACAGCATCAA TGGCTATGTT TTTGATAGTT TGCAGTTGTC AGTTTGTTTG 1951 CATGAGGTGG CATACTGGTA CATTCTAAGC ATTGGAGCAC AGACTGACTT 2001 CCTTTCTGTC TTCTTCTCTG GATATACCTT CAAACACAAA ATGGTCTATG 2051 AAGACACACT CACCCTATTC CCATTCTCAG GAGAAACTGT CTTCATGTCG 2101 ATGGAAAACC CAGGTCTATG GATTCTGGGG TGCCACAACT CAGACTTTCG 2151 GAACAGAGGC ATGACCGCCT TACTGAAGGT TTCTAGTTGT GACAAGAACA 2201 CTGGTGATTA TTACGAGGAC AGTTATGAAG ATATTTCAGC ATACTTGCTG 2251 AGTAAAAACA ATGCCATTGA ACCAAGAAGC TTCTCTCAAA ACGGTACCTC 2301 AGAGTCTGCT ACCCCCGAGT CAGGGCCAGG ATCAGAGCCA GCCACCTCCG 2351 GGTCTGAGAC ACCCGGGACT TCCGAGAGTG CCACCCCTGA GTCCGGACCC 2401 GGGTCCGAGC CCGCCACTTC CGGCTCCGAA ACTCCCGGCA CAAGCGAGAG 2451 CGCTACCCCA GAGTCAGGAC CAGGAACATC TACAGAGCCC TCTGAAGGCT 2501 CCGCTCCAGG GTCCCCAGCC GGCAGTCCCA CTAGCACCGA GGAGGGAACC 2551 TCTGAAAGCG CCACACCCGA ATCAGGGCCA GGGTCTGAGC CTGCTACCAG 2601 CGGCAGCGAG ACACCAGGCA CCTCTGAGTC CGCCACACCA GAGTCCGGAC 2651 CCGGATCTCC CGCTGGGAGC CCCACCTCCA CTGAGGAGGG ATCTCCTGCT 2701 GGCTCTCCAA CATCTACTGA GGAAGGAACC TCAACCGAGC CATCCGAGGG 2751 ATCAGCTCCC GGCACCTCAG AGTCGGCAAC CCCGGAGTCT GGACCCGGAA 2801 CTTCCGAAAG TGCCACACCA GAGTCCGGTC CCGGGACTTC AGAATCAGCA 2851 ACACCCGAGT CCGGCCCTGG GTCTGAACCC GCCACAAGTG GTAGTGAGAC 2901 ACCAGGATCA GAACCTGCTA CCTCAGGGTC AGAGACACCC GGATCTCCGG 2951 CAGGCTCACC AACCTCCACT GAGGAGGGCA CCAGCACAGA ACCAAGCGAG 3001 GGCTCCGCAC CCGGAACAAG CACTGAACCC AGTGAGGGTT CAGCACCCGG 3051 CTCTGAGCCG GCCACAAGTG GCAGTGAGAC ACCCGGCACT TCAGAGAGTG 3101 CCACCCCCGA GAGTGGCCCA GGCACTAGTA CCGAGCCCTC TGAAGGCAGT 3151 GCGCCAGCCT CGAGCGAAAT AACTCGTACT ACTCTTCAGT CAGATCAAGA 3201 GGAAATTGAC TATGATGATA CCATATCAGT TGAAATGAAG AAGGAAGATT 3251 TTGACATTTA TGATGAGGAT GAAAATCAGA GCCCCCGCAG CTTTCAAAAG 3301 AAAACACGAC ACTATTTTAT TGCTGCAGTG GAGAGGCTCT GGGATTATGG 3351 GATGAGTAGC TCCCCACATG TTCTAAGAAA CAGGGCTCAG AGTGGCAGTG 3401 TCCCTCAGTT CAAGAAAGTT GTTTTCCAGG AATTTACTGA TGGCTCCTTT 3451 ACTCAGCCCT TATACCGTGG AGAACTAAAT GAACATTTGG GACTCCTGGG 3501 GCCATATATA AGAGCAGAAG TTGAAGATAA TATCATGGTA ACTTTCAGAA 3551 ATCAGGCCTC TCGTCCCTAT TCCTTCTATT CTAGCCTTAT TTCTTATGAG |
| 3601 GAAGATCAGA GGCAAGGAGC AGAACCTAGA AAAAACTTTG TCAAGCCTAA 3651 TGAAACCAAA ACTTACTTTT GGAAAGTGCA ACATCATATG GCACCCACTA 3701 AAGATGAGTT TGACTGCAAA GCCTGGGCTT ATTTCTCTGA TGTTGACCTG 3751 GAAAAAGATG TGCACTCAGG CCTGATTGGA CCCCTTCTGG TCTGCCACAC 3801 TAACACACTG AACCCTGCTC ATGGGAGACA AGTGACAGTA CAGGAATTTG 3851 CTCTGTTTTT CACCATCTTT GATGAGACCA AAAGCTGGTA CTTCACTGAA 3901 AATATGGAAA GAAACTGCAG GGCTCCCTGC AATATCCAGA TGGAAGATCC 3951 CACTTTTAAA GAGAATTATC GCTTCCATGC AATCAATGGC TACATAATGG 4001 ATACACTACC TGGCTTAGTA ATGGCTCAGG ATCAAAGGAT TCGATGGTAT 4051 CTGCTCAGCA TGGGCAGCAA TGAAAACATC CATTCTATTC ATTTCAGTGG 4101 ACATGTGTTC ACTGTACGAA AAAAAGAGGA GTATAAAATG GCACTGTACA 4151 ATCTCTATCC AGGTGTTTTT GAGACAGTGG AAATGTTACC ATCCAAAGCT 4201 GGAATTTGGC GGGTGGAATG CCTTATTGGC GAGCATCTAC ATGCTGGGAT 4251 GAGCACACTT TTTCTGGTGT ACAGCAATAA GTGTCAGACT CCCCTGGGAA 4301 TGGCTTCTGG ACACATTAGA GATTTTCAGA TTACAGCTTC AGGACAATAT 4351 GGACAGTGGG CCCCAAAGCT GGCCAGACTT CATTATTCCG GATCAATCAA 4401 TGCCTGGAGC ACCAAGGAGC CCTTTTCTTG GATCAAGGTG GATCTGTTGG 4451 CACCAATGAT TATTCACGGC ATCAAGACCC AGGGTGCCCG TCAGAAGTTC 4501 TCCAGCCTCT ACATCTCTCA GTTTATCATC ATGTATAGTC TTGATGGGAA 4551 GAAGTGGCAG ACTTATCGAG GAAATTCCAC TGGAACCTTA ATGGTCTTCT 4601 TTGGCAATGT GGATTCATCT GGGATAAAAC ACAATATTTT TAACCCTCCA 4651 ATTATTGCTC GATACATCCG TTTGCACCCA ACTCATTATA GCATTCGCAG 4701 CACTCTTCGC ATGGAGTTGA TGGGCTGTGA TTTAAATAGT TGCAGCATGC 4751 CATTGGGAAT GGAGAGTAAA GCAATATCAG ATGCACAGAT TACTGCTTCA 4801 TCCTACTTTA CCAATATGTT TGCCACCTGG TCTCCTTCAA AAGCTCGACT 4851 TCACCTCCAA GGGAGGAGTA ATGCCTGGAG ACCTCAGGTG AATAATCCAA 4901 AAGAGTGGCT GCAAGTGGAC TTCCAGAAGA CAATGAAAGT CACAGGAGTA 4951 ACTACTCAGG GAGTAAAATC TCTGCTTACC AGCATGTATG TGAAGGAGTT 5001 CCTCATCTCC AGCAGTCAAG ATGGCCATCA GTGGACTCTC TTTTTTCAGA 5051 ATGGCAAAGT AAAGGTTTTT CAGGGAAATC AAGACTCCTT CACACCTGTG 5101 GTGAACTCTC TAGACCCACC GTTACTGACT CGCTACCTTC GAATTCACCC 5151 CCAGAGTTGG GTGCACCAGA TTGCCCTGAG GATGGAGGTT CTGGGCTGCG 5201 AGGCACAGGA CCTCTACGAC AAAACTCACA CATGCCCACC GTGCCCAGCT 5251 CCAGAACTCC TGGGCGGACC GTCAGTCTTC CTCTTCCCCC CAAAACCCAA 5301 GGACACCCTC ATGATCTCCC GGACCCCTGA GGTCACATGC GTGGTGGTGG 5351 ACGTGAGCCA CGAAGACCCT GAGGTCAAGT TCAACTGGTA TGTGGACGGC 5401 GTGGAAGTGC ATAATGCCAA GACAAAGCCG CGGGAGGAGC AGTACAACAG 5451 CACGTACCGT GTGGTCAGCG TCCTCACCGT CCTGCACCAA GACTGGCTGA 5501 ATGGCAAGGA GTACAAGTGC AAGGTCTCCA ACAAAGCCCT CCCAGCCCCC 5551 ATCGAGAAAA CCATCTCCAA AGCCAAAGGG CAGCCCCGAG AACCACAGGT 5601 GTACACCCTG CCCCCATCCC GGGATGAGCT GACCAAGAAC CAAGTTAGCC 5651 TGACCTGCCT GGTCAAAGGC TTCTATCCCA GCGACATCGC CGTGGAGTGG 5701 GAGAGCAATG GGCAGCCGGA GAACAACTAC AAGACCACGC CTCCCGTGTT 5751 GGACTCCGAC GGCTCCTTCT TCCTCTACTC CAAGCTCACC GTGGACAAGA 5801 GCAGGTGGCA GCAGGGGAAC GTCTTCTCAT GCTCCGTGAT GCATGAGGCT 5851 CTGCACAACC ACTACACGCA GAAGAGCCTC TCCCTGTCTC CGGGTTGA |

| **Amino acid sequence of VWF059A protein - includes signal peptide and D1D2 region: SEQ ID NO: 5** |
|---|
| 1     MIPARFAGVL LALALILPGT LCAEGTRGRS STARCSLFGS DFVNTFDGSM 51    YSFAGYCSYL LAGGCQKRSF SIIGDFQNGK RVSLSVYLGE FFDIHLFVNG 101   TVTQGDQRVS MPYASKGLYL ETEAGYYKLS GEAYGFVARI DGSGNFQVLL 151   SDRYFNKTCG LCGNFNIFAE DDFMTQEGTL TSDPYDFANS WALSSGEQWC 201   ERASPPSSSC NISSGEMQKG LWEQCQLLKS TSVFARCHPL VDPEPFVALC 251   EKTLCECAGG LECACPALLE YARTCAQEGM VLYGWTDHSA CSPVCPAGME |
| 301 YRQCVSPCAR TCQSLHINEM CQERCVDGCS CPEGQLLDEG LCVESTECPC 351 VHSGKRYPPG TSLSRDCNTC ICRNSQWICS NEECPGECLV TGQSHFKSFD 401 NRYFTFSGIC QYLLARDCQD HSFSIVIETV QCADDRDAVC TRSVTVRLPG 451 LHNSLVKLKH GAGVAMDGQD IQLPLLKGDL RIQHTVTASV RLSYGEDLQM 501 DWDGRGRLLV KLSPVYAGKT CGLCGNYNGN QGDDFLTPSG LAEPRVEDFG 551 NAWKLHGDCQ DLQKQHSDPC ALNPRMTRFS EEACAVLTSP TFEACHRAVS 601 PLPYLRNCRY DVCSCSDGRE CLCGALASYA AACAGRGVRV AWREPGRCEL 651 NCPKGQVYLQ CGTPCNLTCR SLSYPDEECN EACLEGCFCP PGLYMDERGD 701 CVPKAQCPCY YDGEIFQPED IFSDHHTMCY CEDGFMHCTM SGVPGSLLPD 751 AVLSSPLSHR SKRSLSCRPP MVKLVCPADN LRAEGLECTK TCQNYDLECM 801 SMGCVSGCLC PPGMVRHENR CVALERCPCF HQGKEYAPGE TVKIGCNTCV 851 CRDRKWNCTD HVCDATCSTI GMAHYLTFDG LKYLFPGECQ YVLVQDYCGS 901 NPGTFRILVG NKGCSHPSVK CKKRVTILVE GGEIELFDGE VNVKRPMKDE 951 THFEVVESGR YIILLLGKAL SVVWDRHLSI SVVLKQTYQE KVCGLCGNFD 1001 GIQNNDLTSS NLQVEEDPVD FGNSWKVSSQ CADTRKVPLD SSPATCHNNI 1051 MKQTMVDSSC RILTSDVFQD CNKLVDPEPY LDVCIYDTCS CESIGDCAAF 1101 CDTIAAYAHV CAQHGKVVTW RTATLCPQSC EERNLRENGY EAEWRYNSCA 1151 PACQVTCQHP EPLACPVQCV EGCHAHCPPG KILDELLQTC VDPEDCPVCE 1201 VAGRRFASGK KVTLNPSDPE HCQICHCDVV NLTCEACQEP GTSESATPES 1251 GPGSEPATSG SETPGTSESA TPESGPGSEP ATSGSETPGT SESATPESGP 1301 GTSTEPSEGS APGSPAGSPT STEEGTSESA TPESGPGSEP ATSGSETPGT 1351 SESATPESGP GSPAGSPTST EEGSPAGSPT STEEGASSDK NTGDYYEDSY 1401 EDISAYLLSK NNAIEPRSFS DKTHTCPPCP APELLGGPSV FLFPPKPKDT 1451 LMISRTPEVT CVVVDVSHED PEVKFNWYVD GVEVHNAKTK PREEQYNSTY 1501 RVVSVLTVLH QDWLNGKEYK CKVSNKALPA PIEKTISKAK GQPREPQVYT 1551 LPPSRDELTK NQVSLTCLVK GFYPSDIAVE WESNGQPENN YKTTPPVLDS 1601 DGSFFLYSKL TVDKSRWQQG NVFSCSVMHE ALHNHYTQKS LSLSPG* |

| **Nucleotide sequence encoding VWF059A protein: SEQ ID NO: 6** |
|---|
| 1 ATGATTCCTG CCAGATTTGC CGGGGTGCTG CTTGCTCTGG CCCTCATTTT 51 GCCAGGGACC CTTTGTGCAG AAGGAACTCG CGGCAGGTCA TCCACGGCCC 101 GATGCAGCCT TTTCGGAAGT GACTTCGTCA ACACCTTTGA TGGGAGCATG 151 TACAGCTTTG CGGGATACTG CAGTTACCTC CTGGCAGGGG GCTGCCAGAA 201 ACGCTCCTTC TCGATTATTG GGGACTTCCA GAATGGCAAG AGAGTGAGCC 251 TCTCCGTGTA TCTTGGGGAA TTTTTTGACA TCCATTTGTT TGTCAATGGT 301 ACCGTGACAC AGGGGGACCA AAGAGTCTCC ATGCCCTATG CCTCCAAAGG 351 GCTGTATCTA GAAACTGAGG CTGGGTACTA CAAGCTGTCC GGTGAGGCCT 401 ATGGCTTTGT GGCCAGGATC GATGGCAGCG GCAACTTTCA AGTCCTGCTG 451 TCAGACAGAT ACTTCAACAA GACCTGCGGG CTGTGTGGCA ACTTTAACAT 501 CTTTGCTGAA GATGACTTTA TGACCCAAGA AGGGACCTTG ACCTCGGACC 551 CTTATGACTT TGCCAACTCA TGGGCTCTGA GCAGTGGAGA ACAGTGGTGT 601 GAACGGGCAT CTCCTCCCAG CAGCTCATGC AACATCTCCT CTGGGGAAAT 651 GCAGAAGGGC CTGTGGGAGC AGTGCCAGCT TCTGAAGAGC ACCTCGGTGT 701 TTGCCCGCTG CCACCCTCTG GTGGACCCCG AGCCTTTTGT GGCCCTGTGT 751 GAGAAGACTT TGTGTGAGTG TGCTGGGGGG CTGGAGTGCG CCTGCCCTGC 801 CCTCCTGGAG TACGCCCGGA CCTGTGCCCA GGAGGGAATG GTGCTGTACG 851 GCTGGACCGA CCACAGCGCG TGCAGCCCAG TGTGCCCTGC TGGTATGGAG 901 TATAGGCAGT GTGTGTCCCC TTGCGCCAGG ACCTGCCAGA GCCTGCACAT 951 CAATGAAATG TGTCAGGAGC GATGCGTGGA TGGCTGCAGC TGCCCTGAGG 1001 GACAGCTCCT GGATGAAGGC CTCTGCGTGG AGAGCACCGA GTGTCCCTGC 1051 GTGCATTCCG GAAAGCGCTA CCCTCCCGGC ACCTCCCTCT CTCGAGACTG 1101 CAACACCTGC ATTTGCCGAA ACAGCCAGTG GATCTGCAGC AATGAAGAAT 1151 GTCCAGGGGA GTGCCTTGTC ACTGGTCAAT CCCACTTCAA GAGCTTTGAC 1201 AACAGATACT TCACCTTCAG TGGGATCTGC CAGTACCTGC TGGCCCGGGA 1251 TTGCCAGGAC CACTCCTTCT CCATTGTCAT TGAGACTGTC CAGTGTGCTG 1301 ATGACCGCGA CGCTGTGTGC ACCCGCTCCG TCACCGTCCG GCTGCCTGGC |
| 1351 CTGCACAACA GCCTTGTGAA ACTGAAGCAT GGGGCAGGAG TTGCCATGGA 1401 TGGCCAGGAC ATCCAGCTCC CCCTCCTGAA AGGTGACCTC CGCATCCAGC 1451 ATACAGTGAC GGCCTCCGTG CGCCTCAGCT ACGGGGAGGA CCTGCAGATG 1501 GACTGGGATG GCCGCGGGAG GCTGCTGGTG AAGCTGTCCC CCGTCTATGC 1551 CGGGAAGACC TGCGGCCTGT GTGGGAATTA CAATGGCAAC CAGGGCGACG 1601 ACTTCCTTAC CCCCTCTGGG CTGGCGGAGC CCCGGGTGGA GGACTTCGGG 1651 AACGCCTGGA AGCTGCACGG GGACTGCCAG GACCTGCAGA AGCAGCACAG 1701 CGATCCCTGC GCCCTCAACC CGCGCATGAC CAGGTTCTCC GAGGAGGCGT 1751 GCGCGGTCCT GACGTCCCCC ACATTCGAGG CCTGCCATCG TGCCGTCAGC 1801 CCGCTGCCCT ACCTGCGGAA CTGCCGCTAC GACGTGTGCT CCTGCTCGGA 1851 CGGCCGCGAG TGCCTGTGCG GCGCCCTGGC CAGCTATGCC GCGGCCTGCG 1901 CGGGGAGAGG CGTGCGCGTC GCGTGGCGCG AGCCAGGCCG CTGTGAGCTG 1951 AACTGCCCGA AAGGCCAGGT GTACCTGCAG TGCGGGACCC CCTGCAACCT 2001 GACCTGCCGC TCTCTCTCTT ACCCGGATGA GGAATGCAAT GAGGCCTGCC 2051 TGGAGGGCTG CTTCTGCCCC CCAGGGCTCT ACATGGATGA GAGGGGGGAC 2101 TGCGTGCCCA AGGCCCAGTG CCCCTGTTAC TATGACGGTG AGATCTTCCA 2151 GCCAGAAGAC ATCTTCTCAG ACCATCACAC CATGTGCTAC TGTGAGGATG 2201 GCTTCATGCA CTGTACCATG AGTGGAGTCC CCGGAAGCTT GCTGCCTGAC 2251 GCTGTCCTCA GCAGTCCCCT GTCTCATCGC AGCAAAAGGA GCCTATCCTG 2301 TCGGCCCCCC ATGGTCAAGC TGGTGTGTCC CGCTGACAAC CTGCGGGCTG 2351 AAGGGCTCGA GTGTACCAAA ACGTGCCAGA ACTATGACCT GGAGTGCATG 2401 AGCATGGGCT GTGTCTCTGG CTGCCTCTGC CCCCCGGGCA TGGTCCGGCA 2451 TGAGAACAGA TGTGTGGCCC TGGAAAGGTG TCCCTGCTTC CATCAGGGCA 2501 AGGAGTATGC CCCTGGAGAA ACAGTGAAGA TTGGCTGCAA CACTTGTGTC 2551 TGTCGGGACC GGAAGTGGAA CTGCACAGAC CATGTGTGTG ATGCCACGTG 2601 CTCCACGATC GGCATGGCCC ACTACCTCAC CTTCGACGGG CTCAAATACC 2651 TGTTCCCCGG GGAGTGCCAG TACGTTCTGG TGCAGGATTA CTGCGGCAGT 2701 AACCCTGGGA CCTTTCGGAT CCTAGTGGGG AATAAGGGAT GCAGCCACCC 2751 CTCAGTGAAA TGCAAGAAAC GGGTCACCAT CCTGGTGGAG GGAGGAGAGA 2801 TTGAGCTGTT TGACGGGGAG GTGAATGTGA AGAGGCCCAT GAAGGATGAG 2851 ACTCACTTTG AGGTGGTGGA GTCTGGCCGG TACATCATTC TGCTGCTGGG 2901 CAAAGCCCTC TCCGTGGTCT GGGACCGCCA CCTGAGCATC TCCGTGGTCC 2951 TGAAGCAGAC ATACCAGGAG AAAGTGTGTG GCCTGTGTGG GAATTTTGAT 3001 GGCATCCAGA ACAATGACCT CACCAGCAGC AACCTCCAAG TGGAGGAAGA 3051 CCCTGTGGAC TTTGGGAACT CCTGGAAAGT GAGCTCGCAG TGTGCTGACA 3101 CCAGAAAAGT GCCTCTGGAC TCATCCCCTG CCACCTGCCA TAACAACATC 3151 ATGAAGCAGA CGATGGTGGA TTCCTCCTGT AGAATCCTTA CCAGTGACGT 3201 CTTCCAGGAC TGCAACAAGC TGGTGGACCC CGAGCCATAT CTGGATGTCT 3251 GCATTTACGA CACCTGCTCC TGTGAGTCCA TTGGGGACTG CGCCGCATTC 3301 TGCGACACCA TTGCTGCCTA TGCCCACGTG TGTGCCCAGC ATGGCAAGGT 3351 GGTGACCTGG AGGACGGCCA CATTGTGCCC CCAGAGCTGC GAGGAGAGGA 3401 ATCTCCGGGA GAACGGGTAT GAGGCTGAGT GGCGCTATAA CAGCTGTGCA 3451 CCTGCCTGTC AAGTCACGTG TCAGCACCCT GAGCCACTGG CCTGCCCTGT 3501 GCAGTGTGTG GAGGGCTGCC ATGCCCACTG CCCTCCAGGG AAAATCCTGG 3551 ATGAGCTTTT GCAGACCTGC GTTGACCCTG AAGACTGTCC AGTGTGTGAG 3601 GTGGCTGGCC GGCGTTTTGC CTCAGGAAAG AAAGTCACCT TGAATCCCAG 3651 TGACCCTGAG CACTGCCAGA TTTGCCACTG TGATGTTGTC AACCTCACCT 3701 GTGAAGCCTG CCAGGAGCCG GGTACATCAG AGAGCGCCAC CCCTGAAAGT 3751 GGTCCCGGGA GCGAGCCAGC CACATCTGGG TCGGAAACGC CAGGCACATC 3801 CGAGTCTGCA ACTCCCGAGT CCGGACCTGG CTCCGAGCCT GCCACTAGCG 3851 GCTCCGAGAC TCCGGGAACT TCCGAGAGCG CTACACCAGA AAGCGGACCC 3901 GGAACCAGTA CCGAACCTAG CGAGGGCTCT GCTCCGGGCA GCCCAGCCGG 3951 CTCTCCTACA TCCACGGAGG AGGGCACTTC CGAATCCGCC ACCCCGGAGT 4001 CAGGGCCAGG ATCTGAACCC GCTACCTCAG GCAGTGAGAC GCCAGGAACG 4051 AGCGAGTCCG CTACACCGGA GAGTGGGCCA GGGAGCCCTG CTGGATCTCC 4101 TACGTCCACT GAGGAAGGGT CACCAGCGGG CTCGCCCACC AGCACTGAAG 4151 AAGGTGCCTC GTCTGACAAG AACACTGGTG ATTATTACGA GGACAGTTAT 4201 GAAGATATTT CAGCATACTT GCTGAGTAAA AACAATGCCA TTGAACCAAG 4251 AAGCTTCTCT GACAAAACTC ACACATGCCC ACCGTGCCCA GCTCCAGAAC |
| 4301 TCCTGGGCGG ACCGTCAGTC TTCCTCTTCC CCCCAAAACC CAAGGACACC 4351 CTCATGATCT CCCGGACCCC TGAGGTCACA TGCGTGGTGG TGGACGTGAG 4401 CCACGAAGAC CCTGAGGTCA AGTTCAACTG GTATGTGGAC GGCGTGGAAG 4451 TGCATAATGC CAAGACAAAG CCGCGGGAGG AGCAGTACAA CAGCACGTAC 4501 CGTGTGGTCA GCGTCCTCAC CGTCCTGCAC CAAGACTGGC TGAATGGCAA 4551 GGAGTACAAG TGCAAGGTCT CCAACAAAGC CCTCCCAGCC CCCATCGAGA 4601 AAACCATCTC CAAAGCCAAA GGGCAGCCCC GAGAACCACA GGTGTACACC 4651 CTGCCCCCAT CCCGGGATGA GCTGACCAAG AACCAAGTTA GCCTGACCTG 4701 CCTGGTCAAA GGCTTCTATC CCAGCGACAT CGCCGTGGAG TGGGAGAGCA 4751 ATGGGCAGCC GGAGAACAAC TACAAGACCA CGCCTCCCGT GTTGGACTCC 4801 GACGGCTCCT TCTTCCTCTA CTCCAAGCTC ACCGTGGACA AGAGCAGGTG 4851 GCAGCAGGGG AACGTCTTCT CATGCTCCGT GATGCATGAG GCTCTGCACA 4901 ACCACTACAC GCAGAAGAGC CTCTCCCTGT CTCCGGGTTG A |

| **FVIII(ELNN)-Fc: SEQ ID NO: 7** |
|---|
| TRRYYLGAVE LSWDYMQSDL GELPVDARFP PRVPKSFPFN TSVVYKKTLF VEFTDHLFNI 60 AKPRPPWMGL LGPTIQAEVY DTVVITLKNM ASHPVSLHAV GVSYWKASEG AEYDDQTSQR 120 EKEDDKVFPG GSHTYVWQVL KENGPMASDP LCLTYSYLSH VDLVKDLNSG LIGALLVCRE 180 GSLAKEKTQT LHKFILLFAV FDEGKSWHSE TKNS[?]MQDRD AASARAWPKM HTVNGYVNRS 240 LPGLIGCHRK SVYWHVIGMG TTPEVHSIFL EGHTFLVRNH RQASLEISPI TFLTAQTLLM 300 DLGQFLLFCH ISSHQHDGME AYVKVDSCPE EPQLRMKNNE EAEDYDDDLT DSEMDVVRFD 360 DDNSPSFIQI RSVAKKHPKT WVHYIAAEEE DWDYAPLVLA PDDRSYKSQY LNNGPQRIGR 420 KYKKVRFMAY TDETFKTREA IQHESGILGP LLYGEVGDTL LIIFKNQASR PYNIYPHGIT 480 DVRPLYSRRL PKGVKHLKDF PILPGEIFKY KWTVTVEDGP TKSDPRCLTR YYSSFVNMER 540 DLASGLIGPL LICYKESVDQ RGNQIMSDKR NVILFSVFDE NRSWYLTENI QRFLPNPAGV 600 QLEDPEFQAS NIMHSINGYV FDSLQLSVCL HEVAYWYILS IGAQTDFLSV FFSGYTFKHK 660 MVYEDTLTLF PFSGETVFMS MENPGLWILG CHNSDFRNRG MTALLKVSSC DKNTGDYYED 720 SYEDISAYLL SKNNAIEPRS FSQNGTSESA TPESGPGSEP ATSGSETPGT SESATPESGP 780 GSEPATSGSE TPGTSESATP ESGPGTSTEP SEGSAPGSPA GSPTSTEEGT SESATPESGP 840 GSEPATSGSE TPGTSESATP ESGPGSPAGS PTSTEEGSPA GSPTSTEEGT STEPSEGSAP 900 GTSESATPES GPGTSESATP ESGPGTSESA TPESGPGSEP ATSGSETPGS E[?]ATSGSETP 960 GSPAGSPTST EEGTSTEPSE GSAPGTSTEP SEGSAPGSEP ATSGSETPGT SESATPESGP 1020 GTSTEPSEGS APASSEITRT TLQSDQEEID YDDTISVEMK KEDFDIYDED ENQSPRSFQK 1080 KTRHYFIAAV ERLWDYGMSS SPHVLRNRAQ SGSVPQFKKV VFQEFTDGSF TQPLYRGELN 1140 EHLGLLGPYI RAEVEDNIMV TFRNQASRPY SFYSSLISYE EDQRQGAEPR KNFVKPNETK 1200 TYFWKVQHHM APTKDEFDCK AWAYFSDVDL EKDVHSGLIG PLLVCHTNTL N[?]AHGRQVTV 1260 QEFALFFTIF DETKSWYFTE NMERNCRAPC NIQMEDPTFK ENYRFHAING YIMDTLPGLV 1320 MAQDQRIRWY LLSMGSNENI HSIHFSGHVF TVRKKEEYKM ALYNLYPGVF ETVEMLPSKA 1380 GIWRVECLIG EHLHAGMSTL FLVYSNKCQT PLGMASGHIR DFQITASGQY GQWAPKLARL 1440 HYSGSINAWS TKEPFSWIKV DLLAPMIIHG IKTQGARQKF SSLYISQFII MYSLDGKKWQ 1500 TYRGNSTGTL MVFFGNVDSS GIKHNIFNPP IIARYIRLHP THYSIRSTLR MELMGCDLNS 1560 CSMPLGMESK AISDAQITAS SYFTNMFATW SPSKARLHLQ GRSNAWRPQV NNPKEWLQVD 1620 FQKTMKVTGV TTQGVKSLLT SMYVKEFLIS SSQDGHQWTL FFQNGKVKVF QGNQDSFTPV 1680 VNSLDPPLLT RYLRIHPQSW VHQIALRMEV LGCEAQDLYD KTHTCPPCPA PELLGGPSVF 1740 LFPPKPKDTL MISRTPEVTC VVVDVSHEDP EVKFNWYVDG VEVHNAKTKP REEQYNSTYR 1800 VVSVLTVLHQ DWLNGKEYKC KVSNKALPAP IEKTISKAKG QPREPQVYTL P[?]SRDELTKN 1860 QVSLTCLVKG FYPSDIAVEW ESNGQPENNY KTTPPVLDSD GSFFLYSKLT VDKSRWQQGN 1920 VFSCSVMHEA LHNHYTQKSL SLSPG 1945 |

**Table 4. Additional chimeric protein sequences**

| **Description / SEQ ID NO.** | **Sequence** |
|---|---|
| Full length FVIII | ATRRYYLGAV ELSWDYMQSD LGELPVDARF PPRVPKSFPF NTSVVYKKTL FVEFTDHLFN 60 IAKPRPPWMG LLGPTIQAEV YDTVVITLKN MASHPVSLHA VGVSYWKASE GAEYDDQTSQ 120 REKEDDKVFP GGSHTYVWQV LKENGPMASD PLCLTYSYLS HVDLVKDLNS GLIGALLVCR 180 EGSLAKEKTQ TLHKFILLFA VFDEGKSWHS ETKNSLMQDR DAASARAWPK MHTVNGYVNR 240 SLPGLIGCHR KSVYWHVIGM GTTPEVHSIF LEGHTFLVRN HRQASLEISP ITFLTAQTLL 300 MDLGQFLLFC HISSHQHDGM EAYVKVDSCP EEPQLRMKNN EEAEDYDDDL TDSEMDVVRF 360 DDDNSPSFIQ IRSVAKKHPK TWVHYIAAEE EDWDYAPLVL APDDRSYKSQ YLNNGPQRIG 420 RKYKKVRFMA YTDETFKTRE AIQHESGILG PLLYGEVGDT LLIIFKNQAS RPYNIYPHGI 480 |
| SEQ ID NO: 8 | |
| | TDVRPLYSRR LPKGVKHLKD FPILPGEIFK YKWTVTVEDG PTKSDPRCLT RYYSSFVNME 540 RDLASGLIGP LLICYKESVD QRGNQIMSDK RNVILFSVFD ENRSWYLTEN IQRFLPNPAG 600 VQLEDPEFQA SNIMHSINGY VFDSLQLSVC LHEVAYWYIL SIGAQTDFLS VFFSGYTFKH 660 KMVYEDTLTL FPFSGETVFM SMENPGLWIL GCHNSDFRNR GMTALLKVSS CDKNTGDYYE 720 DSYEDISAYL LSKNNAIEPR SFSQNSRHPS TRQKQFNATT IPENDIEKTD PWFAHRTPMP 780 KIQNVSSSDL LMLLRQSPTP HGLSLSDLQE AKYETFSDDP SPGAIDSNNS LSEMTHFRPQ 840 LHHSGDMVFT PESGLQLRLN EKLGTTAATE LKKLDFKVSS TSNNLISTIP SDNLAAGTDN 900 TSSLGPPSMP VHYDSQLDTT LFGKKSSPLT ESGGPLSLSE ENNDSKLLES GLMNSQESSW 960 GKNVSSTESG RLFKGKRAHG PALLTKDNAL FKVSISLLKT NKTSNNSATN RKTHIDGPSL 1020 LIENSPSVWQ NILESDTEFK KVTPLIHDRM LMDKNATALR LNHMSNKTTS SKNMEMVQQK 1080 KEGPIPPDAQ NPDMSFFKML FLPESARWIQ RTHGKNSLNS GQGPSPKQLV SLGPEKSVEG 1140 QNFLSEKNKV VVGKGEFTKD VGLKEMVFPS SRNLFLTNLD NLHENNTHNQ EKKIQEEIEK 1200 KETLIQENVV LPQIHTVTGT KNFMKNLFLL STRQNVEGSY DGAYAPVLQD FRSLNDSTNR 1260 TKKHTAHFSK KGEEENLEGL GNQTKQIVEK YACTTRISPN TSQQNFVTQR SKRALKQFRL 1320 PLEETELEKR IIVDDTSTQW SKNMKHLTPS TLTQIDYNEK EKGAITQSPL SDCLTRSHSI 1380 PQANRSPLPI AKVSSFPSIR PIYLTRVLFQ DNSSHLPAAS YRKKDSGVQE SSHFLQGAKK 1440 NNLSLAILTL EMTGDQREVG SLGTSATNSV TYKKVENTVL PKPDLPKTSG KVELLPKVHI 1500 YQKDLFPTET SNGSPGHLDL VEGSLLQGTE GAIKWNEANR PGKVPFLRVA TESSAKTPSK 1560 LLDPLAWDNH YGTQIPKEEW KSQEKSPEKT AFKKKDTILS LNACESNHAI AAINEGQNKP 1620 EIEVTWAKQG RTERLCSQNP PVLKRHQREI TRTTLQSDQE EIDYDDTISV EMKKEDFDIY 1680 DEDENQSPRS FQKKTRHYFI AAVERLWDYG MSSSPHVLRN RAQSGSVPQF KKVVFQEFTD 1740 GSFTQPLYRG ELNEHLGLLG PYIRAEVEDN IMVTFRNQAS RPYSFYSSLI SYEEDQRQGA 1800 EPRKNFVKPN ETKTYFWKVQ HHMAPTKDEF DCKAWAYFSD VDLEKDVHSG LIGPLLVCHT 1860 NTLNPAHGRQ VTVQEFALFF TIFDETKSWY FTENMERNCR APCNIQMEDP TFKENYRFHA 1920 INGYIMDTLP GLVMAQDQRI RWYLLSMGSN ENIHSIHFSG HVFTVRKKEE YKMALYNLYP 1980 GVFETVEMLP SKAGIWRVEC LIGEHLHAGM STLFLVYSNK CQTPLGMASG HIRDFQITAS 2040 GQYGQWAPKL ARLHYSGSIN AWSTKEPFSW IKVDLLAPMI IHGIKTQGAR QKFSSLYISQ 2100 FIIMYSLDGK KWQTYRGNST GTLMVFFGNV DSSGIKHNIF NPPIIARYIR LHPTHYSIRS 2160 TLRMELMGCD LNSCSMPLGM ESKAISDAQI TASSYFTNMF ATWSPSKARL HLQGRSNAWR 2220 PQVNNPKEWL QVDFQKTMKV TGVTTQGVKS LLTSMYVKEF LISSSQDGHQ WTLFFQNGKV 2280 KVFQGNQDSF TPVVNSLDPP LLTRYLRIHP QSWVHQIALR MEVLGCEAQD LY 2332 |
| AE288 | GTSESATPES GPGSEPATSG SETPGTSESA TPESGPGSEP ATSGSETPGT SESATPESGP 60 GTSTEPSEGS APGSPAGSPT STEEGTSESA TPESGPGSEP ATSGSETPGT SESATPESGP 120 GSPAGSPTST EEGSPAGSPT STEEGTSTEP SEGSAPGTSE SATPESGPGT SESATPESGP 180 GTSESATPES GPGSEPATSG SETPGSEPAT SGSETPGSPA GSPTSTEEGT STEPSEGSAP 240 GTSTEPSEGS APGSEPATSG SETPGTSESA TPESGPGTST EPSEGSAP 288 |
| SEQ ID NO: 9 | |
| pSYN VWF059 | TSTEEGASIS DKNTGDYYED SYEDISAYLL SKNNAIEPRS FSDKTH |
| SEQ ID NO: 10 | |
| pSYN VWF059A | TSTEEGASSD KNTGDYYEDS YEDISAYLLS KNNAIEPRSF SDKTH |
| SEQ ID NO: 11 | |
| pSYN FVIII 312 | ATRRYYLGAV ELSWDYMQSD LGELPVDARF PPRV[?]KSFPF NTSVVYKKTL FVEFTDHLFN 60 IAKPRPPWMG LLGPTIQAEV YDTVVITLKN MASH[?]VSLHA VGVSYWKASE GAEYDDQTSQ 120 REKEDDKVFP GGSHTYVWQV LKENGPMASD PLCLTYSYLS HVDLVKDLNS GLIGALLVCR 180 EGSLAKEKTQ TLHKFILLFA VFDEGKSWHS ETKNSLMQDR DAASARAWPK MHTVNGYVNR 240 SLPGLIGCHR KSVYWHVIGM GTTPEVHSIF LEGHTFLVRN HRQASLEISP ITFLTAQTLL 300 MDLGQFLLFC HISSHQHDGM EAYVKVDSCP EEPQLRMKNN EEAEDYDDDL T[?]SEMDVVRF 360 DDDNSPSFIQ IRSVAKKHPK TWVHYIAAEE EDWDYAPLVL APDDRSYKSQ YLNNGPQRIG 420 RKYKKVRFMA YTDETFKTRE AIQHESGILG PLLYGEVGDT LLIIFKNQAS RPYNIYPHGI 480 TDVRPLYSRR LPKGVKHLKD FPILPGEIFK YKWTVTVEDG PTKSDPRCLT RYYSSFVNME 540 RDLASGLIGP LLICYKESVD QRGNQIMSDK RNVILFSVFD ENRSWYLTEN IQRFLPNPAG 600 VQLEDPEFQA SNIMHSINGY VFDSLQLSVC LHEVAYWYIL SIGAQTDFLS VFFSGYTFKH 660 KMVYEDTLTL FPFSGETVFM SMENPGLWIL GCHNSDFRNR GMTALLKVSS C[?]KNTGDYYE 720 DSYEDISAYL LSKNNAIEPR SFSQNGTSES ATPESGPGSE PATSGSETPG TSESATPESG 780 PGSEPATSGS ETPGTSESAT PESGPGTSTE PSEGSAPGSP AGSPTSTEEG TSESATPESG 840 PGSEPATSGS ETPGTSESAT PESGPGSPAG SPTSTEEGSP AGSPTSTEEG TSTEPSEGSA 900 PGTSESATPE SGPGTSESAT PESGPGTSES ATPESGPGSE PATSGSETPG SEPATSGSET 960 PGSPAGSPTS TEEGTSTEPS EGSAPGTSTE PSEGSAPGSE PATSGSETPG TSESATPESG 1020 PGTSTEPSEG SAPASSEITR TTLQSDQEEI DYDDTISVEM KKEDFDIYDE DENQSPRSFQ 1080 KKTRHYFIAA VERLWDYGMS SSPHVLRNRA QSGSVPQFKK VVFQEFTDGS FTQPLYRGEL 1140 NEHLGLLGPY IRAEVEDNIM VTFRNQASRP YSFYSSLISY EEDQRQGAEP RKNFVKPNET 1200 KTYFWKVQHH MAPTKDEFDC KAWAYFSDVD LEKDVHSGLI GPLLVCHTNT LNPAHGRQVT 1260 VQEFALFFTI FDETKSWYFT ENMERNCRAP CNIQMEDPTF KENYRFHAIN GYIMDTLPGL 1320 |
| SEQ ID NO: 12 | |
| | VMAQDQRIRW YLLSMGSNEN IHSIHFSGHV FTVRKKEEYK MALYNLYPGV FETVEMLPSK 1380 AGIWRVECLI GEHLHAGMST LFLVYSNKCQ TPLGMASGHI RDFQITASGQ YGQWAPKLAR 1440 LHYSGSINAW STKEPFSWIK VDLLAPMIIH GIKTQGARQK FSSLYISQFI IMYSLDGKKW 1500 QTYRGNSTGT LMVFFGNVDS SGIKHNIFNP PIIARYIRLH PTHYSIRSTL RMELMGCDLN 1560 SCSMPLGMES KAISDAQITA SSYFTNMFAT WSPSKARLHL QGRSNAWRPQ VNNPKEWLQV 1620 DFQKTMKVTG VTTQGVKSLL TSMYVKEFLI SSSQDGHQWT LFFQNGKVKV FQGNQDSFTP 1680 VVNSLDPPLL TRYLRIHPQS WVHQIALRME VLGCEAQDLY DKTHTCPPCP APELLGGPSV 1740 FLFPPKPKDT LMISRTPEVT CVVVDVSHED PEVKFNWYVD GVEVHNAKTK PREEQYNSTY 1800 RVVSVLTVLH QDWLNGKEYK CKVSNKALPA PIEKTISKAK GQPREPQVYT LPPSRDELTK 1860 NQVSLTCLVK GFYPSDIAVE WESNGQPENN YKTT[?]PVLDS DGSFFLYSKL TVDKSRWQQG 1920 NVFSCSVMHE ALHNHYTQKS LSLSPGK |
| ELNN_AE288_2 | GSPAGSPTST EEGTSESATP ESGPGSEPAT SGSETPGTSE SATPESGPGT STEPSEGSAP 60 GTSTEPSEGS APGTSTEPSE GSAPGTSTEP SEGSAPGTST EPSEGSAPGT STEPSEGSAP 120 GSPAGSPTST EEGTSTEPSE GSAPGTSESA TPESGPGSEP ATSGSETPGT SESATPESGP 180 GSEPATSGSE TPGTSESATP ESGPGTSTEP SEGSAPGTSE SATPESGPGS PAGSPTSTEE 240 GSPAGSPTST EEGSPAGSPT STEEGTSESA TPESGPGTST EPSEGSAP 288 |
| SEQ ID NO: 13 | |
| ELNN AE144_5A | TSESATPESG PGSEPATSGS ETPGTSESAT PESGPGSEPA TSGSETPGTS ESATPESGPG 60 TSTEPSEGSA PGSPAGSPTS TEEGTSESAT PESGPGSEPA TSGSETPGTS ESATPESGPG 120 SPAGSPTSTE EGSPAGSPTS TEEG |
| SEQ ID NO: 14 | |
| a2 Linker of chimeric protein | DKNTGDYYED SYEDISAYLL SKNNAIEPRS FS 32 |
| SEQ ID NO: 15 | |
| Signal Peptide of FVIII | MQIELSTCFFLCLLRFCFS |
| SEQ ID NO: 16 | |
| FVIII fragment 1 of chimeric protein | ATRRYYLGAV ELSWDYMQSD LGELPVDARF PPRVPKSFPF NTSVVYKKTL FVEFTDHLFN 60 IAKPRPPWMG LLGPTIQAEV YDTVVITLKN MASHPVSLHA VGVSYWKASE GAEYDDQTSQ 120 REKEDDKVFP GGSHTYVWQV LKENGPMASD PLCLTYSYLS HVDLVKDLNS GLIGALLVCR 180 EGSLAKEKTQ TLHKFILLFA VFDEGKSWHS ETKNSLMQDR DAASARAWPK MHTVNGYVNR 240 SLPGLIGCHR KSVYWHVIGM GTTPEVHSIF LEGHTFLVRN HRQASLEISP ITFLTAQTLL 300 MDLGQFLLFC HISSHQHDGM EAYVKVDSCP EEPQLRMKNN EEAEDYDDDL TDSEMDVVRF 360 DDDNSPSFIQ IRSVAKKHPK TWVHYIAAEE EDWDYAPLVL APDDRSYKSQ YLNNGPQRIG 420 RKYKKVRFMA YTDETFKTRE AIQHESGILG PLLYGEVGDT LLIIFKNQAS RPYNIYPHGI 480 TDVRPLYSRR LPKGVKHLKD FPILPGEIFK YKWTVTVEDG PTKSDPRCLT RYYSSFVNME 540 RDLASGLIGP LLICYKESVD QRGNQIMSDK RNVILFSVFD ENRSWYLTEN IQRFLPNPAG 600 VQLEDPEFQA SNIMHSINGY VFDSLQLSVC LHEVAYWYIL SIGAQTDFLS VFFSGYTFKH 660 KMVYEDTLTL FPFSGETVFM SMENPGLWIL GCHNSDFRNR GMTALLKVSS CDKNTGDYYE 720 DSYEDISAYL LSKNNAIEPR SFSQN |
| SEQ ID NO. 17 | |
| FVIII fragment 2 of chimeric protein | EITRTTLQSD QEEIDYDDTI SVEMKKEDFD IYDEDENQSP RSFQKKTRHY FIAAVERLWD 60 YGMSSSPHVL RNRAQSGSVP QFKKVVFQEF TDGSFTQPLY RGELNEHLGL LGPYIRAEVE 120 DNIMVTFRNQ ASRPYSFYSS LISYEEDQRQ GAEPRKNFVK PNETKTYFWK VQHHMAPTKD 180 EFDCKAWAYF SDVDLEKDVH SGLIGPLLVC HTNTLNPAHG RQVTVQEFAL FFTIFDETKS 240 WYFTENMERN CRAPCNIQME DPTFKENYRF HAINGYIMDT LPGLVMAQDQ RIRWYLLSMG 300 SNENIHSIHF SGHVFTVRKK EEYKMALYNL YPGVFETVEM LPSKAGIWRV ECLIGEHLHA 360 GMSTLFLVYS NKCQTPLGMA SGHIRDFQIT ASGQYGQWAP KLARLHYSGS INAWSTKEPF 420 SWIKVDLLAP MIIHGIKTQG ARQKFSSLYI SQFIIMYSLD GKKWQTYRGN STGTLMVFFG 480 NVDSSGIKHN IFNPPIIARY IRLHPTHYSI RSTLRMELMG CDLNSCSMPL GMESKAISDA 540 QITASSYFTN MFATWSPSKA RLHLQGRSNA WRPQVNNPKE WLQVDFQKTM KVTGVTTQGV 600 KSLLTSMYVK EFLISSSQDG HQWTLFFQNG KVKVFQGNQD SFTPVVNSLD PPLLTRYLRI 660 HPQSWVHQIA LRMEVLGCEA QDLY |
| SEQ ID NO. 18 | |
| VWF Signal Peptide | MIPARFAGVL LALALILPGT LC |
| SEQ ID NO: 19 | |
| VWF D1D2 domain of chimeric protein | AEGTRGRSST ARCSLFGSDF VNTFDGSMYS FAGYCSYLLA GGCQKRSFSI IGDFQNGKRV 60 SLSVYLGEFF DIHLFVNGTV TQGDQRVSMP YASKGLYLET EAGYYKLSGE AYGFVARIDG 120 SGNFQVLLSD RYFNKTCGLC GNFNIFAEDD FMTQEGTLTS DPYDFANSWA LSSGEQWCER 180 ASPPSSSCNI SSGEMQKGLW EQCQLLKSTS VFARCHPLVD PEPFVALCEK TLCECAGGLE 240 CACPALLEYA RTCAQEGMVL YGWTDHSACS PVCPAGMEYR QCVSPCARTC QSLHINEMCQ 300 ERCVDGCSCP EGQLLDEGLC VESTECPCVH SGKRYPPGTS LSRDCNTCIC RNSQWICSNE 360 ECPGECLVTG QSHFKSFDNR YFTFSGICQY LLARDCQDHS FSIVIETVQC ADDRDAVCTR 420 SVTVRLPGLH NSLVKLKHGA GVAMDGQDIQ LPLLKGDLRI QHTVTASVRL SYGEDLQMDW 480 DGRGRLLVKL SPVYAGKTCG LCGNYNGNQG DDFLTPSGLA EPRVEDFGNA WKLHGDCQDL 540 QKQHSDPCAL NPRMTRFSEE ACAVLTSPTF EACHRAVSPL PYLRNCRYDV CSCSDGRECL 600 CGALASYAAA CAGRGVRVAW REPGRCELNC PKGQVYLQCG TPCNLTCRSL SYPDEECNEA 660 CLEGCFCPPG LYMDERGDCV PKAQCPCYYD GEIFQPEDIF SDHHTMCYCE DGFMHCTMSG 720 VPGSLLPDAV LSSPLSHRSK R |
| SEQ ID NO: 20 | |
| VWF D' domain of chimeric protein | SLSCRPPMVK LVCPADNLRA EGLECTKTCQ NYDLECMSMG CVSGCLCPPG MVRHENRCVA 60 LERCPCFHQG KEYAPGETVK IGCNTCVCRD RKWNCTDHVC DAT 103 |
| SEQ ID NO: 21 | |
| VWF D3 domain of chimeric protein | CSTIGMAHYL TFDGLKYLFP GECQYVLVQD YCGSNPGTFR ILVGNKGCSH PSVKCKKRVT 60 ILVEGGEIEL FDGEVNVKRP MKDETHFEVV ESGRYIILLL GKALSVVWDR HLSISVVLKQ 120 TYQEKVCGLC GNFDGIQNND LTSSNLQVEE DPVDFGNSWK VSSQCADTRK VPLDSSPATC 180 HNNIMKQTMV DSSCRILTSD VFQDCNKLVD PEPYLDVCIY DTCSCESIGD CAAFCDTIAA 240 YAHVCAQHGK VVTWRTATLC PQSCEERNLR ENGYEAEWRY NSCAPACQVT CQHPEPLACP 300 VQCVEGCHAH CPPGKILDEL LQTCVDPEDC PVCEVAGRRF ASGKKVTLNP SDPEHCQICH 360 CDVVNLTCEA CQEP 374 |
| SEQ ID NO: 22 | |
| Fc region | DKTHTCPPCP APELLGGPSV FLFPPKPKDT LMISRTPEVT CVVVDVSHED PEVKFNWYVD 60 GVEVHNAKTK PREEQYNSTY RVVSVLTVLH QDWLNGKEYK CKVSNKALPA PIEKTISKAK 120 GQPREPQVYT LPPSRDELTK NQVSLTCLVK GFYPSDIAVE WESNGQPENN YKTTPPVLDS 180 DGSFFLYSKL TVDKSRWQQG NVFSCSVMHE ALHNHYTQKS LSLSPG |
| SEQ ID NO: 23 | |
| ELNN AE288_3 | GTSESATPES GPGSEPATSG SETPGTSESA TPESGPGSEP ATSGSETPGT SESATPESGP 60 GTSTEPSEGS APGSPAGSPT STEEGTSESA TPESGPGSEP ATSGSETPGT SESATPESGP 120 GSPAGSPTST EEGSPAGSPT STEEGTSTEP SEGSAPGTSE SATPESGPGT SESATPESGP 180 GTSESATPES GPGSEPATSG SETPGSEPAT SGSETPGSPA GSPTSTEEGT STEPSEGSAP 240 GTSTEPSEGS APGSEPATSG SETPGTSESA TPESGPGTST EPSEGSAPAS S 291 |
| SEQ ID NO: 24 | |

The invention pertains also to following:
1. A pharmaceutical composition comprising:
   (a) a chimeric protein comprising
      a first polypeptide chain which comprises a Factor VIII ("FVIII") protein and a first immunoglobulin ("Ig") constant region or a portion thereof, and
      a second polypeptide chain which comprises a von Willebrand Factor ("VWF") protein and a second Ig constant region or a portion thereof;
   (b) sucrose;
   (c) histidine;
   (d) arginine;
   (e) calcium chloride; and
   (f) a poloxamer.
2. The pharmaceutical composition of item 1, wherein the pharmaceutical composition comprises about 1% (w/v) to about 7.5% (w/v) sucrose.
3. The pharmaceutical composition of item 1 or 2, wherein the pharmaceutical composition comprises about 1% (w/v) to about 5% (w/v) sucrose.
4. The pharmaceutical composition of any one of items 1-3, wherein the poloxamer is poloxamer 188 (P188).
5. The pharmaceutical composition of any one of items 1-4, wherein the pharmaceutical composition comprises about 5 mM to about 15 mM histidine.
6. The pharmaceutical composition of any one of items 1-5, wherein the pharmaceutical composition comprises about 200 mM to about 300 mM arginine.
7. The pharmaceutical composition of any one of items 1-6, wherein the pharmaceutical composition comprises about 2.5 mM to about 10 mM calcium chloride.
8. The pharmaceutical composition of any one of items 1-7, wherein the pharmaceutical composition comprises about 0.01% (w/v) to about 1.0% (w/v) poloxamer 188 (P188).
9. The pharmaceutical composition of any one of items 1-8, wherein the pharmaceutical composition comprises:
   (a) about 1% (w/v) to about 5% (w/v) sucrose;
   (b) about 5 mM to about 15 mM histidine;
   (c) about 200 mM to about 300 mM arginine;
   (d) about 2.5 mM to about 10 mM calcium chloride; and
   (e) about 0.01% (w/v) to about 1.0% (w/v) poloxamer 188.
10. The pharmaceutical composition of any one of items 1-9, wherein the pharmaceutical composition comprises:
   (a) about 5% (w/v) sucrose;
   (b) about 10 mM histidine;
   (c) about 250 mM arginine;
   (d) about 5 mM calcium chloride; and
   (e) about 0.1% (w/v) poloxamer 188.
11. The pharmaceutical composition of any one of items 1-10, wherein the pharmaceutical composition comprises:
   (a) about 5% (w/v) sucrose;
   (b) about 10 mM L-histidine;
   (c) about 250 mM L-arginine-HCl;
   (d) about 5 mM calcium chloride; and
   (e) about 0.1% (w/v) poloxamer 188.
12. The pharmaceutical composition of any one of items 1-11, wherein the pharmaceutical composition comprises:
   (a) about 1% (w/v) sucrose;
   (b) about 10 mM histidine;
   (c) about 250 mM arginine;
   (d) about 5 mM calcium chloride; and
   (e) about 0.1% (w/v) poloxamer 188.
13. The pharmaceutical composition of any one of items 1-12, wherein the pharmaceutical composition comprises:
   (a) about 1% (w/v) sucrose;
   (b) about 10 mM L-histidine;
   (c) about 250 mM L-arginine-HCl;
   (d) about 5 mM calcium chloride; and
   (e) about 0.1% (w/v) poloxamer 188.
14. The pharmaceutical composition of item 1, wherein the pharmaceutical composition comprises:
   (a) 10 mg/mL to 60 mg/mL sucrose;
   (b) 1.5 mg/mL to 2.0 mg/mL L-histidine;
   (c) 40 mg/mL to 70 mg/mL L-arginine-HCl;
   (d) 0.4 mg/mL to 0.9 mg/mL calcium chloride; and
   (e) 0.6 mg/mL to 1.6 mg/mL poloxamer 188.
15. The pharmaceutical composition of item 14, wherein the pharmaceutical composition comprises:
   (a) 56.12 mg/ml sucrose;
   (b) 1.74 mg/ml L-histidine;
   (c) 59.11 mg/ml L-arginine-HCl;
   (d) 0.82 mg/ml calcium chloride dihydrate; and
   (e) 1.12 mg/ml poloxamer 188.
16. The pharmaceutical composition of any one of items 14-15, wherein the pharmaceutical composition comprises:
   (a) 56.12 mg/ml sucrose;
   (b) 1.74 mg/ml L-histidine;
   (c) 59.11 mg/ml L-arginine-HCl;
   (d) 0.62 mg/ml calcium chloride; and
   (e) 1.12 mg/ml poloxamer 188.
17. The pharmaceutical composition of item 14, wherein the pharmaceutical composition comprises:
   (a) 11.23 mg/ml sucrose;
   (b) 1.74 mg/ml L-histidine;
   (c) 59.11 mg/ml L-arginine-HCl;
   (d) 0.82 mg/ml calcium chloride dihydrate; and
   (e) 1.12 mg/ml poloxamer 188.
18. The pharmaceutical composition of any one of items 17-18, wherein the pharmaceutical composition comprises:
   (a) 11.23 mg/ml sucrose;
   (b) 1.74 mg/ml L-histidine;
   (c) 59.11 mg/ml L-arginine-HCl;
   (d) 0.62 mg/ml calcium chloride; and
   (e) 1.12 mg/ml poloxamer 188.
19. The pharmaceutical composition of item 14, wherein the pharmaceutical composition comprises:
   (a) about 50 mg/mL sucrose;
   (b) about 1.6 mg/mL L-histidine;
   (c) about 52.7 mg/mL L-arginine-HCl;
   (d) about 0.7 mg/mL calcium chloride dihydrate; and
   (e) about 1 mg/mL poloxamer 188.
20. The pharmaceutical composition of item 14, wherein the pharmaceutical composition comprises:
   (a) about 50 mg/mL sucrose;
   (b) about 1.6 mg/mL L-histidine;
   (c) about 43.6 mg/mL L-arginine;
   (d) about 0.7 mg/mL calcium chloride dihydrate; and
   (e) about 1 mg/mL poloxamer 188.
21. The pharmaceutical composition of item 14, wherein the pharmaceutical composition comprises:
   (a) about 50 mg/mL sucrose;
   (b) about 1.6 mg/mL L-histidine;
   (c) about 52.7 mg/mL L-arginine-HCl;
   (d) about 0.6 mg/mL calcium chloride; and
   (e) about 1 mg/mL poloxamer 188.
22. The pharmaceutical composition of item 14, wherein the pharmaceutical composition comprises:
   (a) about 10 mg/mL sucrose;
   (b) about 1.6 mg/mL L-histidine;
   (c) about 52.7 mg/mL L-arginine-HCl;
   (d) about 0.7 mg/mL calcium chloride dihydrate; and
   (e) about 1 mg/mL poloxamer 188.
23. The pharmaceutical composition of item 14, wherein the pharmaceutical composition comprises:
   (a) about 10 mg/mL sucrose;
   (b) about 1.6 mg/mL L-histidine;
   (c) about 43.6 mg/mL L-arginine;
   (d) about 0.7 mg/mL calcium chloride dihydrate; and
   (e) about 1 mg/mL poloxamer 188.
24. The pharmaceutical composition of item 14, wherein the pharmaceutical composition comprises:
   (a) about 10 mg/mL sucrose;
   (b) about 1.6 mg/mL L-histidine;
   (c) about 52.7 mg/mL L-arginine-HCl;
   (d) about 0.6 mg/mL calcium chloride; and
   (e) about 1 mg/mL poloxamer 188.
25. The pharmaceutical composition of item 14, wherein the pharmaceutical composition comprises:
   (a) about 10 mg/mL sucrose;
   (b) about 1.6 mg/mL L-histidine;
   (c) about 43.6 mg/mL L-arginine;
   (d) about 0.6 mg/mL calcium chloride; and
   (e) about 1 mg/mL poloxamer 188.
26. The pharmaceutical composition of item 14, wherein the pharmaceutical composition comprises:
   (a) 56.12 mg/ml sucrose;
   (b) 1.74 mg/ml L-histidine;
   (c) 48.88 mg/ml L-arginine;
   (d) 0.82 mg/ml calcium chloride dihydrate; and
   (e) 1.12 mg/ml poloxamer 188.
27. The pharmaceutical composition of item 14, wherein the pharmaceutical composition comprises:
   (a) 56.12 mg/ml sucrose;
   (b) 1.74 mg/ml L-histidine;
   (c) 59.11 mg/ml L-arginine-HCl;
   (d) 0.62 mg/ml calcium chloride; and
   (e) 1.12 mg/ml poloxamer 188.
28. The pharmaceutical composition of item 14, wherein the pharmaceutical composition comprises:
   (a) 56.12 mg/ml sucrose;
   (b) 1.74 mg/ml L-histidine;
   (c) 48.88 mg/ml L-arginine;
   (d) 0.62 mg/ml calcium chloride; and
   (e) 1.12 mg/ml poloxamer 188.
29. The pharmaceutical composition of item 14, wherein the pharmaceutical composition comprises:
   (a) 11.23 mg/ml sucrose;
   (b) 1.74 mg/ml L-histidine;
   (c) 48.88 mg/ml L-arginine;
   (d) 0.82 mg/ml calcium chloride dihydrate; and
   (e) 1.12 mg/ml poloxamer 188.
30. The pharmaceutical composition of item 14, wherein the pharmaceutical composition comprises:
   (a) 11.23 mg/ml sucrose;
   (b) 1.74 mg/ml L-histidine;
   (c) 59.11 mg/ml L-arginine-HCl;
   (d) 0.62 mg/ml calcium chloride; and
   (e) 1.12 mg/ml poloxamer 188.
31. The pharmaceutical composition of item 14, wherein the pharmaceutical composition comprises:
   (a) 11.23 mg/ml sucrose;
   (b) 1.74 mg/ml L-histidine;
   (c) 48.88 mg/ml L-arginine;
   (d) 0.62 mg/ml calcium chloride; and
   (e) 1.12 mg/ml poloxamer 188.
32. The pharmaceutical composition of any one of items 1 to 31, wherein the pharmaceutical composition has a pH of about 6.5 to about 7.5.
33. The pharmaceutical composition of any one of items 1 to 32, wherein the pharmaceutical composition has a pH of about 7.0.
34. The pharmaceutical composition of any one of items 1 to 32, wherein the pharmaceutical composition has a pH of about 6.8.
35. The pharmaceutical composition of any one of items 1 to 34, wherein the pharmaceutical composition comprises less than 8.8 mg/mL sodium chloride (NaCl).
36. The pharmaceutical composition of any one of items 1 to 35, wherein the pharmaceutical composition does not comprise NaCl.
37. The pharmaceutical composition of any one of items 1 to 10, wherein the histidine is L-histidine.
38. The pharmaceutical composition of any one of items 1 to 10, wherein the arginine is L-arginine.
39. The pharmaceutical composition of any one of items 1 to 10, which comprises arginine-HCl.
40. The pharmaceutical composition of any one of items 1 to 10, which comprises L-arginine-HCl.
41. The pharmaceutical composition of any one of items 1 to 10, which comprises calcium chloride dihydrate.
42. The pharmaceutical composition of any one of items 1 to 41, wherein the pharmaceutical composition has an osmolality about 525 to about 725 mOsm/kg.
43. The pharmaceutical composition of any one of items 1 to 41, wherein the pharmaceutical composition comprises has an osmolality about 500 to about 650 mOsm/kg.
44. The pharmaceutical composition of any one of items 1 to 43, wherein the pharmaceutical composition comprises has a turbidity of less than about 7 Nephelometric Turbidity Units (NTU).
45. The pharmaceutical composition of any one of items 1 to 44, wherein the first polypeptide chain comprises the amino acid sequence set forth as SEQ ID NO: 1 and the second polypeptide chain comprises the amino acid sequence set forth as SEQ ID NO: 2, wherein the first polypeptide chain and the second polypeptide chain are covalently linked by two disulfide bonds between Fc domains in the first and second polypeptide chains.
46. The pharmaceutical composition of any one of items 1 to 45, wherein the chimeric protein is efanesoctocog alfa.
47. The pharmaceutical composition of any one of items 1 to 46, wherein the pharmaceutical composition has a chimeric protein concentration of about 0.8 to about 1.2 mg/mL.
48. The pharmaceutical composition of any one of items 1 to 47, wherein the pharmaceutical composition comprises 75 IU/mL to 2,000 IU/mL of the chimeric protein.
49. The pharmaceutical composition of item 48, wherein the pharmaceutical composition comprises about 250 IU, 500 **IU,** 1000 IU, 2000 IU, 3000 IU, or 4,000 IU of the chimeric protein.
50. The pharmaceutical composition of any one of items 1 to 48, wherein the pharmaceutical composition comprises at least about 500 **IU** of the chimeric protein or from about 0.5 mg/ml to about 5 mg/ml of the chimeric protein.
51. The pharmaceutical composition of any one of items 1-50, which (i) has been stored at a temperature of about -20°C to about -40°C for at least about 1 to about 36 months; and/or (ii) is a liquid formulation that has been frozen and then thawed 2-5 times.
52. A method of treating hemophilia A in a subject in need thereof, comprising administering to the subject an effective amount of the pharmaceutical composition of any one of items 1 to 51.
53. The method of item 52, wherein the pharmaceutical composition is self-administered.
54. The method of item 52 or 53, wherein the pharmaceutical composition is administered intravenously.
55. The method of any one of items 52 to 54, wherein the pharmaceutical composition is administered intravenously at a dose of 20 IU/kg to 70 IU/kg.
56. The method of any one of items 52 to 55, wherein the pharmaceutical composition is administered intravenously at a dose of 50 IU/kg.
57. The method any one of items 52 to 56, wherein the pharmaceutical composition is administered intravenously once every 7-10 days.
58. The method of item any one of items 52 to 57, wherein the pharmaceutical composition is administered intravenously once weekly.
59. A pharmaceutical kit comprising:
   (i) a first container comprising a lyophilized pharmaceutical composition comprising
      (a) a chimeric protein comprising
         a first polypeptide chain which comprises a Factor VIII ("FVIII") protein or a portion thereof and a first immunoglobulin ("Ig") constant region or a portion thereof, and
         a second polypeptide chain which comprises a von Willebrand Factor ("VWF") protein and a second Ig constant region or a portion thereof;
      (b) sucrose;
      (c) histidine;
      (d) arginine;
      (e) calcium chloride; and
      (f) poloxamer 188, and
   (ii) a second container comprising sterile water.
60. The pharmaceutical kit of item 59, wherein the lyophilized pharmaceutical composition comprises:
   (a) about 10 mg to about 200 mg sucrose;
   (b) about 2.5 mg to about 7.5 mg histidine;
   (c) about 140 mg to about 200 mg arginine;
   (d) about 1.5 mg to about 5 mg calcium chloride; and
   (e) about 1 mg to about 10 mg poloxamer 188.
61. The pharmaceutical kit of any one of items 59-60, which does not comprise NaCl.
62. The pharmaceutical kit of any one of items 59-61, wherein the histidine is L-histidine.
63. The pharmaceutical kit of any one of items 59-62, wherein the arginine is L-arginine.
64. The pharmaceutical kit of any one of items 59-63, which comprises arginine-HCl.
65. The pharmaceutical kit of any one of items 59-64, which comprises L-arginine-HCl.
66. The pharmaceutical kit of any one of items 59-65, which comprises calcium chloride dihydrate.
67. The pharmaceutical kit of any one of items 59-66, wherein the lyophilized pharmaceutical composition comprises:
   (a) about 168.3 mg sucrose;
   (b) about 5.2 mg L-histidine;
   (c) about 177.3 mg L-arginine-HCl;
   (d) about 2.5 mg calcium chloride; and
   (e) about 3.3 mg poloxamer 188.
68. The pharmaceutical kit of any one of items 59-66, wherein the lyophilized pharmaceutical composition comprises:
   (a) about 33.7 mg sucrose;
   (b) about 5.2 mg L-histidine;
   (c) about 146.6 mg L-arginine-HCl;
   (d) about 2.5 mg calcium chloride; and
   (e) about 3.3 mg poloxamer 188.
69. The pharmaceutical kit of any one of items 59-68, wherein the second container comprises about 2 mL to about 5 mL of the sterile water.
70. The pharmaceutical kit of any one of items 59-68, wherein the second container comprises about 3 mL of the sterile water.
71. The pharmaceutical kit of any one of items 59-68, wherein the second container comprises about 3.3 mL of the sterile water.
72. The pharmaceutical kit of any one of items 59-71, wherein the first container is a glass vial comprising a rubber stopper.
73. The pharmaceutical kit of any one of items 59-72, wherein the second container is a syringe body.
74. The pharmaceutical kit of item 73, wherein the sterile water is in the syringe body.
75. The pharmaceutical kit of item 73 or 74, wherein the syringe body is associated with a plunger.
76. The pharmaceutical kit of any one of items 73-75, further comprising an adaptor to connect the glass vial to the syringe body.
77. The pharmaceutical kit of any one of items 73-76, further comprising infusion tubing associated with a needle to be connected to the syringe body, suitable for intravenous infusion.
78. The pharmaceutical kit of any one of items 59-77, wherein the first polypeptide chain comprises the amino acid sequence set forth as SEQ ID NO: 1 and the second polypeptide chain comprises the amino acid sequence set forth as SEQ **ID** NO: 2, wherein the first polypeptide chain and the second polypeptide chain are covalently linked by two disulfide bonds between Fc domains in the first and second polypeptide chains.
79. The pharmaceutical kit of any one of items 59-78, which has been stored at a temperature of about -20°C, about -25°C, about -30°C, about -35°C, or about -40°C for at least about 1 to about 36 months.
80. A method of treating hemophilia A in a subject in need thereof, comprising combining the lyophilized pharmaceutical composition and the sterile water of the kit of any one of items 59-79, and administering to the subject an effective amount of the resulting combination.
81. The method of item 80, wherein the subject combines the lyophilized pharmaceutical composition and the sterile water of the kit.
82. The method of item 80 or 81, wherein the combination is self-administered by the subject.
83. A method of storing a pharmaceutical composition or kit, comprising maintaining the pharmaceutical composition of any one of items 1-50 or the kit of any one of items 59-78 at a temperature of from about -20°C to about -40°C for at least about 1 to about 36 months.

## Claims

1. A pharmaceutical kit comprising:
(i) a first container comprising a lyophilized pharmaceutical composition comprising:
(a) a chimeric protein comprising a first polypeptide chain which comprises the amino acid sequence set forth as SEQ ID NO: 1, and a second polypeptide chain which comprises the amino acid sequence set forth as SEQ ID NO: 2, wherein the first polypeptide chain and the second polypeptide chain are covalently linked by two disulfide bonds between Fc domains in the first and second polypeptide chains;
(b) sucrose;
(c) histidine;
(d) arginine;
(e) calcium chloride; and
(f) a poloxamer,
(ii) a second container comprising sterile water;
wherein upon reconstitution with the sterile water the reconstituted pharmaceutical composition comprises:
(a) the chimeric protein
(b) sucrose;
(c) histidine;
(d) at least about 150 mM arginine;
(e) calcium chloride; and
(f) a poloxamer,
wherein about means ± 10%.

2. The pharmaceutical kit of claim 1, wherein upon reconstitution with the sterile water the reconstituted pharmaceutical composition comprises about 200 mM to 300 mM arginine, wherein about means ± 10%, preferably wherein upon reconstitution with the sterile water the reconstituted pharmaceutical composition comprises 200 mM to 300 mM arginine.

3. The pharmaceutical kit of claim 1 or 2, wherein upon reconstitution with the sterile water the reconstituted pharmaceutical composition comprises 1% (w/v) to 7.5% (w/v) sucrose.

4. The pharmaceutical kit of any one of claims 1 to 3, wherein the poloxamer is poloxamer 188 (P188).

5. The pharmaceutical kit of any one of claims 1 to 4, wherein upon reconstitution with the sterile water the reconstituted pharmaceutical composition comprises 5 mM to 15 mM histidine.

6. The pharmaceutical kit of any one of claims 1 to 5, wherein upon reconstitution with the sterile water the reconstituted pharmaceutical composition comprises about 250 mM arginine, wherein about means ± 10%, preferably wherein upon reconstitution with the sterile water the reconstituted pharmaceutical composition comprises 250 mM arginine.

7. The pharmaceutical kit of any one of claims 1 to 6, wherein upon reconstitution with the sterile water the reconstituted pharmaceutical composition comprises 2.5 mM to 10 mM calcium chloride.

8. The pharmaceutical kit of any one of claims 1 to 7, wherein upon reconstitution with the sterile water the reconstituted pharmaceutical composition comprises 0.01 % (w/v) to 1.0% (w/v) poloxamer 188 (P188).

9. The pharmaceutical kit of any one of claims 1 to 8, wherein upon reconstitution with the sterile water the reconstituted pharmaceutical composition comprises:
(a) about 5% (w/v) sucrose;
(b) about 10 mM histidine;
(c) about 250 mM arginine;
(d) about 5 mM calcium chloride; and
(e) about 0.1% (w/v) poloxamer 188,
wherein about means ± 10%, preferably wherein upon reconstitution with the sterile water the reconstituted pharmaceutical composition comprises:
(a) 5% (w/v) sucrose;
(b) 10 mM histidine;
(c) 250 mM arginine;
(d) 5 mM calcium chloride; and
(e) 0.1% (w/v) poloxamer 188.

10. The pharmaceutical kit of any one of claims 1 to 9,
wherein upon reconstitution with the sterile water the reconstituted pharmaceutical composition comprises has a pH of 6.5 to 7.5, or
wherein upon reconstitution with the sterile water the reconstituted pharmaceutical composition comprises has a pH of about 7.0, wherein about means ± 10%.

11. The pharmaceutical kit of any one of claims 1 to 10, wherein upon reconstitution with the sterile water the reconstituted pharmaceutical composition has a pH of 7.0.

12. The pharmaceutical kit of any one of claims 1 to 11, wherein upon reconstitution with the sterile water the reconstituted pharmaceutical composition comprises less than 8.8 mg/mL sodium chloride (NaCl).

13. The pharmaceutical kit of any one of claims 1 to 12,
wherein the histidine is L-histidine, and/or
wherein the arginine is L-arginine, and/or
wherein the chimeric protein is efanesoctocog alfa.

14. The pharmaceutical kit of any one of claims 1 to 13, wherein the lyophilized pharmaceutical composition comprises 250 IU, 500 IU, 1000 IU, 2000 IU, 3000 IU, or 4,000 IU of the chimeric protein.

15. The pharmaceutical kit of any one of claims 1 to 14, wherein the reconstituted pharmaceutical composition is suitable for intravenous infusion.
